(19) Europäisches Patentamt
European Patent Office
Office européen des brevets

(11) EP 3 178 810 A1

(12) **EUROPEAN PATENT APPLICATION**
published in accordance with Art. 153(4) EPC

(43) Date of publication:
**14.06.2017 Bulletin 2017/24**

(21) Application number: **15829001.5**

(22) Date of filing: **07.08.2015**

(51) Int Cl.:
*C07D 209/40* (2006.01) *A61K 31/4045* (2006.01)
*A61K 31/423* (2006.01) *A61K 31/424* (2006.01)
*A61K 31/428* (2006.01) *A61K 31/429* (2006.01)
*A61P 3/06* (2006.01) *C07D 263/56* (2006.01)
*C07D 417/04* (2006.01) *C07D 498/04* (2006.01)
*C07D 513/04* (2006.01)

(86) International application number:
**PCT/JP2015/072442**

(87) International publication number:
**WO 2016/021706 (11.02.2016 Gazette 2016/06)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **08.08.2014 JP 2014162173**

(71) Applicant: **Kasuma Partners Inc.**
**Tokyo 103-0022 (JP)**

(72) Inventors:
- **MAEKAWA, Hiroki**
  **Tokyo 103-0022 (JP)**
- **YOKOYAMA, Shinji**
  **Nagoya-shi**
  **Aichi 467-0024 (JP)**

(74) Representative: **Godemeyer Blum Lenze Patentanwälte**
**Partnerschaft mbB - werkpatent**
**An den Gärten 7**
**51491 Overath (DE)**

(54) **CONDENSED HETEROCYCLIC COMPOUND**

(57) A compound represented by the general formula (I) [$R^1$ represents a $C_{1-6}$ alkyl group, a halogen atom, or the like; A represents a phenylene group, or the like; X represents -CH($R^3$)-, -O-, -NH-, or the like; Y represents -O-, -NH-, -N=, or -S-;represents a single bond or double bond; n represents 1 to 3; $R^2$ represents a $C_{1-6}$ alkyl group, a $C_{1-6}$ alkoxy group, or the like; and $R^3$ represents hydrogen atom, a $C_{1-6}$ alkyl group, or the like], or a salt thereof, which has a blood LDL cholesterol-reducing action, and is useful as an active ingredient of medicaments.

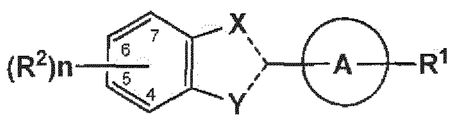

(I)

EP 3 178 810 A1

## Description

### Technical Field

The present invention relates to a condensed heterocyclic compound having a blood LDL cholesterol-reducing action.

### Background Art

It is known that serum LDL (low density lipoprotein) cholesterol level is a major risk factor of coronary artery diseases, and by reducing serum LDL cholesterol level, risk of coronary artery diseases can be lowered. In order to reduce the blood LDL cholesterol level, statin drugs have been widely used. However, blood LDL cholesterol value cannot be satisfactorily controlled even with statin drugs in some cases, and it is further known that use of a statin at a high dose increases risk of rhabdomyolysis. From such an aspect, development of a drug based on an action mechanism different from that of statins and showing still higher efficacy and safety is desired. For example, by inhibiting cholesterol ester transfer protein (CETP), which has an action of transferring a cholesterol ester from HDL cholesterol to LDL cholesterol, very low density lipoprotein (VLDL) cholesterol, or the like, HDL cholesterol can be increased, and therefore researches on CETP inhibitor is being actively conducted.

It is known that PCSK9 (proprotein convertase subtilisin/kexin 9), which is a subtype of proprotein convertases (PCs) belonging to the serine protease family, binds with an LDL receptor on a hepatocyte membrane surface to promote transfer of the LDL receptor into the cell, then the LDL receptor is decomposed in the cell, therefore uptake of blood LDL cholesterol into the liver is inhibited, and blood LDL cholesterol level increases as a result. Accordingly, it is expected that by suppressing the production of PCSK9 or inhibiting the function of the PCSK9 protein of PCSK9, the amount of the LDL receptor can be increased, and as a result, the blood LDL cholesterol level is thereby reduced.

From such point of view as described above, researches on inhibition of the function of PCSK9 or suppression of the production of the same are being conducted. For example, attempts of inhibiting the function with monoclonal antibodies directed to PCSK9, suppression of the production of PCSK9 by RNA interference, and the like have been reported (Non-patent documents 1 to 3). There are also a report that berberine suppresses expression of PCSK9 in the HepG2 cell (Non-patent document 4), a report that 5-azacytidine, which is an annexin A2 activator, promotes binding of PCSK9 with annexin A2 to suppress decomposition of LDL receptor (Patent document 1), and the like.

As for compounds having a benzoxazole ring, various medical uses are known (Patent documents 2 to 10). However, any benzoxazole compounds having a PCSK9 production-suppressing action or inhibitory action against PCSK9 function have not been reported so far.

### Prior art references

### Patent documents

Patent document 1: International Patent Publication WO2009/143633
Patent document 2: Japanese Patent No. 4689960
Patent document 3: Japanese Patent Unexamined Publication (KOHYO) No. 2007-528387
Patent document 4: Japanese Patent Unexamined Publication (KOHYO) No. 2008-510827
Patent document 5: Japanese Patent Unexamined Publication (KOHYO) No. 2008-533012
Patent document 6: Japanese Patent No. 5301999
Patent document 7: Japanese Patent Unexamined Publication (KOHYO) No. 2009-530381
Patent document 8: Japanese Patent Unexamined Publication (KOHYO) No. 2010-530416
Patent document 9: Japanese Patent Unexamined Publication (KOHYO) No. 2011-511803
Patent document 10: International Patent Publication WO2014/37340
Non-patent document 1: Proc. Natl. Acad. Sci. USA., 106, pp.9820-9825, 2009
Non-patent document 2: J. Lipid Res., 48, pp.763-767, 2007
Non-patent document 3: Proc. Natl. Acad. Sci. Ursa., 105, pp.11915-11920, 2008
Non-patent document 4: Atherosclerosis, 201, pp.266-273, 2008

## Summary of the Invention

### Object to be Achieved by the Invention

An object of the present invention is to provide a novel compound that has a blood LDL cholesterol-reducing action, and is useful as an active ingredient of medicaments.

More specifically, the object of the present invention is to provide a novel compound having a PCSK9 production-suppressing action and useful as an active ingredient of a medicament having a blood LDL cholesterol-reducing action.

### Means for Achieving the Object

The inventors of the present invention conducted various researched in order to achieve the aforementioned object. As a result, they found that compounds represented by the following general formula (I) and salts thereof markedly suppress the expression of PCSK9, and are extremely useful as an active ingredient of a medicament having a blood LDL cholesterol-reducing action. The present invention was accomplished on the basis of the aforementioned finding.

The present invention thus provides a compound represented by the following general formula (I) :

[Formula 1]

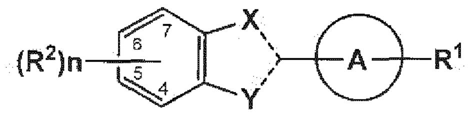

(I)

[in the formula, $R^1$ represents a $C_{1-6}$ alkyl group which may have a substituent, a halogen atom, hydroxy group, nitro group, or an amino group which may have a substituent,

A represents a phenylene group which may have a substituent, or a benzothiazole-diyl group which may have a substituent;

X represents $-CH(R^3)-$, $-C(R^3)=$, -O-, -NH-, -N=, or -S-;

Y represents -O-, -NH-, -N=, or -S-;

the broken lines ---- independently represent a single bond or double bond;

n represents an integer of 1 to 3;

when n is 1, $R^2$ represents a $C_{1-6}$ alkyl group which may have a substituent, a $C_{1-6}$ alkoxy group which may have a substituent, an amino group which may have a substituent, a halogen atom, or cyano group,

when n is 2, two of $R^2$ represent two of the same or different groups selected from the group consisting of a $C_{1-6}$ alkyl group which may have a substituent, a $C_{1-6}$ alkoxy group which may have a substituent, an amino group which may have a substituent, an acyl group which may have a substituent, carbonyl group, a carbamoyl group which may have a substituent, a halogen atom, hydroxy group, nitro group, cyano group, a carbon cyclic group which may have a substituent, and a heterocyclic group which may have a substituent, and two of $R^2$ may bind together to form a 5- to 7-membered carbon ring which may have a substituent, or a 5- to 7-membered heterocyclic ring which may have a substituent,

when n is 3, three of $R^2$ represent a combination of the groups mentioned above as $R^2$ for the compounds where n is 1, or n is 2; and

$R^3$ represents hydrogen atom, a $C_{1-6}$ alkyl group which may have a substituent, an acyl group which may have a substituent, a carbamoyl group which may have a substituent, or an amino group which may have a substituent, provided that the following compounds are excluded:

(1) those compounds wherein n is 2, X is -O-, Y is -N=, and two of $R^2$ are:,

- a combination of carboxamido group and fluorine atom,
- chlorine atom at the 5-position, and an amino group at the 6-position,
- chlorine atom at the 5-position, and nitro group at the 7-position,
- alkyl groups,
- hydroxy group at the 5-position, and bromine atom at the 7-position,
- 1,1-dimethylethyl group at the 6-position, and hydroxy group at the 7-position, or
- methyl group at the 5-position, and trifluorophenyl group at the 7-position;

(2) those compounds wherein n is 2, X is -S-, Y is -N=, and two of $R^2$ are:

- a combination of carboxamido group and fluorine atom,
- methyl group at the 4-position, and chlorine atom at the 5-position,
- hydroxy groups at the 4- and 7-positions,
- alkyl groups, or
- halogen atoms;

(3) those compounds wherein n is 2, X is -NH-, Y is -N=, and two of $R^2$ are:

- a combination of carboxamido group and fluorine atom,
- a combination of thiocarbonylamido group and fluorine atom,
- alkyl groups,
- alkoxy groups, or
- halogen atoms;

(4) those compounds wherein n is 2, X is -C($R^3$)=, Y is -NH-, $R^1$ is trifluoromethyl group, $R^3$ is hydrogen atom, and two of $R^2$ are:

- fluorine atoms,
- bromine atom at the 5-position, and fluorine atom at the 7-position,
- chlorine atom at the 4-position, and chlorine atom at the 6- or 7-position,
- chlorine atom at the 5-position, and fluorine atom at the 7-position,
- chlorine atom at the 5-position, and chlorine atom at the 7-position,
- chlorine atom at the 6-position, and fluorine atom at the 7-position, or
- chlorine atom at the 6-position, and chlorine atom at the 7-position, and

(5) those compounds wherein n is 2, X is -C($R^3$)=, Y is -NH-, $R^1$ is chlorine atom, $R^3$ is hydrogen atom, and two of $R^2$ are:,

- alkyl groups,
- a combination of an alkyl group and a halogen atom,
- chlorine atom at the 4-position, and chlorine atom at the 6-position, or
- fluorine atom at the 4-position, and fluorine atom at the 6-position],

or a salt thereof.

According to a preferred embodiment of the present invention, there is provided a compound represented by the aforementioned general formula (I), wherein: $R^1$ represents a $C_{1-6}$ alkyl group (this $C_{1-6}$ alkyl group may be substituted with one or two or more groups selected from the group consisting of hydroxy group and a halogen atom), a halogen atom, hydroxy group, nitro group, or an amino group; A represents a phenylene group or a benzothiazole-diyl group (these phenylene group and benzothiazole-diyl group may be substituted with one or two or more groups selected from the group consisting of a $C_{1-6}$ alkyl group (this $C_{1-6}$ alkyl group may be substituted with one or two or more groups selected from the group consisting of hydroxy group and a halogen atom), a halogen atom, hydroxy group, nitro group, and an amino group, as a substituent other than $R^1$);
X represents -C($R^3$)=, -O-, -NH-, or -S-;
Y represents -O-, -NH-, -N=, or -S-;
the broken lines ---- independently represents a single bond or double bond;
n represents an integer of 1 to 3;
when n is 1, $R^2$ represents a $C_{1-6}$ alkyl group, a $C_{1-6}$ alkoxy group, an amino group, a halogen atom, or cyano group, provided that these $C_{1-6}$ alkyl group, $C_{1-6}$ alkoxy group, and amino group may be substituted with one or two or more groups selected from the substituents of the substituent group A {a $C_{1-6}$ alkyl group, an acyl group (this acyl group may be substituted with a $C_{1-6}$ alkyl group), a halogen atom, an amino group, and a heterocyclic group},
when n is 2, two of $R^2$ represent two of the same or different groups selected from the group consisting of a $C_{1-6}$ alkyl group (this $C_{1-6}$alkyl group may be substituted with one or two or more halogen atoms), a $C_{1-6}$ alkoxy group (this $C_{1-6}$ alkoxy group may be substituted with one or two or more groups selected from the group consisting of an amino group and a carbon cyclic group), an amino group (this amino group may be substituted with one or two or more groups selected from the substituents of the substituent group B {a $C_{1-6}$ alkyl group (this $C_{1-6}$ alkyl group may be substituted with one or two or more groups selected from the group consisting of hydroxy group, an amino group, a phenyl group, acetylamino group, acetyloxy group, a saturated heterocyclic group which may be substituted with hydroxy group, a partially saturated

heterocyclic group, and an aromatic heterocyclic group), a carbon cyclic group (this carbon cyclic group may be substituted with a halogen atom), a heterocyclic group, and sulfonyl group (this sulfonyl group may be substituted with one or two or more groups selected from the group consisting of dimethylamino group, a phenyl group, and cyclopropyl group)}), an acyl group, carbonyl group, a carbamoyl group (these acyl group and carbamoyl group may be substituted with one or two or more groups selected from the substituents of the substituent group C {a $C_{1-6}$ alkyl group (this $C_{1-6}$ alkyl group may be substituted with one or two or more groups selected from the group consisting of hydroxy group, an amino group, carboxy group, methoxycarbonyl group, and ethoxycarbonyl group), a $C_{1-6}$ alkoxy group, a carbon cyclic group, and a heterocyclic group}), a halogen atom, hydroxy group, nitro group, cyano group, a carbon cyclic group, and a heterocyclic group (these carbon cyclic group and heterocyclic group may be substituted with one or two or more $C_{1-6}$ alkyl groups), two of $R^2$ may bind together to form a 5- to 7-membered carbon ring or heterocyclic ring, and the ring may have one or two or more groups selected from the substituents mentioned above as $R^2$ for the compounds where n is 2,
when n is 3, three of $R^2$ represent a combination of the groups mentioned above as $R^2$ for the compounds where n is 1, or n is 2;
$R^3$ represents hydrogen atom, a $C_{1-6}$ alkyl group, an acyl group, a carbamoyl group, or an amino group, and these $C_{1-6}$ alkyl group, acyl group, carbamoyl group, and amino group may have one or two or more groups selected from the substituents of the substituent group D {a $C_{1-6}$ alkyl group (this $C_{1-6}$ alkyl group may be substituted with one or two or more groups selected from the group consisting of carboxyl group, methoxycarbonyl group, benzylcarbamoyl group, a carbon cyclic group, and a heterocyclic group), a $C_{1-6}$ alkoxy group, a sulfonyl group (this sulfonyl group may be substituted with a $C_{1-6}$ alkyl group), a carbon cyclic group (this carbon cyclic group may be substituted with methoxyphenyl group), or a heterocyclic group (this heterocyclic group may be substituted with a phenyl group)}, or a salt thereof.

In the aforementioned general formula (I), it is more preferred that X is -O-,-S-, or -NH-, and Y is -N=, and it is further preferred that X is -O-, and Y is -N=. It is also preferred that X is -C($R^3$)=, and Y is -NH-, -S-, or -O-, and it is further preferred that X is -C($R^3$)=, and Y is -NH-.

According to a further preferred embodiment of the present invention, there is provided a compound represented by the following general formula (IA):

[Formula 2]

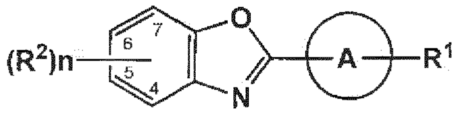

(IA)

($R^1$, A, n, and $R^2$ have the same meanings as those defined above, provided that when n is 2, those compounds having, as $R^2$,

- chlorine atom at the 5-position, and an amino group at the 6-position,
- chlorine atom at the 5-position, and nitro group at the 7-position,
- alkyl groups,
- hydroxy group at the 5-position, and bromine atom at the 7-position,
- 1,1-dimethylethyl group at the 6-position, and hydroxy group at the 7-position, or
- methyl group at the 5-position, and trifluorophenyl group at the 7-position are excluded), or a salt thereof.

In the aforementioned general formula (IA), it is preferred that
$R^1$ represents a $C_{1-6}$ alkyl group (this $C_{1-6}$ alkyl group may be substituted with one or two or more groups selected from the group consisting of hydroxy group and a halogen atom), a halogen atom, hydroxy group, nitro group, or an amino group;
A represents a phenylene group or a benzothiazole-diyl group (these phenylene group and benzothiazole-diyl group may be substituted with one or two or more groups selected from the group consisting of a $C_{1-6}$ alkyl group (this $C_{1-6}$ alkyl group may be substituted with one or two or more groups selected from the group consisting hydroxy group and a halogen atom), a halogen atom, hydroxy group, nitro group, and amino group, as a substituent other than $R^1$);
n represents an integer of 1 to 3;
when n is 1, $R^2$ represents a $C_{1-6}$ alkyl group, a $C_{1-6}$ alkoxy group, an amino group, a halogen atom, or cyano group, provided that these $C_{1-6}$ alkyl group, $C_{1-6}$ alkoxy group, and amino group may be substituted with one or two or more groups selected from the substituents of the substituent group A {a $C_{1-6}$ alkyl group, an acyl group (this acyl group may be substituted with a $C_{1-6}$ alkyl group), a halogen atom, an amino group, and a heterocyclic group},
when n is 2, two of $R^2$ represent two of the same or different groups selected from the group consisting of a $C_{1-6}$ alkyl group (this $C_{1-6}$ alkyl group may be substituted with one or two or more halogen atoms), a $C_{1-6}$ alkoxy group (this $C_{1-6}$ alkoxy group may be substituted with one or two or more groups selected from the group consisting of an amino group

and a carbon cyclic group), an amino group (this amino group may be substituted with one or two or more groups selected from the substituents of the substituent group B {a $C_{1-6}$ alkyl group (this $C_{1-6}$ alkyl group may be substituted with one or two or more groups selected from the group consisting of hydroxy group, an amino group, a phenyl group, acetylamino group, acetyloxy group, a saturated heterocyclic group which may be substituted with hydroxy group, a partially saturated heterocyclic group, and an aromatic heterocyclic group), a carbon cyclic group (this carbon cyclic group may be substituted with a halogen atom), a heterocyclic group, and a sulfonyl group (this sulfonyl group may be substituted with one or two or more groups selected from the group consisting of dimethylamino group, a phenyl group, and cyclopropyl group)}), an acyl group, carbonyl group, a carbamoyl group (these acyl group and carbamoyl group may be substituted with one or two or more groups selected from the substituents of the substituent group C {a $C_{1-6}$ alkyl group (this $C_{1-6}$ alkyl group may be substituted with one or two or more groups selected from the group consisting of hydroxy group, an amino group, carboxy group, methoxycarbonyl group, and ethoxycarbonyl group), a $C_{1-6}$ alkoxy group, a carbon cyclic group, and a heterocyclic group}), a halogen atom, hydroxy group, nitro group, cyano group, a carbon cyclic group, and a heterocyclic group (these carbon cyclic group and heterocyclic group may be substituted with one or two or more $C_{1-6}$ alkyl groups), two of $R^2$ may bind together to form a 5- to 7-membered carbon ring or heterocyclic ring, and the ring may have one or two or more groups selected from the substituents mentioned above as $R^2$ for the compounds where n is 2,

when n is 3, three of $R^2$ represent a combination of the groups mentioned above as $R^2$ for the compounds where n is 1, or n is 2;

$R^3$ represents hydrogen atom, a $C_{1-6}$ alkyl group, an acyl group, a carbamoyl group, or an amino group, and these $C_{1-6}$ alkyl group, acyl group, carbamoyl group, and amino group may have one or two or more groups selected from the substituents of the substituent group D {a $C_{1-6}$ alkyl group (this $C_{1-6}$ alkyl group may be substituted with one or two or more groups selected from the group consisting of carboxyl group, methoxycarbonyl group, benzylcarbamoyl group, a carbon cyclic group, and a heterocyclic group), a $C_{1-6}$ alkoxy group, a sulfonyl group (this sulfonyl group may be substituted with a $C_{1-6}$ alkyl group), a carbon cyclic group (this carbon cyclic group may be substituted with methoxyphenyl group), or a heterocyclic group (this heterocyclic group may be substituted with a phenyl group)}.

In the aforementioned general formula (IA), it is further preferred that A is a phenylene group (this phenylene group may be substituted with one or two or more groups selected from the group consisting of a $C_{1-6}$ alkyl group (this $C_{1-6}$ alkyl group may be substituted with one or two or more groups selected from the group consisting of hydroxy group and a halogen atom), a halogen atom, hydroxy group, nitro group, and an amino group, as a substituent other than $R^1$), and $R^1$ is chlorine atom or trifluoromethyl group, and it is particularly preferred that A is unsubstituted 1,4-phenylene group, and $R^1$ is trifluoromethyl group.

According to another preferred embodiment of the present invention, there is provided a compound represented by the following general formula (IB):

[Formula 3]

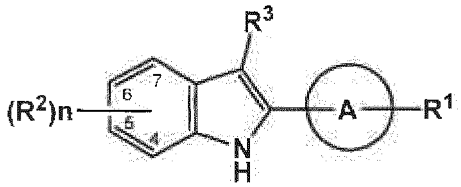

(IB)

($R^1$, A, n, $R^2$, and $R^3$ have the same meanings as those defined above, provided that when n is 2, and $R^3$ is hydrogen atom, the following compounds are excluded:

(a) those compounds wherein $R^1$ is trifluoromethyl group, and two of $R^2$ are:

- fluorine atoms,
- bromine atom at the 5-position, and fluorine atom at the 7-position,
- chlorine atom at the 4-position, and chlorine atom at the 6- or 7-position,
- chlorine atom at the 5-position, and fluorine atom at the 7-position,
- chlorine atom at the 5-position, and chlorine atom at the 7-position,
- chlorine atom at the 6-position, and fluorine atom at the 7-position, or
- chlorine atom at the 6-position, and chlorine atom at the 7-position; and

(b) those compounds wherein $R^1$ is chlorine atom, and two of $R^2$ are:

- alkyl groups,

- a combination of an alkyl group and a halogen atom,
- chlorine atom at the 4-position, and chlorine atom at the 6-position, or
- fluorine atom at the 4-position, and fluorine atom at the 6-position), or a salt thereof.

In the aforementioned general formula (IB), it is further preferred that:

$R^1$ represents a $C_{1-6}$ alkyl group (this $C_{1-6}$ alkyl group may be substituted with one or two or more groups selected from the group consisting of hydroxy group and a halogen atom), a halogen atom, hydroxy group, nitro group, or an amino group:

A represents a phenylene group or a benzothiazole-diyl group (these phenylene group and benzothiazole-diyl group may be substituted with one or two or more groups selected from the group consisting of a $C_{1-6}$ alkyl group (this $C_{1-6}$ alkyl group may be substituted with one or two or more groups selected from the group consisting of hydroxy group and a halogen atom), a halogen atom, hydroxy group, nitro group, and amino group, as a substituent other than $R^1$);

n represents an integer of 1 to 3;

when n is 1, $R^2$ represents a $C_{1-6}$ alkyl group, a $C_{1-6}$ alkoxy group, an amino group, a halogen atom, or cyano group, provided that these $C_{1-6}$ alkyl group, $C_{1-6}$ alkoxy group, and amino group may be substituted with one or two or more groups selected from the substituents of the substituent group A {a $C_{1-6}$ alkyl group, an acyl group (this acyl group may be substituted with a $C_{1-6}$ alkyl group), a halogen atom, an amino group, and a heterocyclic group},

when n is 2, two of $R^2$ represent two of the same or different groups selected from the group consisting of a $C_{1-6}$ alkyl group (this $C_{1-6}$alkyl group may be substituted with one or two or more halogen atoms), a $C_{1-6}$ alkoxy group (this $C_{1-6}$ alkoxy group may be substituted with one or two or more groups selected from the group consisting of an amino group and a carbon cyclic group), an amino group (this amino group may be substituted with one or two or more groups selected from the substituents of the substituent group B {a $C_{1-6}$ alkyl group (this $C_{1-6}$ alkyl group may be substituted with one or two or more groups selected from hydroxy group, an amino group, a phenyl group, acetylamino group, acetyloxy group, a saturated heterocyclic group which may be substituted with hydroxy group, a partially saturated heterocyclic group, and an aromatic heterocyclic group), a carbon cyclic group (this carbon cyclic group may be substituted with a halogen atom), a heterocyclic group, and a sulfonyl group (this sulfonyl group may be substituted with one or two or more groups selected from the group consisting of dimethylamino group, a phenyl group, and cyclopropyl group)}), an acyl group, carbonyl group, a carbamoyl group (these acyl group and carbamoyl group may be substituted with one or two or more groups selected from the substituents of the substituent group C {a $C_{1-6}$ alkyl group (this $C_{1-6}$ alkyl group may be substituted with one or two or more groups selected from the group consisting of hydroxy group, an amino group, carboxy group, methoxycarbonyl group, and ethoxycarbonyl group), a $C_{1-6}$ alkoxy group, a carbon cyclic group, and a heterocyclic group}), a halogen atom, hydroxy group, nitro group, cyano group, a carbon cyclic group, and a heterocyclic group (these carbon cyclic group and heterocyclic group may be substituted with one or two or more $C_{1-6}$ alkyl groups), two of $R^2$ may bind together to form a 5- to 7-membered carbon ring or heterocyclic ring, and the ring may have one or two or more groups selected from the substituents mentioned above as $R^2$ for the compounds where n is 2, when n is 3, three of $R^2$ represent a combination of the groups mentioned above as $R^2$ for the compounds where n is 1, or n is 2;

$R^3$ represents hydrogen atom, a $C_{1-6}$ alkyl group, an acyl group, a carbamoyl group, or an amino group, and these $C_{1-6}$ alkyl group, acyl group, carbamoyl group, and amino group may have one or two or more groups selected from the substituents of the substituent group D {a $C_{1-6}$ alkyl group (this $C_{1-6}$ alkyl group may be substituted with one or two or more groups selected from the group consisting of carboxyl group, methoxycarbonyl group, benzylcarbamoyl group, a carbon cyclic group, and a heterocyclic group), a $C_{1-6}$ alkoxy group, a sulfonyl group (this sulfonyl group may be substituted with a $C_{1-6}$ alkyl group), a carbon cyclic group (this carbon cyclic group may be substituted with methoxyphenyl group), or a heterocyclic group (this heterocyclic group may be substituted with a phenyl group)}.

In the aforementioned general formula (IB), it is further preferred that A is a phenylene group (this phenylene group may be substituted with one or two or more groups selected from the group consisting of a $C_{1-6}$ alkyl group (this $C_{1-6}$ alkyl group may be substituted with one or two or more groups selected from the group consisting of hydroxy group and a halogen atom), a halogen atom, hydroxy group, nitro group, and amino group, as a substituent other than $R^1$), and $R^1$ is chlorine atom or trifluoxomethyl group, and it is particularly preferred that A is unsubstituted 1,4-phenylene group, and $R^1$ is chlorine atom or trifluoromethyl group.

As another aspect of the present invention, there is provided a medicament containing a compound represented by the following general formula (I):

[Formula 4]

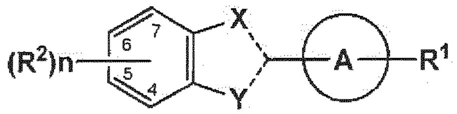

(I)

[in the formula, $R^1$ represents a $C_{1-6}$ alkyl group which may have a substituent, a halogen atom, hydroxy group, nitro group, or an amino group;

A represents a phenylene group which may have a substituent, or a benzothiazole-diyl group which may have a substituent;

X represents $-CH(R^3)-$, $-C(R^3)=$, -O-, -NH-, -N=, or -S-;

Y represents -O-, -NH-, -N=, or -S-;

the broken lines ---- independently represent a single bond or double bond;

n represents an integer of 1 to 3;

when n is 1, $R^2$ represents a $C_{1-6}$ alkyl group which may have a substituent, a $C_{1-6}$ alkoxy group which may have a substituent, an amino group which may have a substituent, a halogen atom, or cyano group,

when n is 2, two of $R^2$ represent two of the same or different groups selected from the group consisting of a $C_{1-6}$ alkyl group which may have a substituent, a $C_{1-6}$ alkoxy group which may have a substituent, an amino group which may have a substituent, an acyl group which may have a substituent, carbonyl group, a carbamoyl group which may have a substituent, a halogen atom, hydroxy group, nitro group, cyano group, a carbon cyclic group which may have a substituent, and a heterocyclic group which may have a substituent, two of $R^2$ may bind together to form a 5- to 7-membered carbon ring which may have a substituent, or a 5- to 7-membered heterocyclic ring which may have a substituent,

when n is 3, three of $R^2$ represent a combination of the groups mentioned above as $R^2$ for the compounds where n is 1, or n is 2;

$R^3$ represents hydrogen atom, a $C_{1-6}$ alkyl group which may have a substituent, an acyl group which may have a substituent, a carbamoyl group which may have a substituent, or an amino group which may have a substituent), or a salt thereof as an active ingredient.

The medicament of the present invention has a blood LDL cholesterol-reducing action, and accordingly, the medicament is useful for therapeutic and/or prophylactic treatment of high LDL cholesterolemia, and can be used for prophylactic and/or therapeutic treatment of arteriosclerosis, lipidosis, or the like accompanied by a high LDL cholesterol condition.

As further aspects of the present invention, there are provided a PCSK9 production-suppressing agent containing a compound represented by the aforementioned general formula (I) or a salt thereof as an active ingredient; and a blood LDL cholesterol-reducing agent containing a compound represented by the aforementioned general formula (I) or a salt thereof as an active ingredient. The present invention also provides use of a compound represented by the aforementioned general formula (I) or a salt thereof for manufacture of the aforementioned medicament; use of a compound represented by the aforementioned general formula (I) or a salt thereof for manufacture of the aforementioned PCSK9 production-suppressing agent; and use of a compound represented by the aforementioned general formula (I) or a salt thereof for manufacture of the aforementioned blood LDL cholesterol-reducing agent.

As further aspects of the present invention, there are provided a method for prophylactic and/or therapeutic treatment of high LDL cholesterolemia of a mammal including human, which comprises the step of administering a prophylactically and/or therapeutically effective amount of a compound represented by the aforementioned general formula (I) or a salt thereof to the mammal; a method for prophylactic and/or therapeutic treatment of arteriosclerosis, lipidosis, or the like accompanied by high LDL cholesterolemia in a mammal including human, which comprises the step of administering a prophylactically and/or therapeutically effective amount of a compound represented by the aforementioned general formula (I) or a salt thereof to the mammal; a method for reducing blood LDL cholesterol in a mammal including human, which comprises the step of administering an effective amount of a compound represented by the aforementioned general formula (I) or a salt thereof to the mammal; and a method for suppressing expression of PCSK9 in a mammal including human, which comprises the step of administering an effective amount of a compound represented by the aforementioned general formula (I) or a salt thereof to the mammal.

## Effect of the Invention

The compounds represented by the general formula (I) and salts thereof of the present invention have an action of reducing blood LDL cholesterol by suppressing expression of PCSK9. Therefore, the medicament of the present

invention containing a compound represented by the general formula (I) or a salt thereof as an active ingredient is useful for prophylactic and/or therapeutic treatment of, besides high LDL cholesterolemia, lipidosis, arteriosclerosis, or the like accompanied by a high LDL cholesterol condition.

Modes for Carrying out the Invention

In the present specification, the term "alkyl group" refers to a linear, branched, or cyclic alkyl group, or an alkyl group consisting of a combination of these. The same shall apply to an alkyl moiety of a substituent having the alkyl moiety (alkoxy group and the like). The halogen atom may be fluorine atom, chlorine atom, bromine atom, or iodine atom. When an expression "which may have a substituent" is used for a certain functional group in this specification, it means that the functional group may have an arbitrary number of substituent at substitutable position of the functional group. When the functional group has two or more substituents, they may be the same or different.

In the general formula (1), $R^1$ represents a $C_{1-6}$ alkyl group which may have a substituent, a halogen atom, hydroxy group, nitro group, or an amino group. When this $C_{1-6}$ alkyl group has a substituent, the substituent is preferably selected from the group consisting of hydroxy group and a halogen atom, and a $C_{1-6}$ alkyl group substituted with fluorine atom is more preferred. Particularly preferred examples include, for example, trifluoromethyl group, and the like, As the halogen atom represented by $R^1$, chlorine atom or fluorine atom is preferred, and fluorine atom is more preferred. As the amino group represented by $R^1$, besides unsubstituted amino group, a dialkylamino group, a monoalkylamino group, an acylamino group, and the like can be used. As $R^1$, chlorine atom, fluorine atom, trifluoromethyl group, and the like are particularly preferred, and trifluoromethyl group is most preferred.

A represents a phenylene group which may have a substituent, or a benzothiazole-diyl group which may have a substituent. As the phenylene group, 1,4-phenylene group (p-phenylene group) is preferred, and as the benzothiazole-diyl group, 2,5-benzothiazole-diyl group and 2,6-benzothiazole-diyl group are preferred. The phenylene group and the benzothiazole-diyl group may have one or two or more groups selected from the group consisting of a $C_{1-6}$ alkyl group (this $C_{1-6}$ alkyl group may be substituted with one or two or more groups selected from the group consisting of hydroxy group and a halogen atom), a halogen atom, hydroxy group, nitro group, and an amino group, as a substituent other than $R^1$, but it is preferred that they do not have any substituent other than $R^1$. As A, a phenylene group is preferred, and 1,4-phenylene group is more preferred. It is particularly preferred that A is 1,4-phenylene group not having any substituent other than $R^1$, and $R^1$ is trifluoromethyl group.

X represents -CH($R^3$)-, -C($R^3$)=, -O-, -NH-, -N=, or -S-, Y represents -O-, -NH-, N=, or -S-, and the broken lines (----) independently represent a single bond or double bond. X preferably represents -C($R^3$)=, -O-, -NH-, or -S-, and Y preferably represents O-, -NH-, -N=, or -S-. In the aforementioned general formula (I), it is more preferred that X is -O-, -S-, or -NH-, and Y is -N=, and it is further preferred that X is -O-, and Y is -N=, which means that the compounds are compounds of the general formula (IA). It is also preferred that X is -C($R^3$)=, and Y is -NH-, -S-, or -O-, and it is further preferred that X is -C($R^3$)=, and Y is -NH-, which means that the compounds are compounds of the general formula (IB).

The symbol n represents an integer of 1 to 3. The expression that n is 1, 2, or 3 means that 1, 2, or 3 of groups $R^2$ exist at arbitrary positions on the ring, respectively, and when 2 or 3 of the groups $R^2$ exist, they may be the same or different.

When n is 1, $R^2$ represents hydrogen atom, a $C_{1-6}$ alkyl group which may have a substituent, a $C_{1-6}$ alkoxy group which may have a substituent, an amino group which may have a substituent, a halogen atom, or cyano group. When the $C_{1-6}$ alkyl group, $C_{1-6}$ alkoxy group, or amino group represented by $R^2$ has a substituent, it may be substituted with one or two or more groups selected from, for example, the substituents of the substituent group A {a $C_{1-6}$ alkyl group, an acyl group (this acyl group may be substituted with a $C_{1-6}$ alkyl group), and a heterocyclic group}.

The acyl group mentioned in the present specification may be an alkylcarbonyl group or an arylcarbonyl group, and for example, such an alkylcarbonyl group as acetyl group and propanoyl group, or such an arylcarbonyl group as benzoyl group can be used. The alkyl moiety of the alkylcarbonyl group may have one or two or more arbitrary substituents such as fluorine atom. For example, trifluoroacetyl group, and the like may be used. The aryl ring of the arylcarbonyl group such as benzoyl group may have one or two or more arbitrary substituents such as an alkyl group, an alkoxy group, an amino group, and a halogen atom. For example, 4-methoxybenzoyl group, 4-aminobenzoyl group, 4-trifluoromethylbenzoyl group, and the like can be used.

As the heterocyclic group mentioned in the present specification, a saturated heterocyclic group, a partially saturated heterocyclic group, or an aromatic heterocyclic group can be used. As the saturated or partially saturated heterocyclic group, for example, a 5- to 7-membered saturated heterocyclic group or partially saturated heterocyclic group having 1 to 3 heteroatoms selected from the group consisting of nitrogen atom, oxygen atom, and sulfur atom is preferred, and more specific examples include pyrrolidino group, 2-pyrrolidinone group, 2,5-pyrrolidinedione group, furyl group, imidazolidinyl group, pyrazolidinyl group, oxathiolanyl group, piperidino group, piperazinyl group, tetrahydropyranyl group, morpholino group, 3-morpholinone group, azepinyl group, oxepinyl group, diazepinyl group, isoindole-1,3-(2H)-dione group, and the like, but not limited to these examples. Among these, pyrrolidino, 2-pyrrolidinone, 2,5-pyrrolidinedione, furyl, piperazinyl, morpholino, 3-morpholinone groups, and the like are preferred. As the aromatic heterocyclic group, for example, a monocyclic or polycyclic aromatic heterocyclic group containing 1 or 2 heteroatoms selected from the group consisting of nitrogen atom, oxygen atom, and sulfur atom is preferred. In the case of a polycyclic aromatic heterocyclic group, it is preferred that each ring is a 5- or 6-membered ring. More specific examples include pyrrolyl group, furyl group, thienyl group, imidazolyl group, pyrazolyl group, oxazolyl group, isoxazolyl group, thiazolyl group, pyridyl group, pyrazinyl group, pyrimidinyl group, pyridazinyl group, indolyl group, benzimidazolyl group, benzoxazolyl group, benzothiazolyl group, quinolyl group, isoquinolyl group, quinazolyl group, and the like, but not limited to these examples. Among these, pyridyl group, pyrazinyl group, and isoxazolyl group are preferred. As the partially saturated heterocyclic group, a group corresponding to any of the aforementioned aromatic heterocyclic groups, but which is partially saturated, can be used. Examples include, for example, dihydropyridyl group, tetrahydroquinolyl group, and the like, but not limited to these examples.

When n is 1, preferred examples of $R^2$ include amino group, methylamino group, dimethylamino group, acetylamino group, chlorine atom, fluorine atom, cyano group, aminomethyl group, monomethylaminomethyl group, trifluoromethyl group, methoxy group, tetrahydrofurylmethoxy group, and the like, but not limited to these examples. When n is 1, preferred examples of $R^2$ in the general formula (IA) include amino group, methylamino group, dimethylamino group, acetylamino group, chlorine atom, fluorine atom, cyano group, aminomethyl group, monomethylaminomethyl group, trifluoromethyl group, methoxy group, tetrahydrofurylmethoxy group, and the like, and when n is 1, preferred examples of $R^2$ in the general formula (IB) include amino group, methylamino group, dimethylamino group, methoxy group, and the like.

When n is 2, groups $R^2$ represent two of the same or different groups selected from the group consisting of a $C_{1-6}$ alkyl group which may have a substituent, a $C_{1-6}$ alkoxy group which may have a substituent, an amino group which may have a substituent, an acyl group which may have a substituent, carbonyl group, a carbamoyl group which may have a substituent, a halogen atom, hydroxy group, nitro group, cyano group, a carbon cyclic group which may have a substituent, and a heterocyclic group which may have a substituent.

More specifically, when n is 2, the $C_{1-6}$ alkyl group represented by $R^2$ may be substituted with one or two or more halogen atoms, and the $C_{1-6}$ alkoxy group represented by $R^2$ may be substituted with one or two or more groups selected from the group consisting of an amino group and a carbon cyclic group. The amino group represented by $R^2$ may be substituted with one or two or more groups selected from the substituents of the substituent group B {a $C_{1-6}$ alkyl group (this $C_{1-6}$ alkyl group may be substituted with one or two or more groups selected from the group consisting of hydroxy group, an amino group, a phenyl group, acetylamino group, acetyloxy group, a saturated heterocyclic group which may be substituted with hydroxy group, a partially saturated heterocyclic group, and an aromatic heterocyclic group), a carbon cyclic group (this carbon cyclic group may be substituted with a halogen atom), a heterocyclic group, and a sulfonyl group (this sulfonyl group may be substituted with one or two or more groups selected from the group consisting of dimethylamino group, a phenyl group, and cyclopropyl group)}. The acyl group and carbamoyl group may be substituted with one or two or more groups selected from the substituents of the substituent group C {a $C_{1-6}$ alkyl group (this $C_{1-6}$ alkyl group may be substituted with one or two or more groups selected from the group consisting of hydroxy group, an amino group, carboxy group, methoxycarbonyl group, and ethoxycarbonyl group), a $C_{1-6}$ alkoxy group, a carbon cyclic group, and a heterocyclic group}. The carbon cyclic group and heterocyclic group may be substituted with one or two or more $C_{1-6}$ alkyl groups.

As the carbon cyclic group mentioned in the present specification, a saturated carbon cyclic group, a partially saturated carbon cyclic group, or an aromatic carbon cyclic group can be used. Examples of the saturated carbon cyclic group include, for example, a $C_{3-8}$ cycloalkyl group, and more specifically, cyclopropyl group and cyclopentyl group are preferred. Examples of the aromatic carbon cyclic group include a monocyclic or polycyclic aromatic carbon cyclic group having 6 to 20 carbon atoms, and more specific examples include, for example, phenyl group, 1-indenyl group, 1-naphthyl group, 2-naphthyl group, 2-anthryl group, 1-acenaphthenyl group, and the like. Among these, phenyl group is preferred. As the partially saturated carbon cyclic group, any one of the aforementioned aromatic carbon cyclic groups, but which is partially saturated, can be used. For example, dihydrophenyl group, tetrahydronaphthyl group, and the like can be used, but not limited to these examples.

When n is 2, two of $R^2$ may bind together to form a 5- to 7-membered carbon ring or heterocyclic ring. The 5- to 7-membered carbon ring or heterocyclic ring formed by 2 of the groups $R^2$ binding together may be a partially saturated ring or an aromatic ring. When 2 of the groups $R^2$ bind together to form a 5- to 7-membered carbon ring or heterocyclic ring, the ring formed from 2 of the groups $R^2$ constitutes a ring condensed to the benzene ring substituted with 2 of the groups $R^2$. Examples of the carbon ring formed by 2 of the groups $R^2$ include benzene ring, dihydrobenzene ring, tetrahydrobenzene ring, and the like, and it is preferred that, as the heterocyclic ring formed by 2 of the groups $R^2$, for example, a 5- to 7-membered partially saturated heterocyclic ring or aromatic heterocyclic ring containing 1 to 3 heteroatoms selected from the group consisting of nitrogen atom, oxygen atom, and sulfur atom is formed. Examples include thiophene ring, furan ring, pyran ring, pyrrole ring, imidazole ring, isothiazole ring, isoxazole ring, pyridine ring, pyrimidine ring, pyrazine ring, pyridazine ring, and a partially saturated ring of these rings. Preferred examples of the 5- to 7-membered carbon ring or heterocyclic ring formed by two of $R^2$ binding together when n is 2 are mentioned below, but they are not limited to these examples.

[Formula 5]

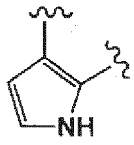
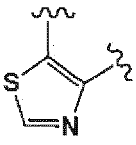
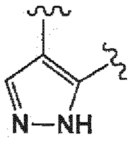
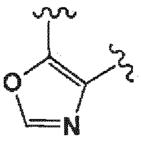
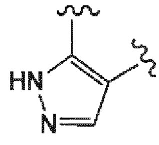
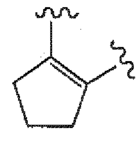
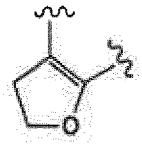

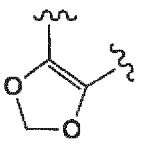
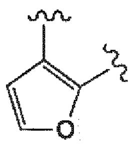
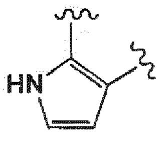
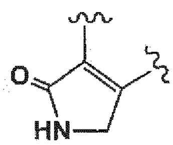
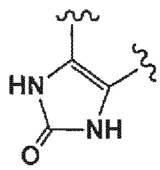
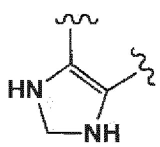

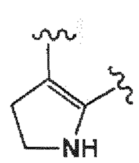
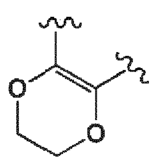
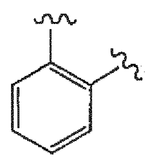
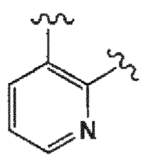
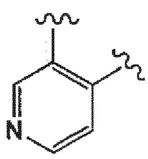
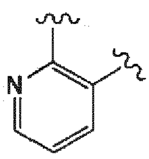

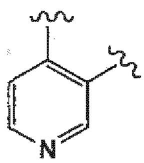
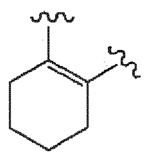
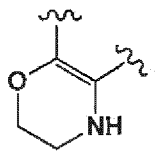

The 5- to 7-membered carbon ring or heterocyclic ring formed by 2 of the groups $R^2$ binding together may have, on the ring, one or two or more groups selected from the substituents mentioned above as $R^2$ of the compounds where n is 2. More specifically, the carbon ring or heterocyclic ring formed by 2 of the groups $R^2$ may be substituted with one or two or more groups selected from the group consisting of, for example, a $C_{1-6}$ alkyl group (this $C_{1-6}$ alkyl group may be substituted with one or two or more groups selected from the group consisting of hydroxy group, methylamino group, and dimethylamino group), acetyl group, carbonyl group (this carbonyl group may be substituted with an alkoxy group), an amino group (this amino group may be substituted with a $C_{1-6}$ alkyl group), a phenyl group (this phenyl group may be substituted with a halogen atom), and a heterocyclic group (piperidinyl group, morpholinyl group, and the like). More specific examples of the substituent on the ring of the 5- to 7-membered carbon ring or heterocyclic ring formed by 2 of the groups $R^2$ binding together include methyl group, ethyl group, isopropyl group, amino group, methylamino group, dimethylamino group, diethylamino group, methylaminomethyl group, dimethylaminomethyl group, (ethyl)(methyl)amino group, methoxy group, phenyl group, p-chlorophenyl group, acetyl group, methoxycarbonyl group, ethoxycarbonyl group, hydroxymethyl group, morpholino group, pyrrolidino group, piperidino group, oxo group, and the like, but not limited to these examples.

Examples of the combination of two of $R^2$ in the compounds where n is 2 include, for example, a combination of two alkyl groups, combination of two halogen atoms (for example, combination of two fluorine atoms, combination of fluorine atom and chlorine atom, combination of two chlorine atoms, and the like), combination of a halogen atom and trifluoromethyl group, combination of a halogen atom and cyano group, combination of a halogen atom and an amino group (this amino group may have a substituent), combination of trifluoromethyl group and an amino group (this amino group may have a substituent), combination of a halogen atom and nitro group, combination of a halogen atom and an alkoxy group (this alkoxy group may have a substituent), combination of two trifluoromethyl groups, combination of a halogen atom and hydroxy group, combination of trifluoromethyl group and hydroxy group, combination of a halogen

atom and an alkyl group, combination of trifluoromethyl group and cyano group, combination of cyano group and methoxy group (this methoxy group may have a substituent), combination of cyano group and an amino group (this amino group may have a substituent), combination of trifluoromethyl group and a $C_{1-6}$ alkyl group, combination of trifluoromethyl group and an alkoxy group (this alkoxy group may have a substituent), combination of trifluoromethyl group and a saturated heterocyclic group (this saturated heterocyclic group may have a substituent), combination of a halogen atom and a saturated heterocyclic group (this saturated heterocyclic group may have a substituent), and the like, but not limited to these examples.

Examples of the combination of two of $R^2$ in the compounds of the formula (IA) wherein n is 2 include a combination of two halogen atoms, combination of a halogen atom and trifluoromethyl group, combination of a halogen atom and cyano group, combination of a halogen atom and an amino group (this amino group may have a substituent), combination of trifluoromethyl group and an amino group (this amino group may have a substituent), combination of a halogen atom and nitro group, combination of a halogen atom and an alkoxy group (this alkoxy group may have a substituent), combination of two trifluoromethyl groups, combination of a halogen atom and hydroxy group, combination of trifluoromethyl group and hydroxy group, combination of a halogen atom and an alkyl group, combination of trifluoromethyl group and cyano group, combination of cyano group and methoxy group (this methoxy group may have a substituent), combination of cyano group and an amino group (this amino group may have a substituent), combination of trifluoromethyl group and a $C_{1-6}$ alkyl group, combination of trifluoromethyl group and an alkoxy group (this alkoxy group may have a substituent), combination of trifluoromethyl group and a saturated heterocyclic group (this saturated heterocyclic group may have a substituent), combination of a halogen atom and a saturated heterocyclic group (this saturated heterocyclic group may have a substituent), and the like, but not limited to these examples. Examples of the combination of two of $R^2$ in the compounds of the formula (IB) where n is 2 include a combination of two alkyl groups, combination of two halogen atoms, and combination of a halogen atom and an alkoxy group (this alkoxy group may have a substituent), but not limited to these examples.

When n is 3, the groups $R^2$ represent an arbitrary combination of $R^2$ explained for the compounds wherein n is 1 (except for the compounds where $R^2$ is hydrogen atom) and $R^2$ explained for the compounds wherein n is 2. Examples include, for example, a combination of three halogen atoms (for example, combination of two fluorine atoms and one chlorine atom and the like), combination of a halogen atom and an amino group (this amino group may have a substituent), combination of a halogen atom, cyano group, and an amino group (this amino group may have a substituent), combination of a halogen atom, cyano group, and an alkoxy group (this alkoxy group may have a substituent), and the like, but not limited to these examples.

$R^3$ represents hydrogen atom, a $C_{1-6}$ alkyl group which may have a substituent, an acyl group which may have a substituent, a carbamoyl group which may have a substituent, or an amino group which may have a substituent. These $C_{1-6}$ alkyl group, acyl group, carbamoyl group, and amino group may have one or two or more groups selected from the substituents of the substituent group D {a $C_{1-6}$ alkyl group (this $C_{1-6}$ alkyl group may be substituted with one or two or more groups selected from the group consisting of carboxyl group, methoxycarbonyl group, benzylcarbamoyl group, a carbon cyclic group, and a heterocyclic group), a $C_{1-6}$ alkoxy group, a sulfonyl group (this sulfonyl group may be substituted with a $C_{1-6}$ alkyl group), a carbon cyclic group (this carbon cyclic group may be substituted with methoxyphenyl group), and a heterocyclic group (this heterocyclic group may be substituted with a phenyl group)}. Examples of $R^3$ include, for example, a $C_{1-6}$ alkyl group substituted with a phenyl group (phenethyl group, benzyl group, and the like), an amino group substituted with an acyl group, an amino group substituted with sulfo group, and the like, and examples of the acyl group that can substitute on the amino group include an alkanoyl group which may have a substituent, carbonyl group substituted with a heterocyclic group which may have a substituent, carbonyl group substituted with an amino group which may have a substituent, a benzoyl group which may have a substituent, and the like, but not limited to these examples.

The compounds represented by the general formula (I) may form an acid addition salt or a base addition salt depending on the type of substituent. Examples of the acid addition salt include, for example, mineral acid salts such as hydrochloride, hydrobromide, hydroiodide, sulfate, nitrate, and phosphate, and organic acid salts such as methanesulfonate (mesylate), p-toluenesulfonate, benzoate, maleate, citrate, fumarate, tartrate, and acetate, but not limited to these examples.

The compounds of the present invention represented by the general formula (I) and salts thereof may form a hydrate or solvate, and these substances also fall within the scope of the present invention. Examples of solvent that constitutes the solvate include, for example, ethanol, hexane, and ethyl acetate, but not limited to these examples. The compounds represented by the general formula (I), salts thereof, hydrates thereof, and solvates thereof may be provided as a crystalline substance, and such a substance in any crystalline form of course fall within the scope of the present invention. The compounds of the present invention represented by the general formula (I) may exist as a stereoisomer based on an asymmetric carbon or double bond, and isomers such as optical isomers in a pure form and diastereoisomers, as well as arbitrary mixtures of isomers, racemates, and the like fall within the scope of the present invention.

Among the compounds of the present invention, the compounds having the benzoxazole nucleus can be

prepared by, for example, the methods A to C mentioned below, but the preparation method is not limited to these examples.

[Formula 6]

Method A

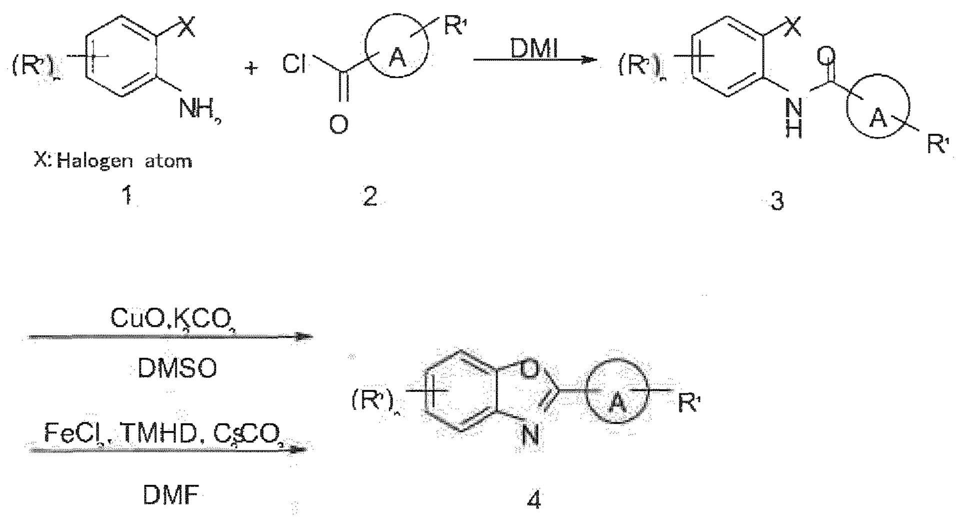


X: Halogen atom

1 2 3

4

By stirring a 2-halogen (fluoro, bromo, iodo)-substituted aniline compound (1) and a substituted benzoic acid chloride (2) with heating in, for example, 1,3-dimethylimidazolidinone, an amide compound (3) can be obtained. As the reaction solvent, besides 1,3-dimethylimidazolidinone, chloroform, tetrahydrofuran, ethyl acetate, benzene, pyridine, and the like can also be used. By stirring the resulting amide compound (3) with a catalytic amount of cupric oxide (nanoparticles) and excess amount of potassium carbonate with heating in dimethyl sulfoxide according to the method of Prasenjit et al. (J. Org. Chem., 74, 8719, 2009), an objective benzoxazole compound (4) can be obtained. As the reaction solvent, N,N-dimethylformamide, toluene, 1,4-dioxane, or the like can also be used instead of dimethyl sulfoxide. The amide compound (3) can also be converted into the objective substance (4) by stirring the amide compound (3) with heating in the presence of a catalytic amount of ferric chloride, 2,2,6,6-tetramethyl-3,5-heptanedione (TMHD), and excess amount of cesium carbonate in N,N-dimethylformamide according the method of Julien et al. (Org. Lett., 10(13), 2665, 2008).

[Formula 7]

Method B

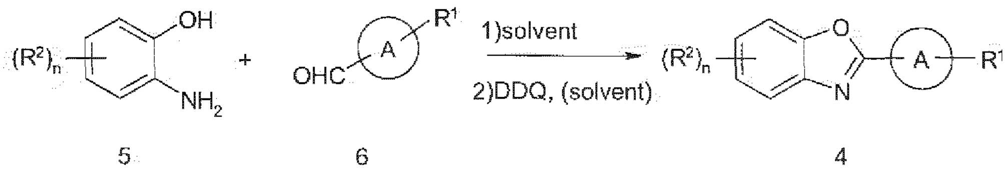


5 6 4

By stirring a 2-aminophenol compound (5) and a substituted benzaldehyde (6) in a solvent such as methanol, ethanol, N,N-dimethylformamide, or 1,4-dioxane with heating, then adding a solvent if needed, adding 2,3-dichloro-5,6-dicyano-1,4-benzoquinone (DDQ), and stirring the resulting mixture at room temperature according to the method of Chang et al. (US2003/0148387), an objective benzoxazole compound (4) can be obtained. As reactions similar to the aforementioned reaction, the reaction can also be performed under the conditions that stirring with heating is performed in chlorobenzene using a combination of N,N-dimethylformamide and iodine, or stirring with heating is performed in ethanol, then sodium cyanide in N,N-dimethylformamide is added, and the resulting mixture is stirred at room temperature.

[Formula 8]

Method C

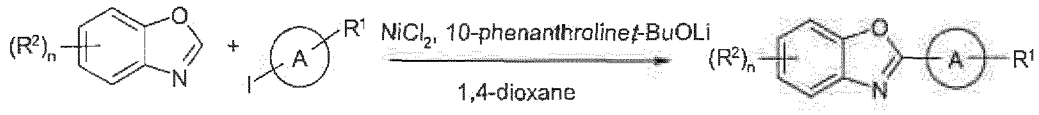


7 8 9

By stirring a 2-unsubstituted benzoxazole compound (7) and an iodo-substituted heterocyclic compound (8) with heating in 1,4-dioxane in the presence of nickel chloride, 1,10-phenanthroline, and lithium t-butoxide, an objective 2-substituted benzoxazole compound (9) can be obtained. In this reaction, as the reaction solvent, dimethoxyethane, toluene, N,N-dimethylformamide, or the like can be used instead of 1,4-dioxane, as the metal catalyst, bis(1,5-cyclooctadiene)nickel(0), nickel acetate tetrahydrate, or the like can be used instead of nickel chloride, and as the ligand, 1,2-bis(diphenylphosphino)ethane, 1,3-bis(diphenylphosphino)propane, 1,4-bis-(diphenylphosphino)butane, 1,1'-bis-(diphenylphosphino)ferrocene, xanthophos, or the like can be used instead of 1,10-phenanthroline. As the base, barium t-butoxide, magnesium t-butoxide, or the like can also be used instead of lithium t-butoxide.

The compounds having the indole nucleus can be prepared by, for example, the following method D or E.

[Formula 9]

Method D

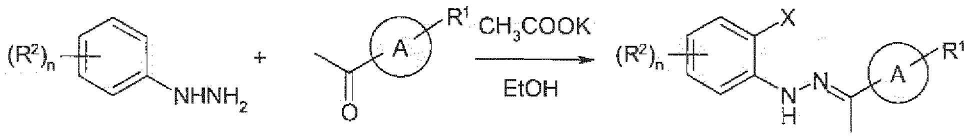


10 11 12

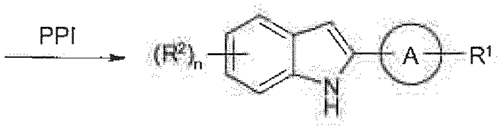


13

After obtaining a hydrazone (12) by adding a hydrazine compound (10) and an acetophenone compound (12) to ethanol and stirring the resulting mixture at room temperature according to the Fischer indole synthesis method (US2011/0071150) of Alam et al., the hydrazone (12) can be converted into an indole compound (13) by stirring it with heating under acidic conditions using polyphosphoric acid or the like. In this reaction, as the reaction solvent, methanol, mixture of acetic acid and water, or the like can be used instead of ethanol, and as the acid, acetic acid, hydrochloric acid, methanesulfonic acid, p-toluenesulfonic acid, or the like can also be used instead of polyphosphoric acid.

[Formula 10]

Method E

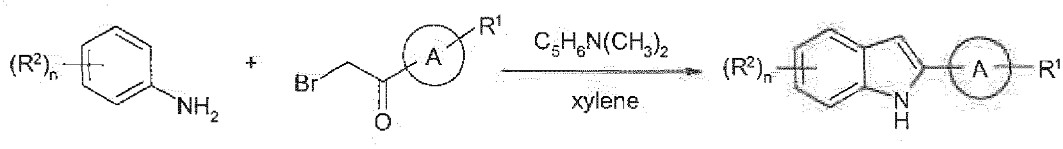


14 15 13

By stirring an aniline compound (14) and a phenacyl bromide compound (15) with heating in a mixture of xylene

and N,N-dimethylaniline according to the method of Kaufmann et al. (Bioorg. Med. Chem., 15(15), 5122, 2007), an objective indole compound (13) can be obtained. In this reaction, as the reaction solvent, N,N-dimethylformamide, N,N-dimethylacetamide, or the like can also be used instead of the mixture of xylene and N,N-dimethylaniline.

Conversion of a functional group can be performed in a conventional manner. For example, chlorination, bromination, or iodination of benzene ring or heterocyclic ring can be performed by adding an equivalent to excess amount of N-chlorosuccinimide, N-bromosuccinimide or N-iodosuccinimide to the compound in a solvent such as N,N-dimethylformamide, and stirring the resulting mixture at room temperature or with heating as required. In this reaction, dichloromethane, acetic acid, or the like can also be used as the reaction solvent instead of N,N-dimethylformamide, and bromine can also be used instead of N-bromosuccinimide.

Trifluoromethyl group can be introduced by adding methyl 2,2-difluoro-2-(fluorosulfonyl)acetate (MDFA) and copper iodide to the compound having a bromo- or iodo-substituted aromatic ring in N,N-dimethylformamide, and stirring the resulting mixture with heating according to the method of Eusterwiemann et al. (J. Org. Chem., 77, 5461, 2012). In this reaction, a combinatory reagent of three kinds of substances, (1,10-phenanthroline)(trifluoromethyl)copper(I) or trimethyl(trifluoromethyl)silane, silver fluoride, and copper, or the like can be used as the reaction reagents instead of the combination of methyl 2,2-difluoro-2-(fluorosulfonyl)acetate (MDFA) and copper iodide.

Cyano group can be introduced by adding excess amount of copper cyanide to the compound having a bromo- or iodo-substituted aromatic ring in N-methylpyrrolidone, and stirring the resulting mixture with heating. Such an objective cyano compound can also be obtained by adding 1/2 equivalent of potassium ferrocyanide, a catalytic amount of palladium acetate, and an equivalent amount of sodium carbonate to the compound having a bromo-substituted aromatic ring in N,N-dimethylacetamide, and stirring the resulting mixture with heating according to the method of Weissmann et al. (US2006/0106223). In this reaction, palladium, or the like can also be used as the catalyst instead of palladium acetate, and cesium carbonate, or the like can also be used as the base instead of sodium carbonate. As a reaction similar to the aforementioned reaction, the reaction can also be performed under the conditions that by adding sodium cyanide and cupric oxide to the compound in N,N-dimethylformamide and stirring the resulting mixture with heating.

The medicament of the present invention has an action of suppressing expression of PCSK9 as specifically demonstrated in the examples mentioned later, and it has an action of reducing blood LDL cholesterol based on the foregoing action. Therefore, the medicament of the present invention containing a compound represented by the general formula (I) or a salt thereof as an active ingredient can be used as a medicament for prophylactic and/or therapeutic treatment of high LDL cholesterolemia. It is also useful as a medicament for prophylactic and/or therapeutic treatment of lipidosis, arteriosclerosis, myocardial infarction, cerebral infarction, hypertension, or the like accompanied by a high LDL cholesterol condition or resulting from a high LDL cholesterol condition.

Although a compound represented by the aforementioned general formula (I) or a physiologically acceptable salt thereof per se as the active ingredient may be administered as the medicament of the present invention, a pharmaceutical composition for oral or parenteral administration can be preferably prepared by a method well known to those skilled in the art, and administered. Examples of dosage form of pharmaceutical composition suitable for oral administration include, for example, tablet, powder, capsule, subtilized granule, solution, granule, syrup, and the like, and examples of dosage form of pharmaceutical composition suitable for parenteral administration include, for example, injection such as preparations for intravenous injection and intramuscular injection, fusion drip, inhalant, eye drop, nose drop, suppository, transdermal preparation, transmucosal preparation, and the like, but they are not limited to these examples.

The aforementioned pharmaceutical composition can be prepared by a method well known to those skilled in the art using pharmaceutical additives widely used in preparation of pharmaceutical compositions in this industry. The pharmaceutical additives are not particularly limited, and can be appropriately chosen depending on form of the pharmaceutical composition, and purpose such as impartation of sustained releasability. Examples include, for example, excipient, binder, filler, disintegrating agent, surfactant, lubricant, dispersing agent, buffering agent, preservative, corrigent, perfume, coating agent, diluent, and the like, but they are not limited to these examples.

Dose of the medicament of the present invention is not particularly limited, and can be appropriately chosen according to type of disease to be prevented or treated, purpose such as prevention or treatment, type of the active ingredient, weight, age, and condition of patient, administration route, and the like. For example, in the case of oral administration, it can be used in an amount in the range of about 0.01 to 500 mg in terms of weight of the active ingredient as a daily dose for adult. However, the dose can be appropriately selected by those skilled in the art, and is not limited to a dose in the aforementioned range.

## Examples

Hereafter, the present invention will be still more specifically explained with reference to examples. However, the scope of the present invention is not limited to the following examples.

Example 1: 6-Chloro-7-trifluoromethyl-2-(4-trifluoromethylphenyl)benzoxazole

[Formula 11]

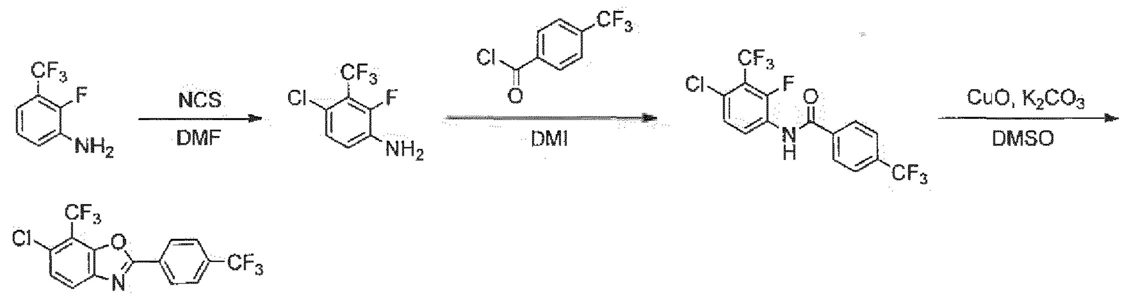


a) 4-Chloro-2-fluoro-3-(trifluoromethyl)aniline

To a solution of 2-fluoro-3-(trifluoromethyl)aniline (5.01 g, 27.97 mmol) in N,N-dimethylformamide (30 mL), N-chlorosuccinimide (4.11 g, 30.76 mmol) was added, and the resulting mixture was stirred at room temperature for one day. To the reaction solution, a 10% solution of sodium thiosulfate was added, the resulting mixture was extracted with diethyl ether, the organic layer was dried over anhydrous magnesium sulfate, and then the solvent was evaporated. The residue was purified by silica gel column chromatography to obtain the title compound (3.32 g, yield 56%).
$^1$H-NMR ($CDCl_3$) $\delta$: 3.90 (2H, br), 6.84 (1H, t, J=8.6Hz), 7.07 (1H, d, J=8.6Hz)

b) N-(4-Chloro-2-fluoro-3-trifluoromethylplienyl)-4-(trifluoromethyl)benzamide

To a solution of the compound obtained in a) (3.27 g, 15.33 mmol) in 1,3-dimethylimidazolidinone (16 mL), p-(trifluoromethyl)benzoyl chloride (2.74 mL, 18.39 mmol) was added, and the resulting mixture was stirred at 130°C for 3 hours. After the reaction mixture was left to cool, water was added to the reaction mixture, and the resulting mixture was extracted with ethyl acetate. The organic layer was dried over anhydrous magnesium sulfate, and then the solvent was evaporated. The residue was purified by silica gel column chromatography to obtain crystals of the title compound (5.27 g, yield 89%).
$^1$H-NMR ($CDCl_3$) $\delta$: 7,38 (1H, d, J=8.6Hz), 7.81 (2H, d, J=8.2Hz), 8.00 (2H, d, J=7.9Hz), 8.09 (1H, br), 8.65 (1H, t, J=8.2Hz)

c) 6-Chloro-7-trifluoromethyl-2-(4-trifluoromethylphenyl)henzoxazole

To a solution of the compound obtained in b) (5.25 g, 13.61 mmol) in dimethyl sulfoxide (15 mL), potassium carbonate (2.82 g, 20.42 mmol), and cupric oxide (54 mg, 0,68 mmol) were added, and the resulting mixture was stirred at 110°C for 16 hours. After the reaction mixture was left to cool, dichloromethane was added to the reaction mixture, and the resulting mixture was filtered through Celite. The solvent was evaporated, and the resulting crystals were recrystallized from dichloromethane and methanol to obtain crystals of the title compound (2.74 g, yield 55%).
$^1$H-NMR ($CDCl_3$) $\delta$: 7.54 (1H, dd, J=0.7Hz, 8.6Hz), 7.82 (2H, d, J=8.2Hz), 7.88 (1H, dd, J=0,7Hz, 8.6Hz), 8.38 (2H, d, J=7.9Hz)
Melting point: 130.0°C

Example 2: 7-Fluoro-6-iodo-2-(4-trifluoromethylphenyl)benzoxazole

[Formula 12]

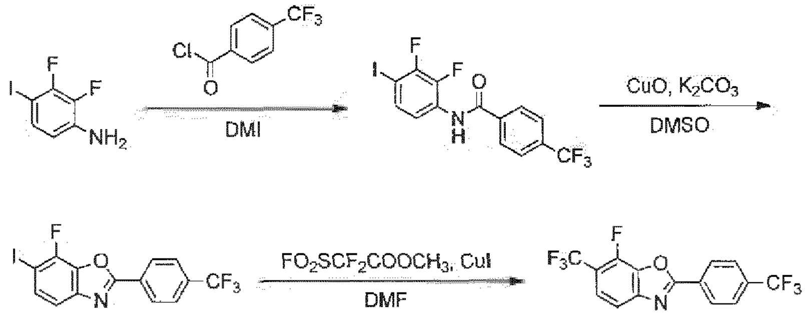


a) N-(2,3-Difluoro-4-iodophenyl)-4-(trifluoromethyl)benzamide

By using 2,3-difluoro-4-iodoaniline (2.55 g, 10.00 mmol) as a starting material, as well as a solution of 1,3-dimethylimidazolidinone (10 mL), and p-(trifluoromethyl)benzoyl chloride (1.79 mL, 12.00 mmol), crystals of the title compound (4.27 g, yield 100%) were obtained in the same manner as that of Example 1, b). $^1$H-NMR ($CDCl_3$) δ: 7.52-7.59 (1H, m), 7.80 (2H, d, J=8.2Hz), 7.99-8.02 (3H, m), 8.05-8.12(1H,m)

b) 7-Fluoro-6-iodo-2-(4-trifluoromethylphenyl)benzoxazole

By using the compound obtained in a) (1.16 g, 2.71 mmol) as a starting material, as well as dimethyl sulfoxide (4 mL), potassium carbonate (562 mg, 4.07 mmol), and cupric oxide (11 mg, 0.14 mmol), crystals of the title compound (175 mg, yield 16%) were obtained in the same manner as that of Example 1, c).
$^1$H-NMR ($CDCl_3$) δ: 7.39 (1H, d, J=8.6Hz), 7.72 (1H, dd, J=5.3Hz, 8.2Hz), 7.81 (2H, d, J=8.2Hz), 8.40 (2H, d, J=8.2Hz)

Example 3: 7-Fluoro-6-trifluoromethyl-2-(4-trifluoromethylphenyl)benzoxazole

To a solution of the compound obtained in Example 2 (122 mg, 0.30 mmol) in N,N-dimethylformamide (2 mL), copper iodide (17 mg, 0.09 mmol), and methyl fluorosulfonyldifluoroacetate (0.11 mL, 0.90 mmol) were added, and the resulting mixture was stirred at 100°C for 23 hours. After the reaction mixture was left to cool, dichloromethane was added to the reaction mixture, the resulting mixture was filtered through Celite, and then the solvent was evaporated. The residue was purified by silica gel column chromatography, and the resulting crystals were washed with methanol to obtain crystals of the title compound (75 mg, yield 72%).
$^1$H-NMR ($CDCl_3$) δ: 7.60-7.69 (2H, m), 7.84 (2H, d, J=8.2Hz), 8.43 (2H, d, J=8.2Hz) Melting point: 107.3°C

Example 4: 4-Chloro-5-trifluoromethyl-2-(4-trifluoromethylphenyl)benzoxazole

[Formula 13]

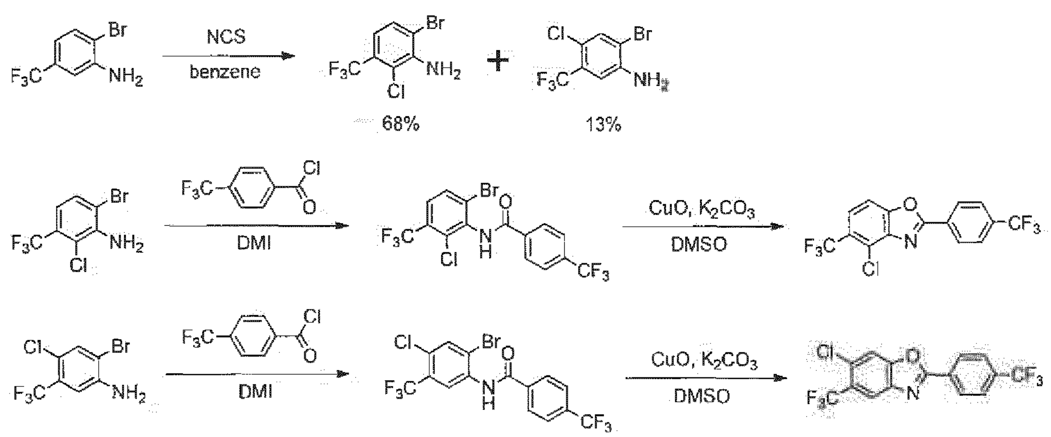

a) A: 6-Bromo-2-chloro-3-(trifluoromethyl)aniline, B: 2-Bromo-4-chloro-5-(trifluoromethyl)aniline

To a solution of 2-bromo-5-(trifluoromethyl)aniline (4.80 g, 20.00 mmol) in N,N-dimethylformamide (35 mL), N-chlorosuccinimide (2.67 g, 20.00 mmol) was added, and the resulting mixture was stirred at 60°C for 2 days. Water was added to the reaction mixture, the resulting mixture was extracted with ethyl acetate, the organic layer was dried over anhydrous magnesium sulfate, and then the solvent was evaporated. The residue was purified by silica gel column chromatography to obtain the title compound A (3.74 g, yield 68%), and the title compound B (0.717 g, yield 13%), A: 6-Bromo-2-chloro-3-(trifluoromethyl)aniline
$^1$H-NMR ($CDCl_3$) δ: 4.78 (2H, br), 6.94 (1H, d, J=7.9Hz), 7.44 (1H, d, J=7.9Hz) B: 2-Bromo-4-chloro-5-(trifluoromethyl)aniline
$^1$H-NMR ($CDCl_3$) δ: 4.27 (2H, br), 7.04 (1H, s), 7.55 (1H, s)

b) N-[6-Bromo-2-chloro-3-(trifluoromethyl)phenyl]-4-(trifluoromethyl)benzamide

By using the compound A obtained in a) (3.64 g, 13.25 mmol) as a starting material, as well as 1,3-dimethylimidazolidinone (15 mL), and p-(trifluoromethyl)benzoyl chloride (2.37 mL, 15.90 mmol), crystals of the title compound (2.01 g, yield 34%) were obtained in the same manner as that of Example 1, b). $^1$H-NMR ($CDCl_3$) δ: 7.59 (1H, d, J=8.6Hz), 7.62-7.65 (1H, m), 7.75 (1H, dd, J=0.7Hz, 8.6Hz), 7.81 (2H, d, J=8.2Hz), 8.09 (2H, d, J=7.9Hz)

c) 4-Chloro-5-trifluoromethyl-2-(4-trifluoromethylphenyl)benzoxazole

By using the compound obtained in b) (1.98 g, 4.44 mmol) as a starting material, as well as a dimethyl sulfoxide solution (5 mL), potassium carbonate (920 mg, 6.66 mmol), and cupric oxide (18 mg, 0.22 mmol), crystals of the title compound (477 mg, yield 29%) were obtained in the same manner as that of Example 1, c).
$^1$H-NMR ($CDCl_3$) δ: 7.61 (1H, d, J=8.6Hz), 7.77 (1H, d, J=8.9Hz), 7.83 (2H, d, J=8.2Hz), 8.45 (2H, d, J=8.2Hz)
Melting point: 118.4°C

Example 5: 6-Chloro-5-trifluoromethyl-2-(4-trifluoromethylphenyl)benzoxazole

a) N-[2-Bromo-4-chloro-5-(trifluoromethyl)phenyl]-4-(trifluoromethyl)benzamide

By using the compound B obtained in Example 4, a) (675 mg, 2.46 mmol) as a starting material, as well as 1,3-dimethylimidazolidinone (4 mL, and p-(trifluoromethyl)benzoyl chloride (0.44 mL, 2.95 mmol), crystals of the title compound (1.04 g, yield 94%) were obtained in the same manner as that of Example 1, b). $^1$H-NMR ($CDCl_3$) δ: 7.78 (1H, s), 7.82 (2H, d, J=8.2Hz), 8.04 (2H, d, J=8.2Hz), 8.46 (1H, br), 9.00 (1H,s)

b) 6-Chloro-5-trifluoromethyl-2-(4-trifluoromethylphenyl)benzoxazole

By using the compound obtained in a) (1.01 g, 2.27 mmol) as a starting material, as well as dimethyl sulfoxide (6 mL), potassium carbonate (470 mg, 3.40 mmol), and cupric oxide (9.0 mg, 0.11 mmol), crystals of the title compound (81 mg, yield 10%) were obtained in the same manner as that of Example 1, c).
$^1$H-NMR ($CDCl_3$) δ: 7.80 (1H, s), 7.83 (2H, d, J=8.2Hz), 8.16 (1H, s), 8.38 (2H, d, J=7.9Hz)

Example 6: 7-Chloro-6-iodo-2-(4-trifluoromethylphenyl)benzoxazole

[Formula 14]

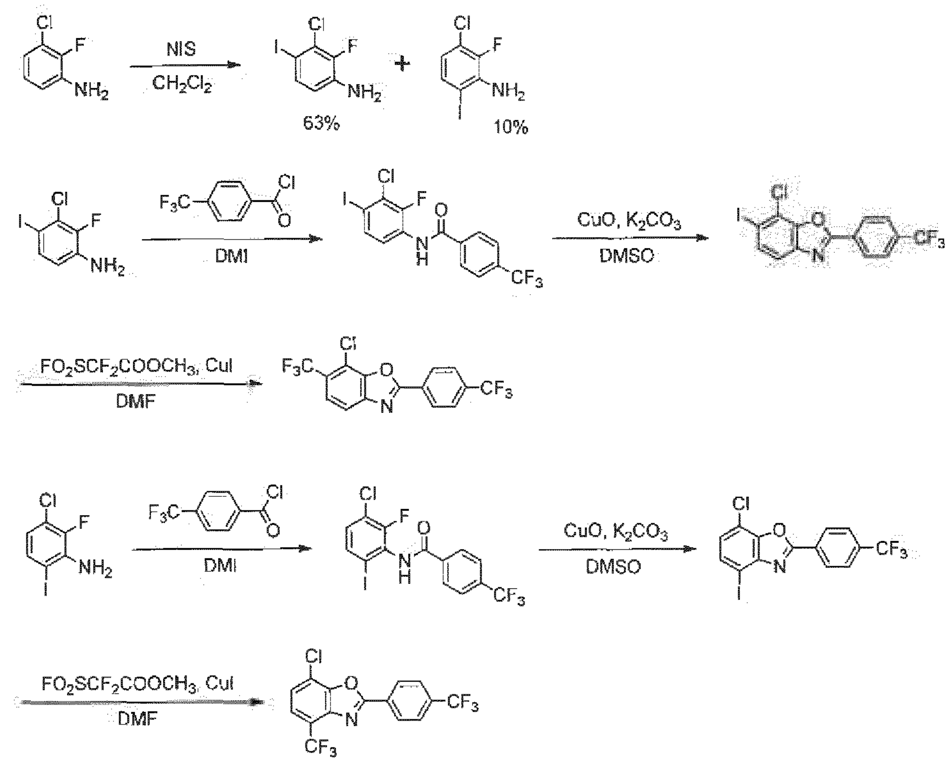


a) A: 3-Chloro-2-fluoro-4-iodoaniline, B: 3-Chloro-2-fluoro-6-iodoaniline

To a solution of 3-chloro-2-fluoroaniline (13.10 g, 90.0 mmol) in dichloromethane (300 mL), N-iodosuccinimide (21.26 g, 94.5 mmol) was added, and the resulting mixture was stirred at room temperature for 4 days. After the reaction, ethyl acetate (40 mL) and hexane (200 mL) were added to the reaction mixture, and the insoluble matter was separated by filtration. The resulting filtrate was purified by silica gel column chromatography to obtain the title compound A (15.43 g, yield 63%), and the title compound B (2.76 g, yield 10%).
A: 3-Chloro-2-fluoro-4-iodoaniline
$^1$H-NMR ($CDCl_3$) δ: 3.84 (2H, br), 6.48 (1H, t, J=8.2Hz), 7.36 (1H, dd, J=2.0Hz, 8.2Hz) B: 3-Chloro-2-fluaro-6-iodoaniline
$^1$H-NMR ($CDCl_3$) δ: 4.24 (2H, br), 6.54 (1H, dd, J=6.9Hz, 8.6Hz), 7.33 (1H, dd, J=2.0Hz, 8.6Hz)

b) N-(3-Chloro-2-fluoro-4-iodophenyl)-4-(trifluoromethyl)benzamide

By using the compound A obtained in a) (8.14 g, 30.0 mmol) as a starting material, as well as 1,3-dimethylimidazolidinone (30 mL), and p-(trifluoromethyl)benzoyl chloride (5.36 mL, 36.0 mmol), crystals of the title compound (9.73 g, yield 73%) were obtained in the same manner as that of Example 1, b). $^1$H-NMR ($CDCl_3$) δ:7.71 (1H, dd, J=2.0Hz, 8.9Hz), 7.80 (2H, d, J=8.6Hz), 7.98-8.01 (3H, m), 8.20 (1H, dd, J=7.6Hz, 8.9Hz)

c) 7-Chloro-6-iodo-2-(4-trifluoromethylphenyl)benzoxazole

By using the compound obtained in b) (4.17 g, 9.41 mmol) as a starting material, as well as dimethyl sulfoxide (10 mL), potassium carbonate (1.95 g, 14.11 mmol), and cupric oxide (37 mg, 0.47 mmol), crystals of the title compound (1.87 g, yield 63%) were obtained in the same manner as that of Example 1, c).
$^1$H-NMR ($CDCl_3$ δ: 7.46 (1H, d, J=8.2Hz), 7.81 (2H, d, J=8.2Hz), 7.87 (1H, d, J=8.2Hz), 8.40 (2H, d, J=7.9Hz)

Example 7: 7-Chloro-6-trifluoromethyl-2-(4-trifluoromethylphenyl)benzoxazole

By using the compound obtained in Example 6 (2.12 g, 5.00 mmol) as a starting material, as well as N,N-dimethylformamide (25 mL), copper iodide (286 mg, 1.50 mmol), and methyl fluorosulfonyldifluoroacetate (1.91 mL, 15.00 mmol), crystals of the title compound (1.75 g, yield 95%) were obtained in the same manner as that of Example 3.
$^1$H-NMR ($CDCl_3$) δ: 7.76 (2H, d, J=1.3Hz), 7.84 (2H, d, J=8.2Hz), 8.44 (2H, d, J=8.2Hz)

Example 8: 7-Chloro-4-trifluoromethyl-2-(4-trifluoromethylphenyl)benzoxazole

a) N-(3-Chloro-2-fluoro-6-iodophenyl)-4-(trifluoromethyl)benzamide

By using the compound B obtained in Example 6, a) (1.04 g, 3.84 mmol) as a starting material, as well as 1,3-dimethylimidazolidinone (5 mL), and p-(trifluoromethyl)benzoyl chloride (0.69 mL, 4.61 mmol), crystals of the title compound (833 mg, yield 49%) were obtained in the same manner as that of Example 1, b).
$^1$H-NMR ($CDCl_3$) δ: 7.17 (1H, dd, J=6.9Hz, 8.6Hz), 7.45 (1H, br), 7.63 (1H, dd, J=2.0Hz, 8.6Hz), 7.81 (2H, d, J=8.2Hz), 8.09 (2H, d, J=7.9Hz)

b) 7-Chloro-4-iodo-2-(4-trifluoromethylphenyl)benzoxazole

By using the compound obtained in a) (798 mg, 1.80 mmol) as a starting material, as well as dimethyl sulfoxide (3 mL), potassium carbonate (373 mg, 2.70 mmol), and cupric oxide (14 mg, 0.18 mmol), crystals of the title compound (154 mg, yield 20%) were obtained in the same manner as that of Example 1, c).
$^1$H-NMR ($CDCl_3$) δ: 7.18 (1H, d, J=8.2Hz), 7.71 (1H, d, J=8.2Hz), 7.81 (2H, d, J=8.2Hz), 8.46 (2H, d, J=8.2Hz)

c) 7-Chloro-4-trifluoromethyl-2-(4-trifluoromethylphenyl)benzoxazole

By using the compound obtained in b) (69 mg, 0.16 mmol) as a starting material, as well as N,N-dimethylformamide (2 mL), copper iodide (9 mg,0.05 mmol), and methyl fluorosulfonyldifluoroacetate (0.06 mL, 0.49 mmol), crystals of the title compound (45 mg, yield 75%) were obtained in the same manner as that of Example 3. $^1$H-NMR ($CDCl_3$) δ: 7.48 (1H, dd, J=0.7Hz, 8.2Hz), 7.62 (1H, dd, J=0.7Hz, 8.6Hz), 7.82 (2H, d, J=8.2Hz), 8.48 (2H, d, J=7.9Hz)

Example 9: 7-Chloro-6-cyano-2-(4-trifluoromethylphenyl)benzoxazole

[Formula 15]

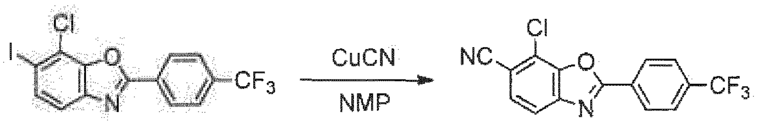


A mixed solution of the compound obtained in Example 6 (605 mg, 1.43 mmol), and copper cyanide (256 mg, 2.86 mmol) in N-methylpyrrolidone (3 mL) was stirred with heating at 120°C for 1,5 hours, and at 140°C for 2 hours. After the reaction mixture was left to cool, aqueous ammonia was added to the reaction mixture, and the insoluble matter was removed by filtration through Celite. The reaction mixture was extracted with chloroform, and the organic layer was washed with saturated aqueous sodium chloride, and dried over anhydrous magnesium sulfate. The organic layer was concentrated, and then the residue was purified by silica gel column chromatography, and dried to obtain crystals of the title compound (200 mg, yield 43%).
$^1$H-NMR ($CDCl_3$) δ: 7.71 (1H, d, J=8.2Hz), 7.91 (1H, d, J=8.2Hz), 7.85 (2H, d, 1=8.2Hz), 8.42-8.46 (2H, m)
Melting point: 244 to 248°C

Example 10: 4-Chloro-6-iodo-2-(4-trifluoromethylphenyl)benzoxazole

[Formula 16]

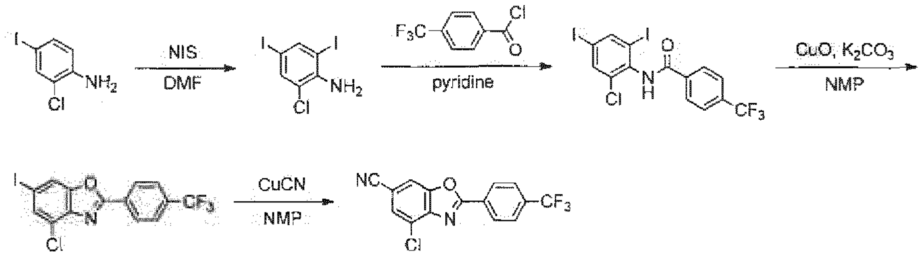


a) 2-Chloro-4,6-diiodoaniline

To a solution of 2-chloro-4-iodoaniline (5.06 g, 19.95 mmol) in N,N-dimethylformamide (100 mL), N-iodosuccinimide (4.52 g, 20.10 mmol) was added under ice cooling, and the resulting mixture was stirred for 1.5 hours under ice cooling, and then at room temperature for 4 days. The reaction mixture was further stirred with heating at 50°C, N-iodosuccinimide (119 mg, 0.53 mmol) was added to the reaction mixture, and the resulting mixture was stirred overnight at 50°C. After the reaction mixture was left to cool, N-iodosuccinimide (2.26 g, 10.03 mmol) was further added to the reaction mixture, and the resulting mixture was stirred at room temperature for 8 days. To the reaction solution, water was added, and the precipitates were separated by filtration, washed with water, and then dried. The resulting crystals were dissolved in ethyl acetate, and the organic layer was dried over anhydrous magnesium sulfate. The organic layer was concentrated, and then the residue was purified by silica gel column chromatography, and dried to obtain crystals of the title compound (4.86 g, yield 64%).
$^1$H-NMR ($CDCl_3$) $\delta$: 4.57 (2H, br), 7.51 (1H, d, J=1.6Hz), 7.81 (1H, d, J=2.0Hz)

b) 2-Chloro-4,6-diiodo-1-(4-trifluoromethyl)benzoylaminobenzene

To a solution of the compound obtained in a) (3.82 g, 10.06 mmol) in pyridine (10 mL), 4-(trifluoromethyl)benzoyl chloride (2.09 g, 10.01 mmol) was added, and the resulting mixture was refluxed with heating for 2 hours. The reaction mixture was left to cool to room temperature, and water was added to the reaction mixture. The precipitates were separated by filtration, washed with water, and dried. The precipitates were purified by silica gel column chromatography and dried to obtain crystals of the title compound (3.82 g, yield 69%).
$^1$H-NMR ($CDCl_3$) $\delta$: 7.59 (1H, br), 7.79 (2H, d, J=8.2Hz), 7.82 (1H, d, J=2.0Hz), 8.07 (2H, d, J-8.2Hz), 8.16 (1H, d, J=1.6Hz)

c) 4-Chloro-6-iodo-2-(4-trifluaxomethylphenyl)benzoxazale

By using the compound obtained in b) (549 mg, 1.00 mmol) as a starting material, as well as cupric oxide (13 mg, 0.17 mmol), potassium carbonate (215 mg, 1.56 mmol), and dimethyl sulfoxide (3 mL), crystals of the title compound (276 mg, yield 65%) were obtained in the same manner as that of Example 1, c).
$^1$H-NMR ($CDCl_3$) $\delta$: 7.73 (1H, d, J=1.3Hz), 7.79 (2H, d, J=8.2Hz), 7.89 (1H, d, J=1.3Hz), 8.39 (2H, d, J=7.9Hz)

Example 11: 4-Chloro-6-evano-2-(4-trifluoromethylphenyl)benzoxazole

By using the compound obtained in Example 10 (623 mg, 1.47 mmol) as a starting material, as well as copper cyanide (266 mg, 2.96 mmol), and N-methylpyrrolidone (3 mL), crystals of the title compound (242 mg, yield 51%) were obtained in the same manner as that of Example 9.
$^1$H-NMR ($CDCl_3$) $\delta$: 7.71 (1H, d, J=1.3Hz), 7.84 (2H, d, J=8.2Hz), 7.87 (1H, d, J= 1.3Hz), 8.45 (2H, d, J=7.9Hz)
Melting point: 186 to 187°C

Example 12: 5,7-Dichloro-6-fluoro-2-(4-trifluoromethylphenyl)benzoxazole

[Formula 17]

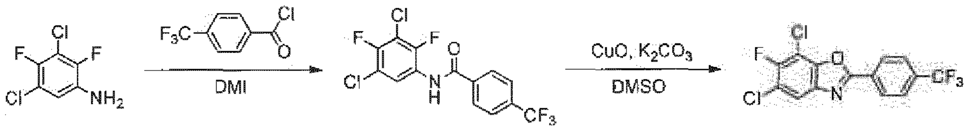


a) N-(3,5-Dichloro-2,4-difluorophenyl)-4-(trifluoromethyl)benzamide

By using 3,5-dichloro-2,4-difluoroaniline (990 mg, 5.00 mmol) as a starting material, as well as 1,3-dimethylimidazolidinone (5 mL), and p-(trifluoromethyl)benzoyl chloride (0.89 mL, 6.00 mmol), crystals of the title compound (1.33 g, yield 72%) were obtained in the same manner as that of Example 1, b). $^1$H-NMR ($CDCl_3$) $\delta$: 7.81 (2H, d, J=8.2Hz), 7.91 (1H, br), 7.99 (2H, d, J=7.9Hz), 8.57 (1H, t, J=7.6Hz)

b) 5,7-Dichloro-6-fluoro-2-(4-trifluoromethylphenyl)benzoxazole

By using the compound obtained in a) (1.31 g, 3.54 mmol) as a starting material, as well as dimethyl sulfoxide (4 mL), potassium carbonate (735 mg, 5.32 mmol), and cupric oxide (14 mg, 0.18 mmol), crystals of the title compound (345 mg, yield 28%) were obtained in the same manner as that of Example 1, c).
$^1$H-NMR ($CDCl_3$) $\delta$: 7.76 (1H, d, J=5.9Hz), 7.82 (2H, d, J=8.2Hz), 8.38 (2H, d, J=8.2Hz)

Example 13: 6-Bromo-7-trifluoromethyl-2-(4-trifluoromethylphenyl)benzoxazole

[Formula 18]

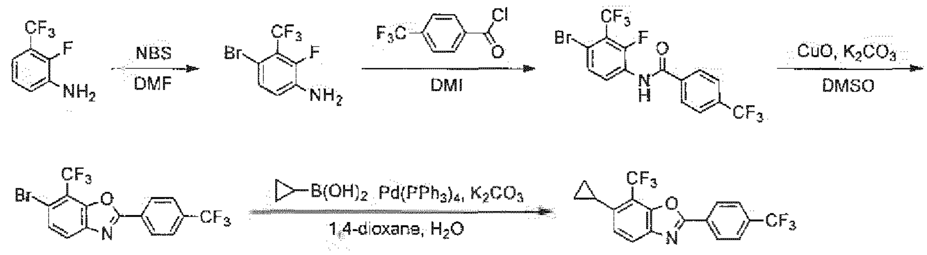


a) 4-Bromo-2-fluoro-3-(trifluoromethyl)aniline

To a solution of 2-fluoro-3-(trifluoromethyl)aniline (1.79 g, 10.0 mmol) in N,N-dimethylformamide (7 mL), N-bromosuccinimide (1.78 g, 10.0 mmol) was added, and the resulting mixture was stirred at room temperature for 2 hours. A 10% solution of sodium thiosulfate was added to the reaction mixture, the resulting mixture was extracted with diethyl ether, the organic layer was dried over anhydrous magnesium sulfate, and then the solvent was evaporated. The residue was purified by silica gel column chromatography to obtain the title compound as oil (2.36 g, yield 91%). $^1$H-NMR ($CDCl_3$) $\delta$: 3.92 (2H, br), 6.77 (1H, t, J=8.9Hz), 7.24-7.28 (1H, m)

b) N-[4-Bromo-2-fluoro-3-(trifluoromethyl)phenyl]-4-(trifluoromethyl)benzamide

By using the compound obtained in a) (7.48 g, 28.98 mmol) as a starting material, as well as 1,3-dimethylimidazolidinone (30 mL), and p-(trifluoromethyl)benzoyl chloride (5.18 mL, 34.77 mmol), crystals of the title compound (12.15 g, yield 97%) were obtained in the same manner as that of Example 1, b). $^1$H-NMR ($CDCl_3$) $\delta$: 7.59 (1H, dd, J=1.3Hz, 8.9Hz), 7.81 (2H, d, J=8.2Hz), 8.00 (2H, d, J=8.2Hz), 8.10 (1H, br), 8.58 (1H, t, J=8.6Hz)

c) 6-Bromo-7-trifluoromethyl-2-(4-trifluoromethylphenyl)benzaxazole

By using the compound obtained in b) (4.30 g, 10.0 mmol) as a starting material, as well as dimethyl sulfoxide (12 mL), potassium carbonate (2.07 g, 15.0 mmol), and cupric oxide (40 mg, 0.50 mmol), crystals of the title compound (2.24 g, yield 55%) were obtained in the same manner as that of Example 1, c).
$^1$H-NMR ($CDCl_3$) $\delta$: 7.73 (1H, d, J=8.9Hz), 7.81 (1H, d, J=8.6Hz), 7.83 (2H, d, J=8.2Hz), 8.39 (2H, d, J=8.2Hz)

Example 14: 6-Cyclopropyl-7-trifluoromethyl-2-(4-trifluoromethylphenyl)benzoxazole

To a suspension of the compound obtained in Example 13 (205 mg, 0.50 mmol) in 1,4-dioxane (3 mL) and water (1 mL), potassium carbonate (76 mg, 0.55 mmol), tetrakis(triphenylphosphine)palladium(0) (29 mg, 0.025 mmol), and cyclopropylboronic acid (47 mg, 0.55 mmol) were added, and the resulting mixture was refluxed overnight with heating. Cyclopropylboronic acid (94 mg, 1.10 mmol) was further added to the reaction mixture, and the resulting mixture was refluxed overnight with heating. After the reaction mixture was left to cool, dichloromethane was added to the reaction mixture, the resulting mixture was filtered through Celite, and the organic layer was dried over anhydrous magnesium sulfate. After the solvent was evaporated, the residue was purified by silica gel column chromatography to obtain crystals of the title compound (110 mg, yield 59%).
$^1$H-NMR ($CDCl_3$) δ: 0.83-0.89 (2H, m), 1.09-1.17 (2H, m), 2.30-2.36 (1H, m), 7.11 (1H, d, J=8.6Hz), 7.79-7.84 (3H, m), 8.38 (2H, d, J=7.9Hz)
Melting point: 110.1°C

Example 15: 6-Amino-7-trifluoromethyl-2-(4-trifluoromethylphenyl)benzoxazole

[Formula 19]

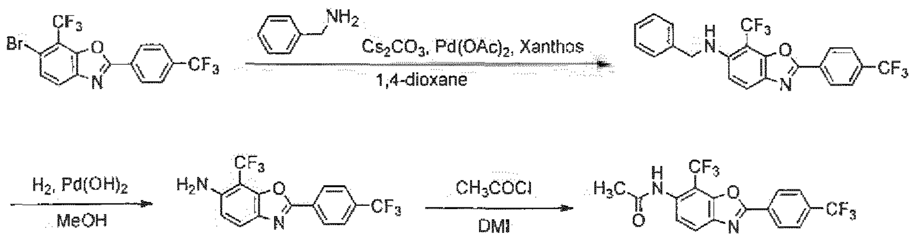


a) 6-Benzylamino-7-trifluoromethyl-2-(4-trifluoromethylphenyl)benzoxazole

To a solution of the compound obtained in Example 13 (6.15 g, 15.0 mmol) in 1,4-dioxane (60 mL), palladium acetate (168 mg, 0.75 mmol), 4,5-bis(diphenylphosphino)-9,9-dimethylxanthene (868 mg, 1.50 mmol), cesium carbonate (5.86 g, 18.0 mmol), and benzylamine (8.20 mL, 75.0 mmol) were added, and the resulting mixture was refluxed overnight with heating. After the reaction mixture was left to cool, dichloromethane was added to the reaction mixture, the resulting mixture was filtered through Celite, and then the organic layer was dried over anhydrous magnesium sulfate. After the solvent was evaporated, the residue was purified by silica gel column chromatography to obtain crystals of the title compound (6.08 g, yield 93%).
$^1$H-NMR ($CDCl_3$) δ: 4.52 (2H, d, J=4.3Hz), 5.12 (1H, br), 6.75 (1H, d, J=8.9Hz), 7.29 7.42 (5H, m), 7.69 (1H, d, J=8.9Hz), 7.77 (2H, d, J=8.2Hz), 8.30 (2H, d, J=8.2Hz)

b) 6-Amino-7-trifluoromethyl-2-(4-trifluoromethylphenyl)benzoxazole

To a solution of the compound obtained in a) (6.08 g, 13.9 mmol) in methanol (80 mL), palladium hydroxide (265 mg) was added, and the resulting mixture was stirred overnight at room temperature under a hydrogen atmosphere. The reaction mixture was filtered through Celite, the solvent was evaporated, and then the residue was purified by silica gel column chromatography to obtain crystals of the title compound (4.75 g, yield 98%).
$^1$H-NMR ($CDCl_3$) δ: 4.44 (2H, br), 6.75 (1H, d, J=8.6Hz), 7.68 (1H, d, J=8.6Hz), 7.77 (2H, d, J=8.2Hz), 8.30 (2H, d, J=8.2Hz)

Example 16: 6-Acetamido-7-trifluoromethyl-2-(4-trifluoromethylphenyl)benzoxazole

To a solution of the compound obtained in Example 15 (208 mg, 0.60 mmol) in 1,3-dimethylimidazolidinone (2 mL), acetyl chloride (0.06 mL, 0.90 mmol) was added, and the resulting mixture was stirred at 50°C for 4 hours. After the reaction mixture was left to cool, water was added to the reaction mixture, and the resulting mixture was extracted with dichloromethane. The organic layer was dried over anhydrous magnesium sulfate, then the solvent was evaporated, and the resulting crystals were washed with diethyl ether to obtain crystals of the title compound (202 mg, yield 87%).
$^1$H-NMR ($CDCl_3$) δ: 2.28 (3H, s), 7.50 (1H, br), 7.81 (2H, d, J=8.2Hz), 7.95 (1H, d, J=8.6Hz), 8.12-8.16 (1H, m), 8.37 (2H, d, J=8.2Hz)

Example 17: 6-(3-Methoxycarbonylpropanamido)-7-trifluoromethyl-2-(4-trifluoromethylphenyl)benzoxazole

[Formula 20]

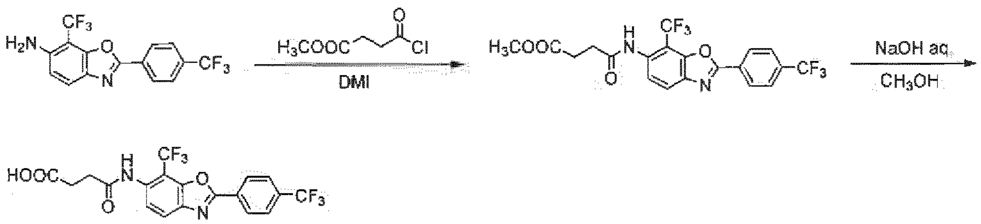


By using the compound obtained in Example 15 (1.38 g, 4.00 mmol), 1,3-dimethylimidazolidinone (6 mL), and methyl 4-chloro-4-oxobutyrate (0.74 mL, 6.00 mmol), crystals of the title compound (1.30 g, yield 70%) were obtained in the same manner as that of Example 16.
$^1$H-NMR ($CDCl_3$) δ: 2.74-2.83 (4H, m), 3.75 (3H, s), 7.80-7.83 (3H, m), 7.93 (1H, d, J=8.9Hz), 8.08-8.12 (1H, m), 8.37 (2H, d, J=8.2Hz)

Example 18: 6-(3-Carbonylpropanamido)-7-trifluoromethyl-2-(4-trifluoromethylphenyl)benzoxazole

To a suspension of the compound obtained in Example 17 (1.20 g, 2.60 mmol) in methanol (12 mL), 2 N sodium hydroxide solution (1.95 mL, 3.90 mmol) was added, and the resulting mixture was stirred at 50°C for 1 hour. After the reaction, the reaction mixture was neutralized with 1 N hydrochloric acid, and the deposited crystals were separated by filtration, and washed with diisopropyl ether to obtain crystals of the title compound (1.04 g, yield 90%).
$^1$H-NMR (DMSO-$d_6$) δ: 2.49-2.62 (4H, m), 7.47 (1H, d, J=8.2Hz), 8.03 (2H, d, J=8.2Hz), 8.14 (1H, d, J=8.6Hz), 8.39 (2H, d, J=8.6Hz), 10.01 (1H, s), 12.19 (1H, s)

Example 19: 6-Cyano-7-trifluoromethyl-2-(4-trifluoromethylphenyl)benzoxazole

[Formula 21]

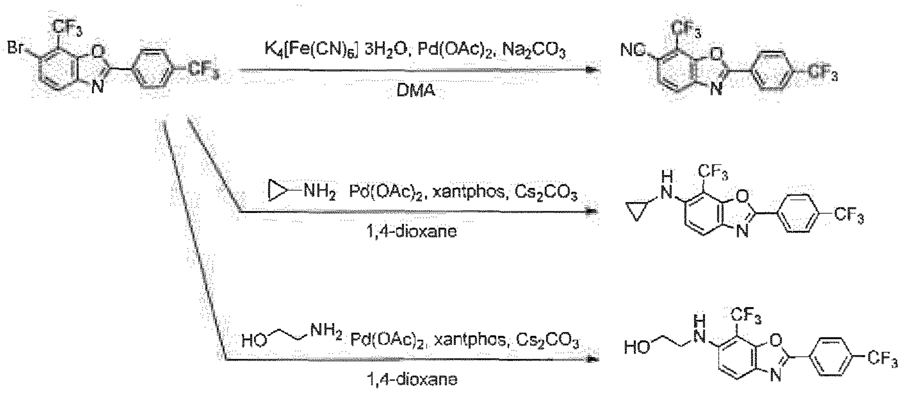


To a solution of the compound obtained in Example 13 (82 mg, 20 mmol) in N,N-dimethylacetamide (2 mL), sodium carbonate (21 mg, 0.20 mmol), palladium acetate (5 mg, 0.02 mmol), and potassium ferrocyanide (42 mg, 0.10 mmol) were added, and the resulting mixture was stirred at 80°C for 16 hours. After the reaction mixture was left to cool, dichloromethane was added to the reaction mixture, the resulting mixture was filtered through Celite, and then the organic layer was dried over anhydrous magnesium sulfate. The solvent was evaporated, and then the resulting residue was purified by silica gel column chromatography to obtain crystals of the title compound (48 mg, yield 67%).

$^1H$-NMR ($CDCl_3$) δ: 7.86 (3H, d, J=8.2Hz), 8.07 (1H, dd, J=0.7Hz, 8.2Hz), 8.44 (2H, d, J=7.9Hz)

Example 20: 6-(Cyclopropylamino)-7-trifluoromethyl-2-(4-trifluoromethylphenyl)benzoxazole

By using the compound obtained in Example 13 (205 mg, 0.50 mmol), 1,4-dioxane (5 mL), palladium acetate (28 mg, 0.13 mmol), 4,5-bis(diphenylphosphino)-9,9-dimethylxanthene (87 mg, 0.15 mmol), cesium carbonate (195 mg, 0.60 mmol), and cyclopropylamine (0.35 mL, 5.00 mmol), crystals of the title compound (68 mg, yield 35%) were obtained in the same manner as that of Example 15, a).
$^1H$-NMR ($CDCl_3$) δ: 0,60-0.66 (2H, m), 0.85-0.92 (2H, m), 2.54-2.57 (1H, m), 5.06-5.08 (1H, m), 7.26-7.30 (1H, m), 7.75-7.79 (3H, m), 8.30 (2H, d, J=7.9Hz)

Example 21: 6-(2-Hydroxyethylamino)-7-trifluoromethyl-2-(4-trifluoromethylphenyl)benzoxazole

By using the compound obtained in Example 13 (205 mg, 0.50 mmol), 1,4-dioxane (5 mL), palladium acetate (11 mg, 0.05 mmol), 4,5-bis(diphenylphosphino)-9,9-dimethylxanthene (58 mg, 0.10 mmol), cesium carbonate (195 mg, 0.60 mmol), and 2-aminoethanol (0.15 mL, 2.50 mmol), crystals of the title compound (145 mg, yield 74%) were obtained in the same manner as that of Example 15, a).
$^1H$-NMR (DMSO-$d_6$) δ: 3.30-3.36 (2H,m), 3.62 (2H, q, J=5.6Hz), 4.94 (1H, t, J=5.3Hz), 5.74 (1H, br), 6.99 (1H, d, J=9.2Hz), 7.88 (1H, d, J=8.9Hz), 7.97 (2H, d, J=8.2Hz), 8.28 (2H, d, J=8.2Hz)
Melting point: 165.3°C

Example 22: 7-Chloro-6-cyano-4-methoxy-2-(4-trifluoromethylphenyl)benzoxazole

[Formula 22]

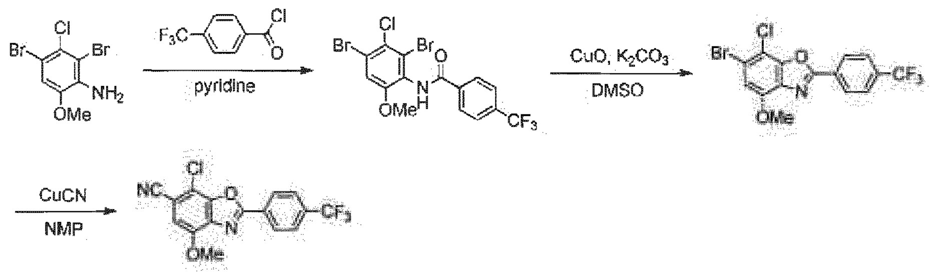


a) N-(2,4-Dibromo-3-chloro-6-methoxyphenyl)-4-(trifluoromethyl)benzamide

By using 3-chloro-2,4-dibromo-6-methoxyaniline (3.15 g, 10.0 mmol) as a starting material, as well as pyridine (10 mL), and 4-(trifluoromethyl)benzoyl chloride (20.8 g, 10.0 mmol), crystals of the title compound (4.70 g, yield 96%) were obtained in the same manner as that of Example 10, b).
$^1H$-NMR ($CDCl_3$) δ: 3.85 (3H, s), 7.25 (1H, s), 7.45 (1H, br), 7.75-7.78 (2H, m), 8.04 (2H, d, J=7.9Hz)

b) 7-Chloro-6-bromo-4-methoxy-2-(4-trifluoromethylphenyl)benzoxazole

By using the compound obtained in a) (490 mg, 1.00 mmol) as a starting material, as well as cupric oxide (4.5 mg, 0.06 mmol), potassium carbonate (206 mg, 1.49 mmol), and dimethyl sulfoxide (3 mL), crystals of the title compound (246 mg, yield 60%) were obtained in the same manner as that of Example 1, c).
$^1H$-NMR ($CDCl_3$) δ: 4.08 (3H, s), 7.11 (1H, s), 7.79 (2H, d, J=8.2Hz), 8.42 (2H, d, J=8.2Hz)

c) 7-Chloro-6-cyano-4-methoxy-2-(4-trifluoromethylphenyl)benzoxazole

By using the compound obtained in b) (1.073 g, 2.64 mmol) as a starting material, as well as copper cyanide (355 mg, 3.96 mmol), and N-methylpyrrolidone (10 mL), crystals of the title compound (133 mg, yield 14%) were obtained in the same manner as that of Example 9.
$^1H$-NMR ($CDCl_3$) δ: 4.13 (3H, s), 7.12 (1H, s), 7.83 (2H, d, J=8.2Hz), 8.46 (2H, d, J=8.2Hz)

Example 23: 2-Ethyl-7-(4-trifluoromethylphenyl)benzo[2,1-d:3,4-d']bisoxazole

[Formula 23]

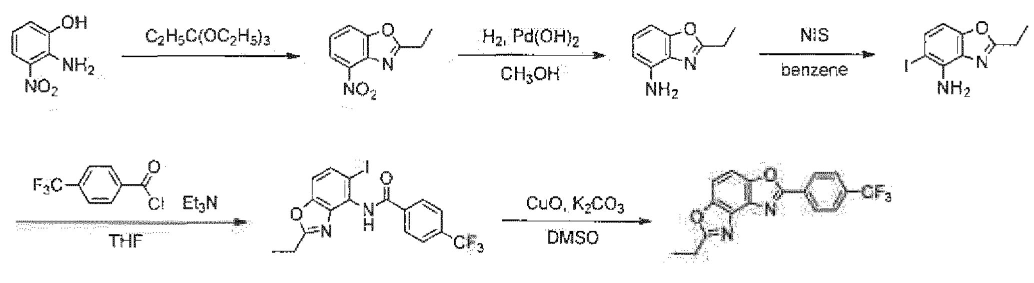


a) 4-Nitro-2-ethylbenzoxazole

Under a nitrogen atmosphere, ethyl orthopropionate (10 mL) was added to 2-amino-3-nitrophenol (4.6 g, 30 mmol), and the resulting mixture was heated at 100°C for 2 hours. The reaction mixture was left to cool to room temperature, and the resulting solid was recrystallized from hexane to obtain crystals of the title compound (5.2 g, yield 90%).
$^1$H-NMR ($CDCl_3$) δ: 1.50 (3H, t, J=7.6Hz), 3.11 (2H, q, J=7.6Hz), 7.46 (1H, t, J=8.4Hz), 7.83 (1H, dd, J=1.1Hz, 8.4Hz), 8.17 (1H, dd, J=0.5Hz, 8.1Hz)

b) 4-Amino-2-ethylbenzoxazole

To the compound obtained in a) (5.2 g, 27 mmol), methanol (35 mL) and a catalytic amount of palladium hydroxide (80 mg) were added, and the resulting mixture was stirred at room temperature for 5 days under a hydrogen atmosphere. The reaction mixture was filtered through Celite, then the solvent was evaporated under reduced pressure, and the residue was dried to obtain the title compound as an oily substance (4.3 g, yield 98%).
$^1$H-NMR ($CDCl_3$) δ: 1.43 (3H, t, J=7.6Hz), 2.93 (2H, q, J=7.6Hz), 4.24 (2H, br), 6.56 (1H, d, J=7.6Hz), 6.86 (1H, dd, J=0.5Hz, 8.1Hz), 7.07 (1H, t, J=8.1Hz)

c) 4-Amino-2-ethyl-5-iodobenzoxazole

Under a nitrogen atmosphere, N-iodosuccinimide (3.5 g, 15.4 mmol), and acetic acid (927 μL, 16.2 mmol) were added to a solution of the compound obtained in b) (2.5 g, 15.4 mmol) in benzene (90 mL), and the resulting mixture was stirred at room temperature for 5 days. The reaction mixture was filtered, and then the solvent was evaporated under reduced pressure. The resulting residue was dissolved in chloroform, the organic layer was washed with saturated aqueous sodium hydrogencarbonate, water, and saturated aqueous sodium chloride, and dried over anhydrous magnesium sulfate, and then the solvent was evaporated under reduced pressure. The resulting residue was purified by silica gel column chromatography to obtain crystals of the title compound (1.0 g, yield 23%).
$^1$H-NMR (DMSO-$d_6$) δ: 1.33 (3H, t, J=7.6Hz), 2.92 (2H, q, J=7.6Hz), 5.48 (2H, br), 6.73 (1H, d, J=8.6Hz), 7.47 (1H, d, J=8.4Hz)

d) N-(2-Ethyl-5-iodobenzoxazol-4-yl)-4-trifluoromethylbenzamide

Under a nitrogen atmosphere, triethylamine (265 μL, 1.9 mmol), and p-trifluoromethylbenzoyl chloride (267 μL, 1.8 mmol) were added to a solution of the compound obtained in c) (460 mg, 1.6 mmol) in tetrahydrofuran (12 mL), and the resulting mixture was stirred overnight at room temperature. The solvent was evaporated, and the resulting residue was recrystallized from hexane to obtain crystals of the title compound (317 mg, yield 43%).
$^1$H-NMR (DMSO-$d_6$) δ: 1.31 (3H, t, J=7.8Hz), 2.97 (2H, q, J=7.8Hz), 7.54 (1H, d, J=8.6Hz), 7.88 (1H, d, J=7.8Hz), 7.97 (2H, d, J=7.8Hz), 8.25 (2H, d, J=8.1Hz), 10.72 (1H, s)

e) 2-Ethyl-7-(4-trifluoromethylphenyl)benzo[2,1-d:3,4-d']bisoxazole

By using the compound obtained in d) (317 mg, 0.7 mmol) as a starting material, as well as cupric oxide (6.4 mg, 0.1 mmol), potassium carbonate (332 mg, 2.4 mmol), and dimethyl sulfoxide (2 mL), crystals of the title compound (153 mg, yield 29%) were obtained in the same manner as that of Example 1, c).
$^1$H-NMR (DMSO-$d_6$) δ: 1.42 (3H, t, J=7.6Hz), 3.07 (2H, q, J=7.6Hz), 7.84 (2H, s), 8.02 (2H, d, J=8.6Hz), 8.45 (2H, d, J=8.1Hz)
Melting point: 235.0°C

Example 24: 6-(Trifluoromethyl)-2-[4-(trifluoromethyl)phenyl]benzoxazole

[Formula 24]

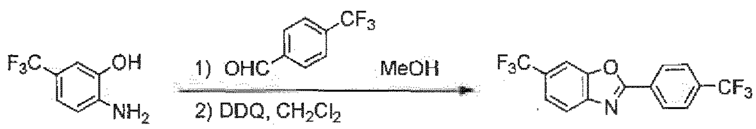


Under a nitrogen atmosphere, p-trifluoromethylbenzaldehyde (268 μL, 2.0 mmol) was added to a solution of 4-amino-3-hydroxybenzotrifluoride (354 mg, 2.0 mmol) in methanol (10 mL), and then the resulting mixture was warmed to 50°C, and stirred overnight. Methanol was evaporated under reduced pressure, and then the residue was dissolved in dichloromethane (10 mL) under a nitrogen atmosphere, 2,3-dichloro-5,6-dicyano-1,4-benzoquinone (499 mg, 2.2 mmol) was added to the solution, and the resulting mixture was stirred at room temperature for 3 hours. Then, saturated aqueous sodium carbonate was added to the reaction mixture, the organic layer was washed with saturated aqueous sodium carbonate, and saturated aqueous sodium chloride, and then dried over magnesium sulfate, and the solvent was evaporated under reduced pressure. The resulting residue was purified by silica gel column chromatography, and dried under reduced pressure to obtain crystals of the title compound (430mg, yield 65%).
$^1$H-NMR ($CDCl_3$) δ: 7.68 (1H, dd, J=1.6Hz, 8.4Hz), 7.83 (1H, d, J=8.1Hz), 7.91 (2H, d, J=7.8Hz), 8.41 (2H, d, J=8.1Hz)

Example 25:7-(Trifluoromethyl)-2-[4-(trifluoromethyl)phenyl]benzoxazole

[Formula 25]

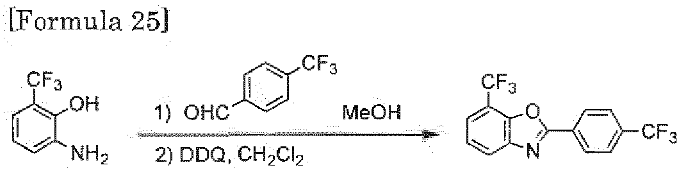


By using 3-amino-2-hydroxybenzotrifluoride (124 mg, 0.7 mmol), p-trifluoromethylbenzaldehyde (94 μL, 0.7 mmol), methanol (5 mL), 2,3-dichloro-5,6-dicyano-1,4-benzoquinone (175 mg, 0.8 mmol), and dichloromethane (6 mL), crystals of the title compound (142 mg, yield 61%) were obtained in the same manner as that of Example 24.
$^1$H-NMR, ($CDCl_3$) δ: 7.4-7.5 (1H, m), 7.65 (1H, d, J=7.8Hz), 7.82 (2H, d, J=8.1Hz), 7.99 (1H, d, J=7.8Hz), 8.42 (1H, d, J=7.8Hz)

Example 26: 5-Chloro-4-(trifluoromethyl)-2-[4-(trifluoromethyl)phenyl]benzoxazole

[Formula 26]

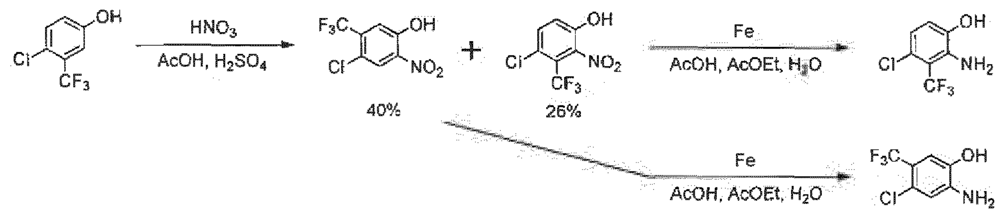


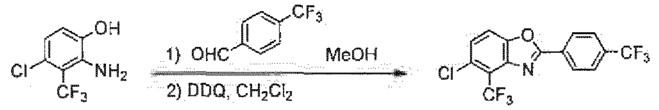


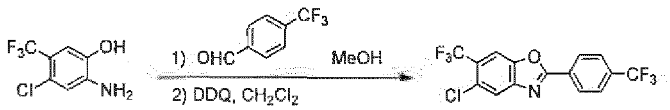


a) A: 2-Chloro-5-hydroxy-4-nitrobenzotrifluoride, B: 2-Chloro-5-hydroxy-4-nitrob e nzotrifluoride

Under ice cooling, sulfuric acid (3 mL) was added to a solution of 2-chloro-5-hydroxybenzotrifluoride (5 g, 25.4 mmol) in acetic acid (20 mL). 69% Nitric acid (2 mL) was slowly added dropwise to the reaction mixture, and the resulting mixture was warmed to room temperature, and stirred for 3 hours. The reaction mixture was poured into ice water, the resulting mixture was neutralized with sodium hydrogencarbonate, and then ethyl acetate was added to the reaction mixture. The organic layer was washed 3 times with water, then washed with saturated aqueous sodium chloride, dried over anhydrous magnesium sulfate, and filtered, and then the solvent was evaporated under reduced pressure. The resulting residue was purified by flash column chromatography, and dried to obtain crystals of the title compound A (1.6 g, yield 26%) and crystals of the title compound B (2.5g, yield 40%).
A: 2-Chloro-5-hydroxy-4-nitrobenzotrifluoride
$^1$H-NMR ($CDCl_3$) δ: 7.57 (1H, s), 8.27 (1H, s), 10.43 (1H, s)
B: 2-Chloro-5-hydroxy-4-nitrobenzotrifluoride
$^1$H-NMR ($CDCl_3$) δ: 7.23 (1H, d, J=8.9Hz), 7.52 (1H, d, J=9.2Hz)

b) 2-Amino-6-chloro-3-hydroxybenzotrifluoride

To a solution of the compound A obtained in a) (1.6 g, 6.6 mmol) in ethyl acetate (6 mL), water (8 mL), and acetic acid (8 mL) were added, then iron powder (2.6 g, 47.0 mmol) was added to the mixture, and the resulting mixture was stirred at 80°C for 1 hour. The reaction mixture was left to cool to room temperature, and then extracted 3 times with ethyl acetate and water, and the resulting organic layer was washed with saturated aqueous sodium hydrogencarbonate, and then with saturated aqueous sodium chloride, and dried over anhydrous magnesium sulfate. Then, the solvent was evaporated under reduced pressure, and the residue was dried to obtain crystals of the title compound (1.4 g, yield 97%).
$^1$H-NMR ($CDCl_3$) δ: 5.20 (2H, br), 6.67 (1H, d, J=8.6Hz), 6.73 (1H, d, J=8.4Hz)

c) 5-Chlaro-4-(trifluoromethyl)-2-[4-trifluoromethyl)phenyl]benzoxazole

By using the compound obtained in b) (317 mg, 1.5 mmol) as a starting material, as well as p-trifluoromethylbenzaldehyde (201 μL, 1.5 mmol), methanol (7 mL), dichloromethane (7 mL), and 2,3-dichloro-5,6-dicyano-1,4-benzoquinone (375 mg, 1.7 mmol), crystals of the title compound (204 mg, yield 37%) were obtained in the same manner as that of Example 24.
$^1$H-NMR ($CDCl_3$) δ: 7.53 (1H, d, J=8.9Hz), 7.72 (1H, d, J=8.6Hz), 7.81 (2H, d, J=7.8Hz), 8.42 (1H, d, J=8.4Hz)

Example 27: 5-Chloro-6-(trifluoromethyl)-2-[4-(trifluoromethyl)phenyl]benzoxazole

a) 4-Amino-2-chloro-5-hydraxybenzotrifluoride

By using the compound B obtained in Example 26, a) (2.5 g, 10.2 mmol) as a starting material, as well as ethyl acetate (8 mL), water (12 mL), acetic acid (12 mL), and iron powder (2.9 g, 51.0 mmol), crystals of the title compound (2.1 g, yield 97%) were obtained in the same manner as that of Example 26, b).
$^1$H-NMR ($CDCl_3$) δ: 6.77 (1H, s), 7.00 (1H, s)

b) 5-Chloro-6-(trifluoromethyl)-2-[4-(trifluoromethyl)phenyl]benzoxazole

By using the compound obtained in a) (423 mg, 2.0 mmol) as a starting material, as well as p-trifluoromethylbenzaldehyde (268 μL, 2.0 mmol), methanol (10 mL), dichloromethane (10 mL), and 2,3-dichloro-5,6-dicyano-1,4-benzoquinone (499 mg, 2.2 mmol), crystals of the title compound (97 mg, yield 13%) were obtained in the same manner as that of Example 24.
$^1$H-NMR ($CDCl_3$) δ: 7.84 (2H, d, J=8.4Hz), 7.94 (1H, s), 7.99 (1H, s), 8.39 (2H, d, J=8.4Hz)

Example 28: 7-Chloro-5-(trifluoromethyl)-2-[4-(trifluoromethyl)phenyl]benzoxazole

[Formula 27]

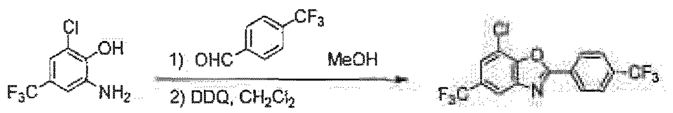


By using 3-amino-5-chloro-4-hydroxybenzotrifluoride (317 mg, 1.5 mmol), p-trifluoromethylbenzaldehyde (201 μL, 1.5 mmol), methanol (8 mL), 2,3-dichloro-5,6-dicyano-1,4-benzoquinone (375 mg, 1.7 mmol), and dichloromethane (8 mL), crystals of the title compound (289 mg, yield 53%) were obtained in the same manner as that of Example 24.
$^1$H-NMR ($CDCl_3$) δ: 7.69 (1H, d, J=1.1Hz), 7.84 (2H, d, J=8.4Hz), 7.98 (1H, d, J=0.8Hz), 8.43 (2H, d, J=7.8Hz)
Melting point: 75.1°C

Example 29: 6-Cyano-2-[4-(trifluoromethyl)phenyl]benzoxazole

[Formula 28]

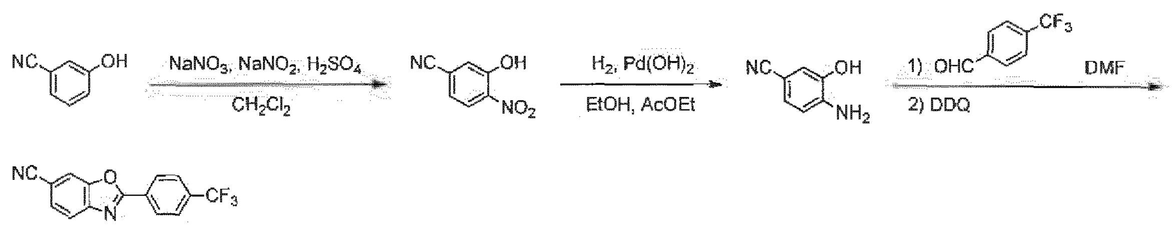


a) 3-Hydroxy-4-nitrobenzonitrile

To a solution of 3-hydroxybenzonitrile (5 g, 42.0 mmol) in dichloromethane (84 mL), a solution of sodium nitrate (3.9 g, 46.2 mmol) in 3 M sulfuric acid (42 mL), and then sodium nitrite (29 mg, 0.4 mmol) were added, and the resulting mixture was stirred overnight at room temperature. The reaction mixture was washed twice with water, and then with saturated aqueous sodium chloride, the organic layer was dried over anhydrous magnesium sulfate, and then the solvent was evaporated under reduced pressure. The residue was purified by silica gel column chromatography, and dried to obtain crystals of the title compound (1.6 g, yield 23%).
$^1$H-NMR (DMSO-$d_6$) δ: 7.44 (1H, dd, J=1.6Hz, 8.6Hz), 7.51 (1H, d, =1.6Hz), 8.02 (1H, d, J=8.6Hz), 11.86 (1H,s)

b) 4-Amino-3-hydroxybenzonitrile

3-Hydroxy-4-nitrobenzonitrile (1.6 g, 9.6 mmol) was dissolved in a mixture of ethanol (40 mL) and N,N-dimethylformamide (20 mL), palladium hydroxide (30 mg) was added to the solution, and the resulting mixture was stirred overnight at room temperature under a hydrogen atmosphere. The reaction mixture was filtered, then the solvent was evaporated under reduced pressure, and the residue was dried to obtain crystals of the title compound (771 mg, yield 60%).

$^1$H-NMR (DMSO-$d_6$) δ: 5.57 (2H, br), 6.62 (1H, d, J=8.4Hz), 6.86 (1H, d, J=2.2Hz), 6.99 (1H, dd, J=1.9Hz, 8.1Hz)

c) 6-Cyano-2-[4-(trifluoromethyl)phenyl]benzoxazole

Under a nitrogen atmosphere, p-trifluoromethylbenzaldehyde (268 μL, 2.0 mmol) was added to a solution of the compound obtained in b) (268 mg, 2.0 mmol) in N,N-dimethylformamide (8 mL), and then the resulting mixture was warmed to 110°C and stirred overnight. The reaction mixture was left to cool to room temperature, then 2,3-dichloro-5,6-dicyano-1,4-benzoquinone (499 mg, 2.2 mmol) was added to the reaction mixture, and the resulting mixture was stirred at 110°C for 3 hours. Then, saturated aqueous sodium carbonate was added to the reaction mixture, the organic layer was washed with saturated aqueous sodium carbonate and saturated aqueous sodium chloride, and then dried over magnesium sulfate, and the solvent was evaporated under reduced pressure. The resulting residue was purified by silica gel column chromatography, and dried under reduced pressure to obtain crystals of the title compound (100 mg, yield 17%).
$^1$H-NMR ($CDCl_3$) δ: 7.70 (1H, dd, J=1.6Hz, 8.1Hz), 7.8-7.9 (3H, m), 7.95 (1H, dd, J=0.5Hz, 1..4Hz), 8.41 (2H, d, J=8.4Hz)
Melting point: 218.9°C

Example 30: 4-Nitro-2-(4-trifluoromethylphenyl)benzoxazole

[Formula 29]

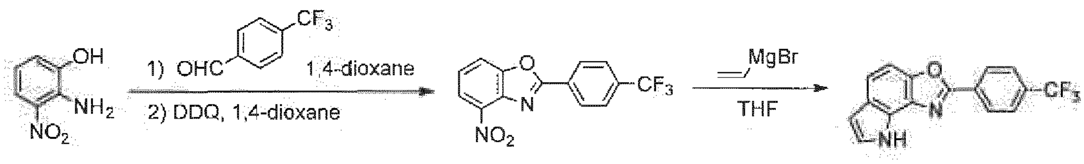


A mixture of 2-amino-3-nitrophenol (9.25 g, 60.03 mmol), 4-(trifluoromethyl)benzaldehyde (10.49 g, 60.22 mmol), and Molecular Sieve 4A (15 g) in 1,4-dioxane (90 mL) was refluxed over 3 nights with heating. The reaction mixture was left to cool to room temperature, 2,3-dichloro-5,6-dicyano-1,4-benzoquinone (20.45 g, 90.07 mmol), and 1,4-dioxane (45 mL) were added to the reaction mixture, and the resulting mixture was stirred at 50°C for 30 minutes, and stirred with heating at 120°C for 2 hours. After the reaction mixture was left to cool, the organic layer was filtered through Celite, and concentrated, the concentration residue and the residue remained on Celite were thoroughly washed with chloroform. The resulting organic layer was washed 3 times with saturated aqueous sodium carbonate, and then washed with saturated aqueous sodium chloride. The organic layer was dried over anhydrous magnesium sulfate, the solvent was evaporated, and then the residue was purified by silica gel column chromatography, and dried to obtain crystals of the title compound (9.53 g, yield 51%).
$^1$H-NMR CCDCl$_3$) δ: 7.56 (1H, t, J=8.2Hz), 7.84 (2H, d, J=8.2Hz), 7.97 (1H, dd, J=1.0Hz, 8.2Hz), 8.25 (1H, dd, J=0.7Hz, 8.2Hz), 8.51 (2H, d, 8.2Hz)

Example 31: 2-(4-Trifluoromethylphenyl)-8H-pyrrolo[2,3-e]benzoxazole

To a solution of the compound obtained in Example 30 (382 mg, 1.24 mmol) in tetrahydrofuran (3 mL), vinylmagnesium bromide (14% solution in tetrahydrofuran, about 1 mol/L, 5 mL, 5 mmol) was added at -40°C, and the resulting mixture was stirred for 1 hour. Saturated aqueous ammonium chloride was added to the reaction mixture, the resulting mixture was extracted with chloroform, and the organic layer was dried over anhydrous magnesium sulfate, and then concentrated. The residue was purified by silica gel column chromatography, and dried to obtain crystals of the title compound (63 mg, yield 17%).
$^1$H-NMR ($CDCl_3$) δ: 6.71 (1H, dd, J=2.3Hz, 3.3Hz), 7.28 (1H, dd, J=2.3Hz, 3.3Hz), 7.41 (1H, d, J=8.9Hz), 7.66 (1H, d, J=8.6Hz), 7.77-7.80 (2H, m), 8.36-8.39 (2H, m), 9.19 (1H, br)

Melting point: 240 to 241°C

Example 32: 4-Amino-2-(4-trifluoromethylphenyl)benzoxazole

[Formula 30]

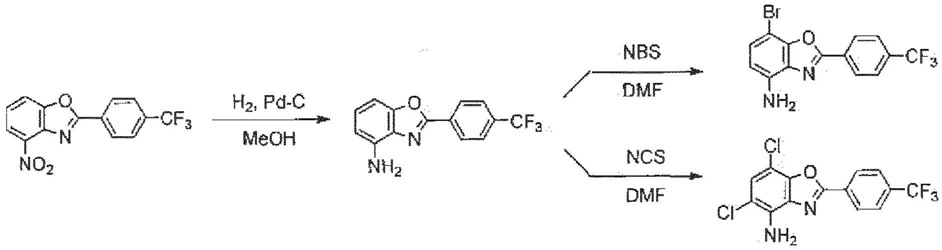


To a solution of the compound obtained in Example 30 (905 mg, 2.94 mmol) in methanol (50 mL), 10% palladium-activated carbon (111 mg) was added, and the resulting mixture was stirred overnight at room temperature under a hydrogen atmosphere. The reaction mixture was filtered through Celite, and then the residue was washed with chloroform. The filtrate and the washing solution were combined, and concentrated under reduced pressure, and the residue was purified by silica gel column chromatography, and dried to obtain the title compound (633 mg, yield 78%). $^1$H-NMR ($CDCl_3$) δ: 4.40 (2H, br), 6.63 (1H, dd, J=1.0Hz, 7.9Hz), 6.97 (1H, dd, J=1.0Hz, 8.2Hz), 7.17 (1H, t, J=7.9Hz), 7.75-7.78 (2H, m), 8.32-8.35 (2H, m)

Example 33: 4-Amino-7-bromo-2-(4-trifluoromethylphenyl)benzoxazole

Under ice cooling, N-bromosuccinimide (178 mg, 1.00 mmol) was added to a solution of the compound obtained in Example 32 (277 mg, 1.00 mmol) in N,N-dimethylformamide (4 mL), and the resulting mixture was stirred for 2.5 hours. Water was added to the reaction mixture, and then the precipitates were separated by filtration, and washed with water. The precipitates were dissolved in chloroform, and the organic layer was dried over anhydrous magnesium sulfate. The organic layer was concentrated, and the residue was purified by silica gel column chromatography, and dried to obtain crystals of the title compound (326 mg, yield 91%).
$^1$H-NMR ($CDCl_3$) δ: 4.40 (2H, br), 6.54 (1H, d, J=8.2Hz), 7.27 (1H, d, J=8.6Hz), 7.76-7.80 (2H, m), 8.34-8.38 (2H, m)
Melting point: 160 to 162°C

Example 34: 4-Amino-5,7-dichloro-2-(4-trifluoromethylphenyl)benzoxazole

Under ice cooling, N-chlorosuccinimide (137 mg, 1.03 mmol) was added to a solution of the compound obtained in Example 32 (279 mg, 1.00 mmol) in N,N-dimethylformamide (4 mL), and the resulting mixture was stirred under ice cooling for 3.5 hours and then at room temperature for 3.5 hours, and further stirred overnight with heating at 60°C. To the reaction solution, N-chlorosuccinimide (136 mg, 1.02 mmol) was further added, and the resulting mixture was stirred at 60°C for 2 hours. Water was added to the reaction solution, and the precipitates were separated by filtration, and washed with water. The precipitates were dissolved in chloroform, and the organic layer was dried over anhydrous magnesium sulfate. The organic layer was concentrated, and the residue was purified by silica gel column chromatography, and dried to obtain crystals of the title compound (318 mg, yield 91%).
$^1$H-NMR. ($CDCl_3$) δ: 4.73 (2H, br), 7.28 (1H, s), 7.77-7.80 (2H, m), 8.33-8.37 (2H, m)
Melting point: 159 to 160°C

Example 35: A: 4-Amino-5-chloro-2-(4-trifluoromethylphenyl)benzoxazole, B: 4-Amino-7-chloro-2-(4-trifluoromethylphenyl)benzoxazole

[Formula 31]

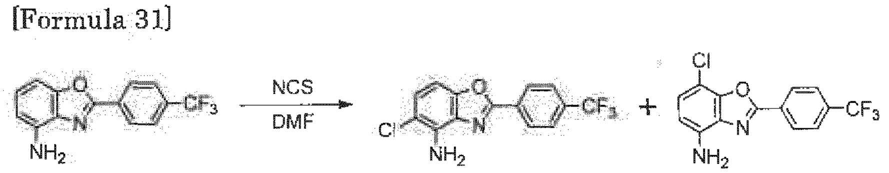


Under ice cooling, N-chlorosuccinimide (183 mg, 1.37 mmol) was added to a solution of the compound obtained in Example 32 (377 mg, 1.35 mmol) in N,N-dimethylformamide (5.4 mL), and then the resulting mixture was stirred at room temperature over 3 nights. To the reaction solution, saturated aqueous sodium hydrogencarbonate and water were

added, and the precipitates were separated by filtration, and washed with water. The precipitates were dissolved in chloroform, and the organic layer was dried over anhydrous magnesium sulfate. The organic layer was concentrated, and the residue was purified by silica gel column chromatography, and dried to obtain crystals of the title compound A (174 mg, yield 41%) and crystals of the title compound B (134 mg, yield 32%).
A: 4-Amino-5-chloro-2-(4-trifluoromethylphenyl)benzoxazole
$^1$H-NMR ($CDCl_3$) δ: 4.75 (2H, br), 6.91 (1H, d, J=8.6Hz), 7.26 (1H, d, J=8.6Hz), 7.77 (2H, d, J=8.6Hz), 8.30-8.34 (2H, m)
Melting point: 132 to 134°C
B: 4-Amino-7-chloro-2-(4-trifluoromethylphenyl)benzoxazole
$^1$H-NMR ($CDCl_3$) δ: 4.39 (2H, br), 6.57 (1H, d, J=8.6Hz), 7.14 (1H, d, J=8.6Hz), 7.78 (2H, d, J=7.9Hz), 8.35-8.39 (2H, m)
Melting point: 149 to 150°C

Example 36: 4-Amino-7-chloro-5-cyano-2-(4-trifluoromethylphenyl)benzoxazole

[Formula 32]

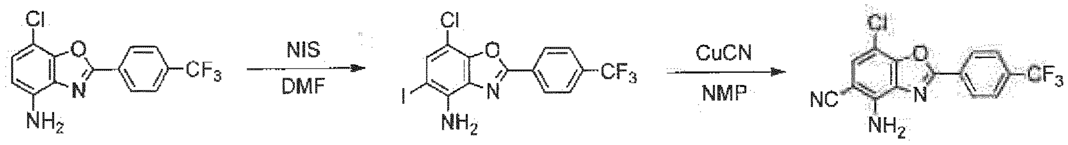


a) 4-Amino-7-chloro-5-iodo-2-(4-trifluoromethylphenyl)benzoxazole

To a solution of the compound B obtained in Example 35 (341 mg, 1.09 mmol) in N,N-dimethylformamide (5 mL), N-iodosuccinimide(373 mg, 1.66 mmol) was added, and then the resulting mixture was stirred at room temperature over 3 nights. Water was added to the reaction mixture, and the precipitates were separated by filtration, and washed with water. The precipitates were dissolved in chloroform, and the organic layer was dried over anhydrous magnesium sulfate. The organic layer was concentrated, and the residue was purified by silica gel column chromatography, and dried to obtain crystals of the title compound (446 mg, yield 93%).
$^1$H-NMR ($CDCl_3$) δ: 4.80 (2H, br), 7.59 (1H, s), 7.77-7.80 (2H, m), 8.33-8.37 (2H, m)

b) 4-Amino-7-chloro-5-cyano-2-(4-trifluoromethylphenyl)benzoxazole

By using the compound obtained in a) (428 mg, 0.97 mmol) as a starting material, as well as copper cyanide (131 mg, 1.46 mmol), and N-methylpyrrolidone (4 mL), crystals of the title compound (109 mg, yield 33%) were obtained in the same manner as that of Example 9.
$^1$H-NMR ($CDCl_3$) δ: 5.20 (2H, br), 7.39 (1H, s), 7.81 (2H, d, J=8.2Hz), 8.36 (2H, d, J=7.9Hz)
Melting point: 250 to 251°C

Example 37: A: 4-Amino-7-iodo-2-(4-trifluoromethylphenyl)benzoxazole, B: 4-Amino-5-iodo-2-(4-trifluoromethylphenyl)benzoxazole

[Formula 33]

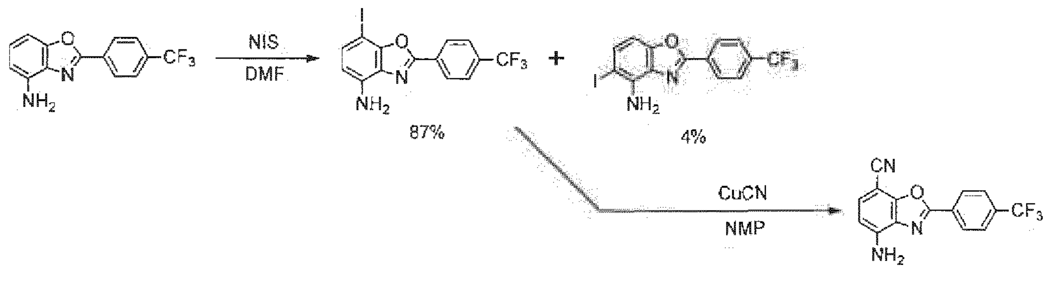


Under ice cooling, N-iodosuccinimide (2.69 g, 11.95 mmol) was added to a solution of the compound obtained in Example 32 (3.31 g, 11.90 mmol) in N,N-dimethylformamide (48 mL), and the resulting mixture was stirred for 1.5

hours. Water was added to the reaction mixture, and the precipitates were separated by filtration, and washed with water. The precipitates were dissolved in chloroform, and the organic layer was dried over anhydrous magnesium sulfate. The organic layer was concentrated, and the residue was purified by silica gel column chromatography, and dried to obtain crystals of the title compound A (4.19 g, yield 87%) and crystals of the title compound B (185 mg, yield 4%).
A: 4-Amino-7-iodo-2-(4-trifluoromethylphenyl)benzoxazole
$^1$H-NMR ($CDCl_3$) δ: 4.42 (2H, br), 6.48 (1H, d, J=8.2Hz), 7.43 (1H, d, J=8.2Hz), 7.78 (2H, d, J=8.2Hz), 8.35 (2H, d, J=8.2Hz)
B: 4-Amino-5-iodo-2-(4-trifluoromethylphenyl)benzoxazole
$^1$H-NMR ($CDCl_3$) δ: 4.82 (2H, br), 6.80 (1H, d, J=8.6Hz), 7.59 (1H, d, J=8.6Hz), 7.76-7.79 (2H, m), 8.30-8.33 (2H, m)

Example 38: 4-Amino-5,7-diiodo-2-(4-trifluoromethylphenyl)benzoxazole

By using the compound A obtained in Example 37 (203 mg, 0.50 mmol) as a starting material, as well as copper cyanide (90 mg, 1.01 mmol), and N-methylpyrrolidone (1 mL), crystals of the title compound (79 mg, yield 52%) were obtained in the same manner as that of Example 9.
$^1$H-NMR ($CDCl_3$) δ: 4.94 (2H, br), 6.60 (1H, d, J=8.2Hz), 7,45 (1H, d, J=8.6Hz), 7.78-7.82 (2H, m), 8.33-8.39 (2H, m)
Melting point: 171 to 174°C

Example 39: 7-Methylamino-2-(4-trifluoromethylphenyl)thiazolo[4,5-e]benzoxazole

[Formula 34]

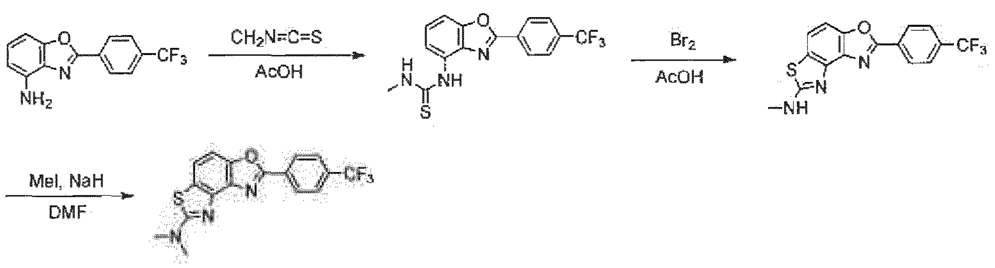


a) 1-Methyl-3-[2-(4-trifluoromethylphenyl)benzoxazol-4-yl]thiourea

To a mixture of the compound obtained in Example 32 (1.30 g, 4.67 mmol) with acetic acid (5 mL), methyl isothiocyanate (376 mg, 5.14 mmol) was added, and the resulting mixture was stirred at 100°C for 4 hours. After the reaction mixture was left to cool, acetic acid (2 mL) was added to the reaction mixture, and the precipitates were separated by filtration, washed with a small volume of acetic acid (smaller than 1 mL), and dried to obtain crystals of the title compound (1.04 g, yield 63%).
$^1$H-NMR ($CDCl_3$) δ: 3.30 (3H, d, J=4.6Hz), 7.14 (1H, br), 7.35-7.43 (2H, m), 7.81-7.85 (2H, m), 8.24 (1H, br), 8.31-8.34 (2H, m), 9.14 (1H, br)

b) 7-Methylamino-2-(4-trifluoromethylphenyl)thiazolo[4,5-e]benzoxazole

To a suspension of the compound obtained in a) (1.03 g, 2.94 mmol) in acetic acid (5 mL), bromine (151 μL, 3-10 mmol) was added, and the resulting mixture was stirred at 50 to 60°C for 1 hour with heating. The reaction mixture was left to cool, and then made alkaline with 2 N aqueous sodium hydroxide, and then the precipitates were separated by filtration, washed with water, and dried to obtain crystals of the title compound (1.04 g, yield 101%).
$^1$H-NMR (DMSO-$d_6$) δ: 3.04-3.05 (3H, m), 7.49 (1H, d, J=8.6Hz), 7.81 (1H, d, J=8.6Hz), 7.99 (2H, d, J=8.2Hz), 8.29 (1H, app-q, J=4.6Hz), 8.43 (2H, d, 7.9Hz)

Example 40: 7-Dimethylamino-2-(4-trifluoromethylphenyl)thiazolo[4,5-e]benzoxazole

At room temperature, sodium hydride (60% in oil, 27 mg, 0.68 mmol) was added to a suspension of the compound obtained in Example 39 (197 mg, 0.56 mmol) in N,N-dimethylformamide (2 mL), and the resulting mixture was stirred for 25 minutes. Then, methyl iodide (39 μL, 0.63 mmol) was added to the reaction mixture, and the resulting mixture was stirred at room temperature for 1 hour, and left overnight. Water was added to the reaction mixture, the precipitates

were separated by filtration, washed with water, and then dissolved in chloroform, and the organic layer was dried over anhydrous magnesium sulfate. The organic layer was concentrated, and the residue was purified by silica gel column chromatography, and dried to obtain crystals of the title compound (134 mg, yield 65%).
$^1H$-NMR ($CDCl_3$) δ: 3.32 (6H, s), 7.33 (1H, d, J=8.6Hz), 7.59 (1H, d, J=8.2Hz), 7.75-7.80 (2H, m), 8.44-8.49 (2H, m)
Melting point: 198 to 201°C

Example 41: 4-Aminomethyl-2-(4-trifluoromethylphenyl)benzoxazole

[Formula 35]

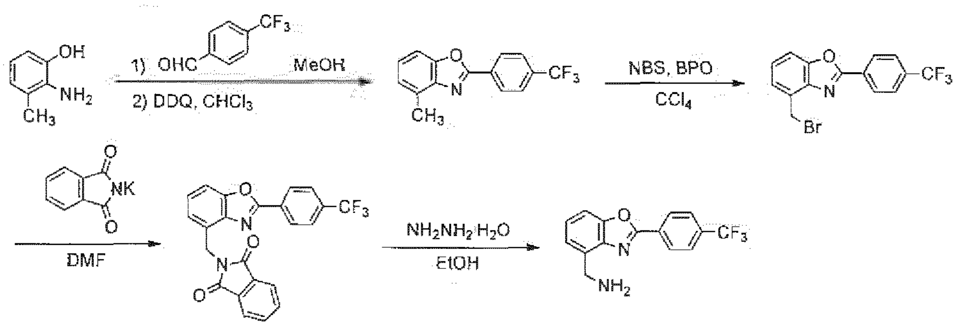


a) 4-Methyl-2-(4-trifluoromethylphenyl)benzoxazole

A mixture of 2-amino-3-methylphenol (3.42 g, 27.8 mmol), and 4-(trifluoromethyl)benzaldehyde (4.84 g, 27.8 mmol) in methanol (28 mL) was stirred with heating at 60°C for 2 hours. After the solvent was evaporated, chloroform (100 mL) was added to the residue. 2,3-Dichloro-5,6-dicyano-1,4-benzoquinone (9.49 g, 41.8 mmol) was added to the reaction mixture under ice cooling, and the resulting mixture was stirred overnight at room temperature, and then stirred with heating at 60°C for 2 hours. The reaction mixture was left to cool, and diluted with chloroform, saturated aqueous sodium carbonate was added to the reaction mixture, and the resulting mixture was filtered through Celite. The filtrate was extracted with chloroform, and the organic layer was washed with saturated aqueous sodium carbonate, and then with saturated aqueous sodium chloride. The organic layer was dried over anhydrous magnesium sulfate, concentrated, and then dried to obtain crystals of the title compound (6.70 g, yield 87%).
$^1H$-NMR ($CDCl_3$) δ: 2.69 (3H, s), 7.18 (1H, dt, J=7.6Hz, 1.0Hz), 7.29 (1H, t, J=7.9Hz), 7.43 (1H, d, J=8.6Hz), 7.78 (2H, d, J=8.2Hz), 8.39 (2H, d, J=7.9Hz)

b) 4-Bromomethyl-2-(4-trifluoromethylphenyl)benzoxazole

To a suspension of the compound obtained in a) (6.63 g, 23.91 mmol) in carbon tetrachloride (50 mL), N-bromosuccinimide (4.46 g, 25.07 mmol), and benzoyl peroxide (purity 75%, 580 mg, 1.80 mmol) were added, and the resulting mixture was stirred with refluxing by heating for 5 hours. The reaction mixture was left to cool to room temperature, and filtered, and the residue was washed with carbon tetrachloride. The filtrate and the washing solution were combined, and concentrated, and the residue was purified by silica gel column chromatography, and dried to obtain crystals of the title compound (6.92 g, yield 81%).
$^1H$-NMR ($CDCl_3$) δ: 4.95 (2H, s), 7.37 (1H, t, J=7.9Hz), 7.45 (1H, dd, J=1.3Hz, 7.9Hz), 7.56 (1H, dd, J=1.3Hz. 7.9Hz), 7.78-7.81 (2H, m), 8.39-8.43 (2H, m)

c) 2-{[2-(4-Trifluoromethylphenyl)benzoxazol-4-yl]-methyl}-1H-isoindole-1,3(2H)-dione

To a solution of the compound obtained in b) (3.00 g, 8.41 mmol) in N,N-dimethylformamide (120 mL), phthalimide potassium (1.64 g, 8.83 mmol) was added, and the resulting mixture was stirred overnight with heating at 70°C. The solvent was evaporated from the reaction mixture, then water was added to the residue, and the resulting mixture was stirred. The precipitates were separated by filtration, washed with water, and then dried to obtain crude crystals of the title compound (3.44 g, 97%). The crude crystals were purified by silica gel column chromatography, and dried to obtain crystals of the title compound (2,40 g, yield 67%).
$^1H$-NMR ($CDCl_3$) δ: 5.35 (2H, s), 7.28 (1H, d, J=7.6Hz), 7.34 (1H, t, J=7.9Hz), 7.50 (1H, dd, J=1.0Hz, 7.9Hz), 7.69 (2H, d, J=8.6Hz), 7.74-7.81 (2H, m), 7.89-7.9 (2H, m), 8.13 (2H, d, J=8.2Hz)

d) 4-Aminomethyl-2-(4-trifluoromethylphenyl)benzoxazole

At 70°C, hydrazine monohydrate (2 mL) was added to a suspension of the compound obtained in c) (2.35 g, 5.57 mmol) in ethanol (87 mL), and the resulting mixture was refluxed by heating for 2 hours. To the reaction solution, ethanol (100 mL) was added, the resulting mixture was heated, and then left to cool, and the insoluble matter was removed by filtration. Water was added to ethanol of the filtrate, and the precipitates were separated by filtration, and dried to obtain crude crystals of the title compound (1.79 g, 110%). The crude crystals (500 mg) were suspended in chloroform (500 mL), and the suspension was refluxed by heating. The insoluble matter was separated by filtration, chloroform of the filtrate was evaporated, and then the residue was dried to obtain crystals of the title compound (446 mg, yield 27%).
$^1$H-NMR (DMSO-$d_6$) δ: 4.13 (2H, s), 7.41-7.50 (2H, m), 7.67 (1H, dd, J=2.3Hz, 7.3Hz), 7.98 (2H, d, J=8.2Hz), 8.41 (2H, d, J=7.9Hz)
Melting point: 118°C

Example 42: 2-[4-(Trifluoromethyl)phenyl]oxazolo[4,5-f]isoquinoline

[Formula 36]

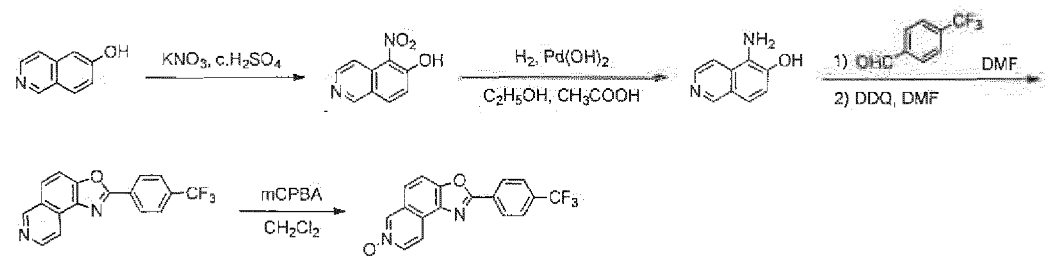


a) 6-Hydroxy-5-nitroisoquinoline

Concentrated sulfuric acid (10 mL) was added to 6-hydroxyisoquinoline (1.5 g, 10 mmol), and then the resulting mixture was stirred at 0°C for 5 minutes. Potassium nitrate (1.1 g, 11 mmol) was added to the reaction mixture, and the resulting mixture was stirred at 0°C for 2 hours. The reaction mixture was poured into ice, and the resulting mixture was adjusted to pH 8 to 9 with aqueous ammonia. Then, the deposited solid was collected by filtration, and dried to obtain crystals of the title compound (1.1 g, yield 59%).
$^1$H-NMR (DMSO-$d_6$) δ: 7.1-7.2 (1H, m), 7.52 (1H, d, J=6.5Hz), 8.02 (1H, d, J=9.2Hz), 8.2-8.3 (1H, m), 9.05 (1H, s)

b) 5-Amino-6-hydroxyisoquinoline

The compound obtained in a) (1.1 g, 5.9 mmol) was dissolved in a mixture of ethanol (40 mL) and acetic acid (40 mL), palladium hydroxide (20 mg) was added to the solution, and the resulting mixture was stirred overnight at room temperature under a hydrogen atmosphere. The reaction mixture was filtered, then the solvent was evaporated under reduced pressure, and the residue was dried to obtain crystals of the title compound (960 mg, quantitative).
$^1$H-NMR (DMSO-$d_6$) δ: 7.20 (1H, d, J=8.4Hz), 7.26 (1H, d, J=8.6Hz), 7.83 (1H, d, J=6.2Hz), 8.22 (1H, d, J=5.9Hz), 8.97 (1H,s)

c) 2-[4-(Trifluoromethyl)phenyl]oxazolo[4,5-f]isoquinoline

By using the compound obtained in b) (160 mg, 1.0 mmol) as a starting material, as well as N,N-dimethylformamide (5 mL) solution, 4-(trifluoromethyl)benzaldehyde (134 μL, 1.0 mmol), and 2,3-dichloro-5,6-dicyano-1,4-benzoquinone (250 mg, 1,1 mmol), crystals of the title compound (96 mg, yield 31%) were obtained in the same manner as that of Example 29, c).
$^1$H-NMR (DMSO-$d_6$) δ: 8.04 (2H, d, J=8.9Hz), 8.25 (2H, d, J=5.9Hz), 8.32 (1H, d, J=5.7Hz), 8.50 (2H, d, J=8.4Hz), 8.75 (1H, d, J=5.9Hz), 9.52 (1H, s)

Example 43: 2-[4-(Trifluoromethyl)phenyl]oxazolo[4,5-f]isoquinoline 7-oxide

To a solution of the compound obtained in Example 42 (63 mg, 0.2 mmol) in dichloromethane (5 mL), m-chloroperbenzoic acid (69 mg, 0.4 mmol) was slowly added under ice cooling, and then the resulting mixture was warmed to room temperature, and stirred for 2 hours under a nitrogen atmosphere. To the reaction solution, 20% sodium sulfite was added, and the resulting mixture was washed twice, then washed with saturated sodium carbonate and saturated aqueous sodium chloride, dried over anhydrous magnesium sulfate, and filtered. Then, the solvent was evaporated under reduced pressure, and the residue was dried to obtain crystals of the title compound (56 mg, yield 85%).
$^1$H-NMR (DMSO-$d_6$) δ: 8.0-8.1 (3H, m), 8.22 (1H, d, J=8.9Hz), 8.35 (2H, s), 8.48 (1H, d, J=8.6Hz), 9.18 (1H, s)
Melting point: higher than 300°C

Example 44: 4-Chloro-2-(6-chloro-benzothiazol-2-yl)benzoxazole

[Formula 37]

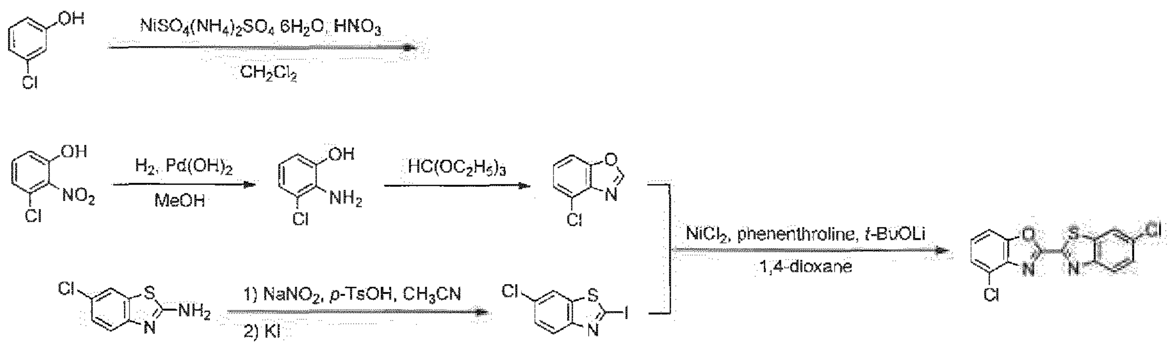


a) 3-Chloro-2-nitrophenol

Under ice cooling, ammonium nickel(II) sulfate hexahydrate (5.9 g, 15 mmol) was added to a solution of 3-chlorophenol (3.9 g, 30 mmol) in dichloromethane (45 mL), nitric acid (specific gravity 1.42, 2.5 mL) was slowly added dropwise to the mixture, and the resulting mixture was warmed to room temperature, and stirred for 3 hours. The reaction mixture was poured into ice, water was added to the mixture, and the resulting mixture was neutralized with sodium hydrogencarbonate. The resulting organic layer was dried over anhydrous magnesium sulfate, then the solvent was evaporated under reduced pressure, and the resulting residue was purified by silica gel column chromatography to obtain crystals of the title compound (1.7 g, yield 33%). $^1$H-NMR ($CDCl_3$) δ: 6.98 (1H, dd, J=2.2Hz, 9.2Hz), 7.19 (1H, d, J=2.4Hz), 8.07 (1H, d, J=9.2Hz), 10.66 (1H, s)

b) 2-Amino-3-chlorophenol

By using the compound obtained in a) (1.7 g, 10 mmol) as a starting material, as well as palladium hydroxide (20 mg), and methanol (30 mL), crystals of the title compound (1.2 g, yield 88%) were obtained in the same manner as that of Example 23, b).
$^1$H-NMR ($CDCl_3$) δ: 6.8-7.3 (3H, m), 9.83 (2H, br), 10.71 (1H, s)

c) 4-Chloro-benzoxazole

Triethyl orthoformate (3 mL) was added to the compound obtained in b) (574 mg, 4 mmol), and the resulting mixture was stirred at 105°C for 4 hours. The reaction mixture was left to cool to room temperature, and the resulting residue was purified by silica gel column chromatography to obtain crystals of the title compound (538 mg, yield 88%),
$^1$H-NMR ($CDCl_3$) δ: 7.3-7.4 (2H, m), 7.52 (1H, dd, J=1.4Hz, 7.8Hz), 8.15 (1H, s)

d) 2-Iodo-6-chloro-benzothiazole

Under ice cooling, p-toluenesulfonic acid hydrate (11.4 g) was added to a solution of 2-amino-6-chloro-1,3-benzothiazole (3.7 g, 20 mmol) in acetonitrile (60 mL), and then a solution of sodium nitrite (1.7 g, 24 mmol) dissolved

in water (15 mL) was slowly added dropwise to the mixture, and the resulting mixture was stirred at 0°C for 1 hour. A solution of potassium iodide (5.0 g, 30 mmol) dissolved in water (20 mL) was added dropwise to the reaction mixture, and then the resulting mixture was stirred at room temperature for 3 hours. The solvent was evaporated, the resulting residue was dissolved in ethyl acetate, the solution was washed successively with 2 N sodium hydroxide solution, 1 N sodium thiosulfate solution, 1 N hydrochloric acid, saturated aqueous sodium carbonate, and saturated aqueous sodium chloride, and dried over magnesium sulfate, and then the solvent was evaporated under reduced pressure. The residue was purified by silica gel column chromatography to obtain crystals of the title compound (1.7 g, yield 28%).
$^1$H-NMR ($CDCl_3$) δ: 7.42 (1H, dd, J=2.4Hz, 8.9Hz), 7.84 (1H, d, J=1.9Hz), 7.94 (1H, d, J=8.9Hz)

e) 4-Chloro-2-(6-chloro-benzothiazol-2-yl)benzoxazole

Under a nitrogen atmosphere, the compound obtained in d) (296 mg, 1.0 mmol), nickel chloride (1.3 mg), 1,10-phenanthroline (1.8 mg), and lithium t-butoxide (160 mg, 2.0 mmol) were added to a solution of the compound obtained in c) (184 mg, 1.2 mmol) in 1,4-dioxane (4 mL), and the resulting mixture was stirred overnight with heating at 90°C. The reaction mixture was left to cool to room temperature, the insoluble matter was removed by using Celite, the filtrate was evaporated under reduced pressure, and the resulting residue was purified by silica gel column chromatography to obtain crystals of the title compound (60 mg, yield 19%).
$^1$H-NMR (DMSO-$d_6$) δ: 7.6-7.7 (3H, m), 7.9-8.0 (1H, m), 8.26 (1H, d, J=8.9Hz), 8.4-8.5 (1H, m)
Melting point: 268.2°C

Example 45: 3-Amino-6,7-dichloro-2-(4-trifluoromethylphenyl)indole

[Formula 38]

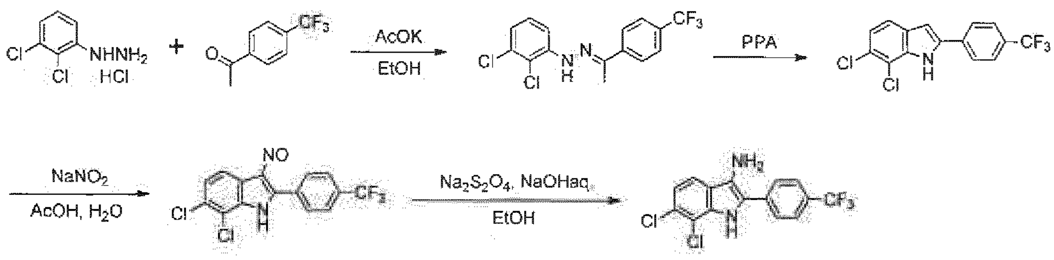


a) (E)-1-(2,3-Dichlorophenyl)-2-{1-[4-(trifluoromethyl)phenyl]ethylidene}hydrazine

To a solution of 2,3-dichlorophenylhydrazine hydrochloride (1.07 g, 5.00 mmol), and 4-acetylbenzotrifluoride (941 mg, 5.00 mmol) in ethanol (20 mL), potassium acetate (981 mg, 10.00 mmol) was added, and the resulting mixture was stirred overnight at room temperature. After completion of the reaction, the solvent was evaporated, then water was added to the residue, and the resulting mixture was extracted with ethyl acetate. The extract was dried over anhydrous magnesium sulfate, and the solvent was evaporated to obtain the title compound (1.65 g, yield 95%).
$^1$H-NMR ($CDCl_3$) δ: 2.34 (3H, s), 7.00 (1H, dd, J=1.3Hz, 8.2Hz), 7.20 (1H, t, J=8.2Hz), 7.59 (1H, dd, J=1.3Hz, 8.2Hz), 7.64 (2H, d, J=8.6Hz), 7.91 (2H, d, J=8.6Hz), 8.01 (1H, br)

b) 6,7-Dichloro-2-[4-(trifluoromethyl)phenyl]-1H-indole

A mixture of the compound obtained in a) (347 mg, 1.00 mmol) and polyphosphoric acid (1 g) was stirred at 110°C for 2 hours. Water was added to the reaction mixture, the resulting mixture was extracted with ethyl acetate, and the extract was dried over anhydrous magnesium sulfate. After the solvent was evaporated, the residue was purified by dry silica gel column chromatography to obtain crystals of the title compound (187 mg, yield 57%).
1H-NMR ($CDCl_3$) δ: 6.92 (1H, d, J=2.3Hz), 7.22 (1H, d, J=8.6Hz), 7.47 (1H, d, J=8.6Hz), 7.72 (2H, d, J=8.6Hz), 7.79 (2H, d, J=8.9Hz), 8.53(1H,br)

c) 6,7-Dichloro-3-nitroso-2-[4-(trifluoromethyl)phenyl]-1H-indole

To a solution of the compound obtained in b) (4.95 g, 15.0 mmol) in a mixture of acetic acid (80 mL) and water (3 mL), sodium nitrite (1.04 g, 15.0 mmol) was added, and the resulting mixture was stirred at room temperature for 1.5

hours- Water was added to the reaction mixture, and the resulting crystals were collected by filtration to obtain the title compound (5.22 g, yield 97%).
1H-NMR $(CDCl_3)$ δ: 7.42 (1H, d, J=7.9Hz), 7.72 (2H, d, J=7.9Hz), 8.00 (1H, d, J=7.9Hz), 8.44 (2H, d, J=7.9Hz), 8.94 (1H, br)

d) 3-Amino-6,7-dichloro-2-(4-trifluoromethylphenyl)indole

To a solution of the compound obtained in c) (5.22 g, 14.5 mmol) in ethanol (70 mL), 2 N sodium hydroxide solution (72.7 mL, 145.4 mmol), and sodium hyposulfite (7.59 g, 43.6 mmol) were added, and the resulting mixture was refluxed with heating for 6 hours. After the reaction mixture was left to cool, water (35 mL) and ethanol (35 mL) were added to the reaction mixture, and the resulting crystals were collected by filtration to obtain the title compound (2.32 g, yield 46%).
1H-NMR $(CDCl_3)$ δ: 3.70 (2H, br), 7.19 (1H, d, J=8.6Hz), 7.37 (1H, d, J=8.9Hz), 7.72-7.83 (5H, m)

Example 46: 2-Methyl-7-[**4-**(trifluoromethyl)phenyl]-8H-pyrrolo[2,3-e]benzoxazole

[Formula 39]

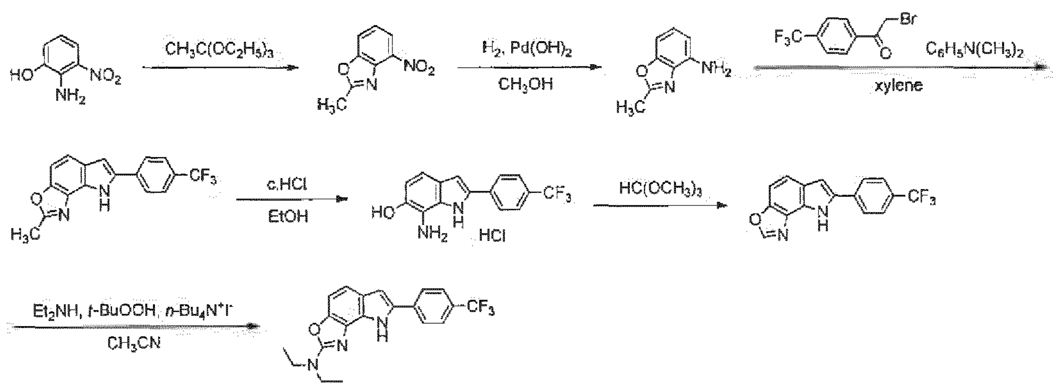


a) 2-Methyl-4-nitrobenzoxazole

A suspension of 2-amino-3-nitrophenol (6.16 g, 40.00 mmol) and ethyl orthoacetate (12 mL) was stirred at 100°C 1 for hour. After the reaction mixture was left to cool, diisopropyl ether was added to the reaction mixture, and the resulting crystals were collected by filtration to obtain crystals of the title compound (6.49 g, yield 91%).
1H-NMR $(CDCl_3)$ δ: 2.79 (3H, s), 7.46 (1H, t, J=8.2Hz), 7.82 (1H, dd, J=1.0Hz, 8.2Hz), 8.18 (1H, dd, J=1.0Hz, 8.2Hz)

b) 4-Amino-2-methylbenzoxazole

To a solution of the compound obtained in a) (6.49 g, 36.43 mmol) in methanol (70 mL), palladium hydroxide (277 mg) was added, and the resulting mixture was stirred for 2 days under a hydrogen atmosphere. After completion of the reaction, the reaction mixture was filtered through Celite, the solvent was evaporated, and then the residue was purified by silica gel column chromatography to obtain crystals of the title compound (5.25 g, yield 97%).
1H-NMR $(CDCl_3)$ δ: 2.61 (3H, s), 6.56 (1H, dd, J=0.7Hz, 7.9Hz), 6.86 (1H, dd, J=0.7Hz, 8.2Hz), 7.07 (1H, t, J=8.2Hz)

c) 2-Methyl-7-4-(trifluoromethyl)phenyl]-8H-pyrrolo[2,3-e]benzoxazole

A solution of the compound obtained in b) (5.25 g, 35.43 mmol), and 4-trifluoromethylphenacyl bromide (9.46 g, 35.43 mmol) in N,N-dimethylaniline (20 mL) and xylene (40 mL) was stirred at 170°C for 17 hours. After the reaction mixture was left to cool, 2 N hydrochloric acid was added to the reaction mixture, and the resulting mixture was extracted with ethyl acetate. The organic layer was dried over anhydrous magnesium sulfate, then the solvent was evaporated, and the resulting crystals were washed with methanol, and collected by filtration to obtain crystals of the title compound (1.95 g, yield 17%).
1H-NMR $(CDCl_3)$ δ: 2.69 (3H, s), 7.03 (1H, d, J=2.3Hz), 7.32 (1H, d, J=8.6Hz), 7.55 (1H, d, J=8.9Hz), 7.70 (2H, d, J=8.9Hz), 7.77 (2H, d, J=8.6Hz), 9.25 (1H, br)

Example 47: 2-Diethylamino-7-(4-trifluoromethylphenyl)-8H-pyrrolo[2,3-e]benzoxazole

a) 7-Amino-6-hydroxy-2-(4-trifluoromethylphenyl)-1H-indole hydrochloride

To a suspension of the compound obtained in Example 46 (1.90 g, 6.00 mmol) in ethanol (40 mL), concentrated hydrochloric acid (5 mL) was added, and the resulting mixture was stirred at 70°C for 16 hours. After the reaction mixture was left to cool, the solvent was evaporated, diisopropyl ether was added to the residue, and the deposited crystals were collected by filtration to obtain crystals of the title compound (1.32 g, yield 67%).
1H-NMR (DMSO-$d_6$) δ: 6.78 (1H, d, J=8.6Hz), 7.08 (1H, d, J=2.0Hz), 7.35 (1H, d, J=8.2Hz), 7.81 (2H, d, J=8.2Hz), 8.05 (2H, d, J=8.2Hz), 10.12 (1H, br), 11.92 (1H, br)

b) 7-[4-(Trifluoromethyl)phenyl]-8H-pyrrolo[2,3-e]benzoxazole

A suspension of the compound obtained in a) (143 mg, 0.44 mmol) and methyl orthoformate (2 mL) was stirred at 100°C for 1 hour. After the reaction mixture was left to cool, the solvent was evaporated, and the residue was purified by silica gel column chromatography to obtain crystals of the title compound (91 mg, yield 69%). 1H-NMR ($CDCl_3$) δ: 7.06 (1H, d, J=2.3Hz), 7.41 (1H, d, J=8.6Hz), 7.65 (1H, d, J=8.6Hz), 7.71 (2H, d, J=8.2Hz), 7.80 (2H, d, J=8.2Hz), 8.13 (1H, s), 9.45 (1H, br)

c) 2-Diethylamino-7-(4-trifiuoromethylphenyl)-8H-pyrrolo[2,3-e]benzoxazole

To a solution of acetic acid (0.05 mL, 0.90 mmol), and tert-butyl hydroperoxide (0.05 mL, 0.45 mmol) in acetonitrile (2 mL), a solution of tetrabutyl ammonium iodide (5.6 mg, 0.015 mmol), diethylamine (0.31 mL, 3.01 mmol), and the compound obtained in b) (91 mg, 0.30 mmol) in acetonitrile (6 mL) was added, and the resulting mixture was refluxed by heating for 6 hours. After the reaction mixture was left to cool, 1 N sodium hydroxide solution was added to the reaction mixture, and the resulting mixture was extracted with ethyl acetate. Then, the extract was dried over anhydrous magnesium sulfate, and the solvent was evaporated. The residue was purified by silica gel column chromatography, and the resulting crystals were washed with methanol to obtain crystals of the title compound (67 mg, yield 60%).
1H-NMR ($CDCl_3$) δ: 1.32 (6H, t, J=7.3Hz), 3.63 (4H, q, J=7.3Hz), 6.97 (1H, d, J=2.3Hz), 7.16 (1H, d, J=8.6Hz), 7.26-7.29 (1H, m), 7.67 (2H, d, J=8.2Hz), 7.77 (2H, d, J=8.2Hz), 9.16 (1H, br)

Melting point: 203.3°C

Test Example 1: Influence of test compound on PCSK9 protein production amount in HepG2 cells

A test compound was added to culture supernatant of cells of the human liver cancer-derived cell strain, HepG2 cells, and intracellular amount of the PCSK9 protein was measured by the enzyme-linked immunosorbent assay (ELISA) after 24 hours. Specifically, the HepG2 cells were inoculated into wells of a 96-well microplate at a density of 5 to 7.5 x $10^4$ cells/well, and the culture supernatant was exchanged with the MEM medium containing 10% lipoprotein deficient serum (LPDS) on the next day. After the cells were further cultured for one day, the culture supernatant was exchanged with the 10% LPDS-containing MEM medium containing a test compound, and the cells were treated with the compound for about 24 hours. The medium for treating the cells was prepared by dissolving the test compound in dimethyl sulfoxide (DMSO), and adding the resulting solution to the 10% LPDS-containing medium. The medium for treating cells for control was prepared by adding DMSO to the 10% LPDS-containing medium at the same final concentration. After completion of the treatment of the cells, a solubilization buffer (1% Nonidet P-40 in phosphate buffered saline) was added to the cells to extract proteins, and they were used as a sample.

The PCSK9 protein concentration and the total protein concentration in the sample were measured by using Human PCSK9 ELISA Kit produced by R&D Systems (Cat. No. DPC900), and BCA Protein Assay Reagent Kit produced by Thermo Scientific (Cat. No. 23227) according to the protocols described in the attached manuals, respectively. The PCSK9 protein concentration in each sample was calculated as a concentration per unit mass of proteins. Then, by using PCSK9 production suppressing ratios at various concentrations obtained in accordance with the equation mentioned below, 50% PCSK9 production-suppressing concentration ($IC_{50}$) of each compound was calculated with a linear regression equation. The results are shown in Table 1 mentioned below.

Equation: PCSK9 production-suppressing ratio (%) = [(Average PCSK9 protein concentration in control group - Average PCSK9 protein concentration in test compound-treated group)/Average PCSK9 protein concentration in control group] x 100

[Table 1]

| Test compound | Structural formula | $IC_{50}$ (nM) |
|---|---|---|
| Example 1 | 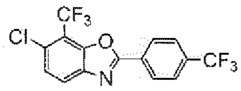 | 0.802 |
| Example 3 | 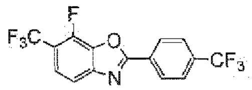 | 8.19 |
| Example 4 | 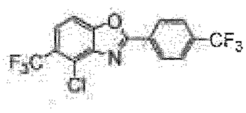 | 0.426 |
| Example 6 | 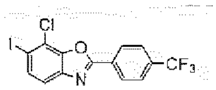 | 2.50 |
| Example 7 | 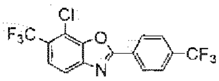 | 0.036 |
| Example 8 | 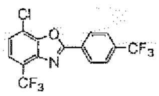 | 0.153 |
| Example 9 | 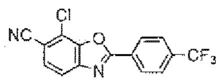 | 0.0182 |
| Example 11 | 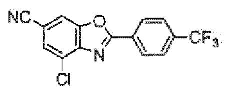 | 1.34 |
| Example 12 | 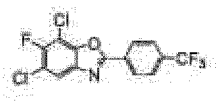 | 2.94 |
| Example 14 | 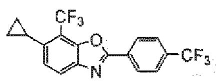 | 4.54 |
| Example 16 | 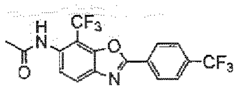 | 2.08 |
| Example 18 | 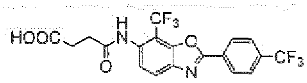 | 16.3 |

(continued)

| Test compound | Structural formula | $IC_{50}$ (nM) |
|---|---|---|
| Example 19 | 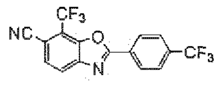 | 0.188 |
| Example 20 | 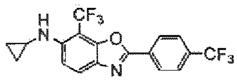 | 3.95 |
| Example 21 | 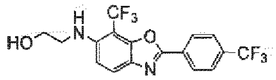 | 5.85 |
| Example 22 | 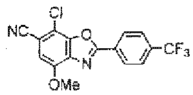 | 99.0 |
| Example 23 | 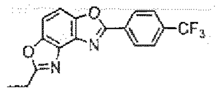 | 52.3 |
| Example 24 | 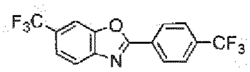 | 21.4 |
| Example 25 | 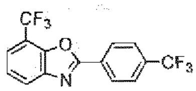 | 24.5 |
| Example 27 | 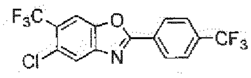 | 12.3 |
| Example 28 | 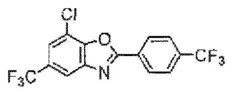 | 1.35 |
| Example 29 | 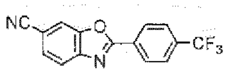 | 27.0 |
| Example 31) | 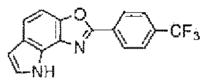 | 29.7 |
| Example 33 | 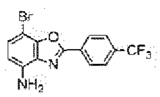 | 4.96 |
| Example 34 | 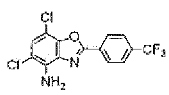 | 20.2 |
| Example 35B | 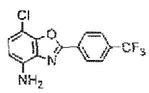 | 1.24 |

(continued)

| Test compound | Structural formula | $IC_{50}$ (nM) |
|---|---|---|
| Example 36 | 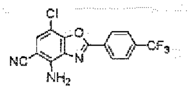 | 5.17 |
| Example 38 | 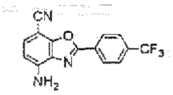 | 27.3 |
| Example 40 | 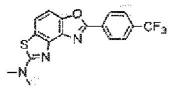 | 3.97 |
| Example 41 | 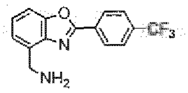 | 19.2 |
| Example 43 | 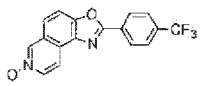 | 25.6 |
| Example 44 | 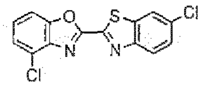 | 13.9 |
| Example 45 | 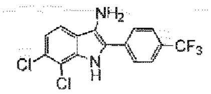 | 2.68 |
| Example 47 | 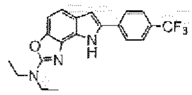 | 45.4 |
| Reference Example | Berberine chloride | > 10 μM |

Test Example 2: Efficacy test in dietary hyperlipidemia hamsters

As test animals, the Slc:Syrian male hamsters were used. In order to create dietary hyperlipidemia models, the hamsters were fed ad libitum with a high fat diet (mixed diet of 0.5% cholesterol and 10% palm oil) for a period from two weeks before the start of the administration of the test compound to the end of the experiment. Blood was collected one day before the day of the start of the administration, and the total blood cholesterol concentration was measured by the direct method using an automatic analyzer. On the basis of this total blood cholesterol concentration, the animals were divided into groups. From the next day, the test compound was orally administered once a day for seven days, and the animals were dissected on the day following the day of the final administration. The LDL-cholesterol concentration in the collected blood was measured by the direct method using an automatic analyzer, and LDL cholesterol decreasing ratio was calculated in accordance with the following equation for each group. The results are shown in Table 2.

Equation: LDL cholesterol decreasing ratio (%) = [(Average LDL-cholesterol concentration of control group - Average LDL-cholesterol concentration of test compound-administered group)/Average LDL-cholesterol concentration of control group] x 100

[Table 2]

| Test compound | Structural formula | Dose (mg/kg) | Decreasing ratio (%) |
|---|---|---|---|
| Example 6 | 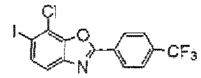 | 5 | 41.8 |
| Example 7 | 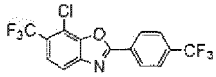 | 0.01<br>0.04 | 45.5<br>56.9 |
| Example 19 | 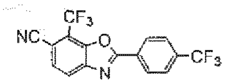 | 1 | 35.2 |
| Example 24 | 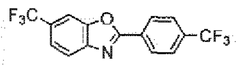 | 1 | 32.6 |
| Example 28 | 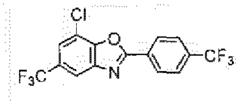 | 0.2 | 63.6 |
| Reference Example | Phenofibrate | 50 | 25.7 |

Test Example 3: Toxicity test in normal hamsters

By using the Slc:Syrian male hamsters of 5 weeks old, the test was performed for total five groups, a solvent control group, groups administered with the compound of Example 7 (0.1 and 1 mg/kg/day), and groups administered with the compound of Example 24 (5 and 50 mg/kg). The administration was performed by orally administering an administration suspension containing the test compound suspended in 1% CMC-Na once a day for two weeks. Death and abnormalities of the general symptoms were not observed during the administration period in all the administration groups.

Preparation Example 1: Tablet

| | |
|---|---|
| Active ingredient | 5.0 mg |
| Starch | 10.0 mg |
| Lactose | 73.0 mg |
| Carboxymethylcellulose calcium | 10.0 mg |
| Talc | 1.0 mg |
| Magnesium stearate | 1.0 mg |
| Total weight | 100.0 mg (one tablet) |

Starch, lactose, and carboxymethylcellulose calcium are added to the active ingredient, and they are mixed well. 10% of starch paste is added to the aforementioned mixed powder, and they are mixed by stirring to prepare granules. After dried, the granules are adjusted to a particle size of about 1,000 μm, talc and magnesium stearate are mixed with the granules, and by tableting the resulting mixture, tablets are obtained.

Preparation Example 2: Capsule

| | |
|---|---|
| Active ingredient | 50 mg |
| Lactose | 100 mg |
| Corn starch | 28 mg |
| Magnesium stearate | 2 mg |
| Total weight | 200 mg (one capsule) |

Capsules are obtained with the aforementioned composition according to the method described in Japanese Pharmacopoeia XIV, General rules for preparations.

Preparation Example 3: Granule

| | |
|---|---|
| Active ingredient | 30 mg |
| Lactose | 20 mg |
| Hydroxypropylmethylcellulose 2910 | 13 mg |
| Crystalline cellulose | 115 mg |
| Crospovidone | 20 mg |
| Magnesium stearate | 2 mg |
| Total weight | 207 mg |

An aqueous solution of hydroxypropylmethylcellulose 2910 (solid content, 13 weight parts) is added to a mixture of the active ingredient, lactose, and crystalline cellulose, the resulting mixture is kneaded, and subjected to extrusion granulation, and the resulting granules are subjected to size adjustment granulation, dried and sieved to obtain granules.

## Claims

**1.** A compound represented by the following general formula (I):

[Formula 1]

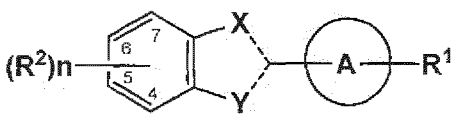

(I)

[in the formula, $R^1$ represents a $C_{1-6}$ alkyl group which may have a substituent, a halogen atom, hydroxy group, nitro group, or an amino group which may have a substituent;
A represents a phenylene group which may have a substituent, or a benzothiazole-diyl group which may have a substituent;
X represents -CH($R^3$)-, -C($R^3$)=, -O-, -NH-, -N=, or -S-;
Y represents -O-, -NH-, -N=, or -S-;
the broken lines ---- independently represent a single bond or double bond;
n represents an integer of 1 to 3;
when n is 1, $R^2$ represents a $C_{1-6}$ alkyl group which may have a substituent, a $C_{1-6}$ alkoxy group which may have a substituent, an amino group which may have a substituent, a halogen atom, or cyano group,
when n is 2, two of $R^2$ represent two of the same or different groups selected from the group consisting of a $C_{1-6}$ alkyl group which may have a substituent, a $C_{1-6}$ alkoxy group which may have a substituent, an amino group which may have a substituent, an acyl group which may have a substituent, carbonyl group, a carbamoyl group which may have a substituent, a halogen atom, hydroxy group, nitro group, cyano group, a carbon cyclic group which may have a substituent, and a heterocyclic group which may have a substituent, and two of $R^2$ may bind together to form a 5- to 7-membered carbon ring which may have a substituent, or a 5- to 7-membered heterocyclic ring which may have a substituent,
when n is 3, three of $R^2$ represent a combination of the groups mentioned above as $R^2$ for the compounds where n is 1, or n is 2; and
$R^3$ represents hydrogen atom, a $C_{1-6}$ alkyl group which may have a substituent, an acyl group which may have a substituent, a carbamoyl group which may have a substituent, or an amino group which may have a substituent, provided that the following compounds are excluded:

(1) those compounds wherein n is 2, X is -O-, Y is -N=, and two of $R^2$ are:

- a combination of carboxamido group and fluorine atom,
- chlorine atom at the 5-position, and an amino group at the 6-position,
- chlorine atom at the 5-position, and nitro group at the 7-position,

- alkyl groups,
- hydroxy group at the 5-position, and bromine atom at the 7-position, - 1,1-dimethylethyl group at the 6-position, and hydroxy group at the 7-position, or
- methyl group at the 5-position, and trifluorophenyl group at the 7-position;

(2) those compounds wherein n is 2, X is -S-, Y is -N=, and two of $R^2$ are:

- a combination of carboxamido group and fluorine atom,
- methyl group at the 4-position, and chlorine atom at the 5-position,
- hydroxy groups at the 4- and 7-positions,
- alkyl groups, or
- halogen atoms;

(3) those compounds wherein n is 2, X is -NH-, Y is -N=, and two of $R^2$ are:

- a combination of carboxamido group and fluorine atom,
- a combination of thiocarbonylamido group and fluorine atom,
- alkyl groups,
- alkoxy groups, or
- halogen atoms;

(4) those compounds wherein n is 2, X is -C($R^3$)=, Y is -NH-, $R^1$ is trifluoromethyl group, $R^3$ is hydrogen atom, and two of $R^2$ are:

- fluorine atoms,
- bromine atom at the 5-position, and fluorine atom at the 7-position,
- chlorine atom at the 4-position, and chlorine atom at the 6- or 7-position,
- chlorine atom at the 5-position, and fluorine atom at the 7-position,
- chlorine atom at the 5-position, and chlorine atom at the 7-position,
- chlorine atom at the 6-position, and fluorine atom at the 7-position, or
- chlorine atom at the 6-position, and chlorine atom at the 7-position; and

(5) those compounds wherein n is 2, X is -C($R^3$)=, Y is -NH-, $R^1$ is chlorine atom, $R^3$ is hydrogen atom, and two of $R^2$ are:

- alkyl groups,
- a combination of an alkyl group and a halogen atom,
- chlorine atom at the 4-position, and chlorine atom at the 6-position, or
- fluorine atom at the 4-position, and fluorine atom at the 6-position],

or a salt thereof.

**2.** The compound or a salt thereof according to claim 1, wherein:

$R^1$ represents a $C_{1-6}$ alkyl group (this $C_{1-6}$ alkyl group may be substituted with one or two or more groups selected from the group consisting of hydroxy group and a halogen atom), a halogen atom, hydroxy group, nitro group, or an amino group;
A represents a phenylene group or a benzothiazole-diyl group (these phenylene group and benzothiazole-diyl group may be substituted with one or two or more groups selected from the group consisting of a $C_{1-6}$ alkyl group (this $C_{1-6}$ alkyl group may be substituted with one or two or more groups selected from the group consisting of hydroxy group and a halogen atom), a halogen atom, hydroxy group, nitro group, and an amino group, as a substituent other than $R^1$);
X represents -C($R^3$)=, -O-, -NH-, or -S-;
Y represents -O-, -NH-, -N=, or -S-;
the broken lines ---- independently represent a single bond or double bond;
n represents an integer of 1 to 3;
when n is 1, $R^2$ represents a $C_{1-6}$ alkyl group, a $C_{1-6}$ alkoxy group, an amino group, a halogen atom, or cyano group, provided that these $C_{1-6}$ alkyl group, $C_{1-6}$ alkoxy group, and amino group may be substituted with one

or two or more groups selected from the substituents of the substituent group A {a $C_{1-6}$ alkyl group, an acyl group (this acyl group may be substituted with a $C_{1-6}$ alkyl group), a halogen atom, an amino group, and a heterocyclic group},
when n is 2, two of $R^2$ represent two of the same or different groups selected from the group consisting of a $C_{1-6}$ alkyl group (this $C_{1-6}$alkyl group may be substituted with one or two or more halogen atoms), a $C_{1-6}$ alkoxy group (this $C_{1-6}$ alkoxy group may be substituted with one or two or more groups selected from the group consisting of an amino group and a carbon cyclic group), an amino group (this amino group may be substituted with one or two or more groups selected from the substituents of the substituent group B {a $C_{1-6}$ alkyl group (this $C_{1-6}$ alkyl group may be substituted with one or two or more groups selected from the group consisting of hydroxy group, an amino group, a phenyl group, acetylamino group, acetyloxy group, a saturated heterocyclic group which may be substituted with hydroxy group, a partially saturated heterocyclic group, and an aromatic heterocyclic group), a carbon cyclic group (this carbon cyclic group may be substituted with a halogen atom), a heterocyclic group, and a sulfonyl group (this sulfonyl group may be substituted with one or two or more groups selected from the group consisting of dimethylamino group, phenyl group, and cyclopropyl group)}), an acyl group, carbonyl group, a carbamoyl group (these acyl group and carbamoyl group may be substituted with one or two or more groups selected from the substituents of the substituent group C {a $C_{1-6}$ alkyl group (this $C_{1-6}$ alkyl group may be substituted with one or two or more groups selected from the group consisting of hydroxy group, an amino group, carboxy group, methoxycarbonyl group, and ethoxycarbonyl group), a $C_{1-6}$ alkoxy group, a carbon cyclic group, and a heterocyclic group}), a halogen atom, hydroxy group, nitro group, cyano group, a carbon cyclic group, and a heterocyclic group (these carbon cyclic group and heterocyclic group may be substituted with one or two or more $C_{1-6}$ alkyl groups), two of $R^2$ may bind together to form a 5- to 7-membered carbon ring or heterocyclic ring, and the ring may have one or two or more groups selected from the substituents mentioned above as $R^2$ for the compounds where n is 2,
when n is 3, three of $R^2$ represent a combination of the groups mentioned above as $R^2$ for the compounds where n is 1, or n is 2;
$R^3$ represents hydrogen atom, a $C_{1-6}$ alkyl group, an acyl group, a carbamoyl group, or an amino group, and these $C_{1-6}$ alkyl group, acyl group, carbamoyl group, and amino group may have one or two or more groups selected from the substituents of the substituent group D {a $C_{1-6}$ alkyl group (this $C_{1-6}$ alkyl group may be substituted with one or two or more groups selected from the group consisting of carboxyl group, methoxycarbonyl group, benzylcarbamoyl group, a carbon cyclic group, and a heterocyclic group), a $C_{1-6}$ alkoxy group, a sulfonyl group (this sulfonyl group may be substituted with a $C_{1-6}$ alkyl group), a carbon cyclic group (this carbon cyclic group may be substituted with methoxyphenyl group), or a heterocyclic group (this heterocyclic group may be substituted with a phenyl group)}.

**3.** The compound or a salt thereof according to claim 1 or 2, wherein X is -O-, -S-, or -NH-, and Y is -N=, or X is -C($R^3$)=, and Y is -NH-, -S-, or -O-.

**4.** A compound represented by the following general formula (IA):

[Formula 2]

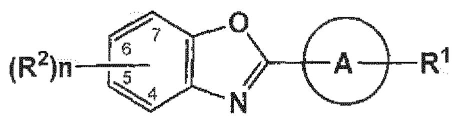

(IA)

($R^1$, A, n, and $R^2$ have the same meanings as those defined in claim 1, provided that when n is 2, those compounds having, as $R^2$:

- chlorine atom at the 5-position, and an amino group at the 6-position,
- chlorine atom at the 5-position, and nitro group at the 7-position,
- alkyl groups,
- hydroxy group at the 5-position, and bromine atom at the 7-position, 1,1-dimethylethyl group at the 6-position, and hydroxy group at the 7-position, or
- methyl group at the 5-position, and trifluorophenyl group at the 7-position are excluded), or a salt thereof.

**5.** The compound or a salt thereof according to claim 4, wherein:

$R^1$ represents a $C_{1-6}$ alkyl group (this $C_{1-6}$ alkyl group may be substituted with one or two or more groups selected from the group consisting of hydroxy group and a halogen atom), a halogen atom, hydroxy group, nitro group, or an amino group;
A represents a phenylene group or a benzothiazole-diyl group (these phenylene group and benzothiazole-diyl group may be substituted with one or two or more groups selected from the group consisting of a $C_{1-6}$ alkyl group (this $C_{1-6}$ alkyl group may be substituted with one or two or more groups selected from the group consisting of hydroxy group and a halogen atom), a halogen atom, hydroxy group, nitro group, and an amino group, as a substituent other than $R^1$);
n represents an integer of 1 to 3;
when n is 1, $R^2$ represent hydrogen atom, a $C_{1-6}$ alkyl group, a $C_{1-6}$ alkoxy group, an amino group, a halogen atom, or cyano group, provided that these $C_{1-6}$ alkyl group, $C_{1-6}$ alkoxy group, and amino group may be substituted with one or two or more groups selected from the substituents of the substituent group A {a $C_{1-6}$ alkyl group, an acyl group (this acyl group may be substituted with a $C_{1-6}$ alkyl group), a halogen atom, an amino group, and a heterocyclic group},
when n is 2, two of $R^2$ represent two of the same or different groups selected from the group consisting of a $C_{1-6}$ alkyl group (this $C_{1-6}$alkyl group may be substituted with one or two or more halogen atoms), a $C_{1-6}$ alkoxy group (this $C_{1-6}$ alkoxy group may be substituted with one or two or more groups selected from the group consisting of an amino group and a carbon cyclic group), an amino group (this amino group may be substituted with one or two or more groups selected from the substituents of the substituent group B {a $C_{1-6}$ alkyl group (this $C_{1-6}$ alkyl group may be substituted with one or two or more groups selected from the group consisting of hydroxy group, an amino group, a phenyl group, acetylamino group, acetyloxy group, a saturated heterocyclic group which may be substituted with hydroxy group, a partially saturated heterocyclic group, and an aromatic heterocyclic group), a carbon cyclic group (this carbon cyclic group may be substituted with a halogen atom), a heterocyclic group, and
a sulfonyl group (this sulfonyl group may be substituted with one or two or more groups selected from the group consisting of dimethylamino group, a phenyl group, and cyclopropyl group)}), an acyl group, carbonyl group, a carbamoyl group (these acyl group and carbamoyl group may be substituted with one or two or more groups selected from the substituents of the substituent group C {a $C_{1-6}$ alkyl group (this $C_{1-6}$ alkyl group may be substituted with one or two or more groups selected from the group consisting of hydroxy group, an amino group, carboxy group, methoxycarbonyl group, and ethoxycarbonyl group), a $C_{1-6}$ alkoxy group, a carbon cyclic group, and a heterocyclic group}), a halogen atom, hydroxy group, nitro group, cyano group, a carbon cyclic group, and a heterocyclic group (these carbon cyclic group and heterocyclic group may be substituted with one or two or more $C_{1-6}$ alkyl groups), two of $R^2$ may bind together to form a 5- to 7-membered carbon ring or heterocyclic ring, and the ring may have one or two or more groups selected from the substituents mentioned above as $R^2$ for the compounds where n is 2,
when n is 3, three of $R^2$ represent a combination of the groups mentioned above as $R^2$ for the compounds where n is 1, or n is 2;
$R^3$ represents hydrogen atom, a $C_{1-6}$ alkyl group, an acyl group, a carbamoyl group, or an amino group, and these $C_{1-6}$ alkyl group, acyl group, carbamoyl group, and amino group may have one or two or more groups selected from the substituents of the substituent group D {a $C_{1-6}$ alkyl group (this $C_{1-6}$ alkyl group may be substituted with one or two or more groups selected from the group consisting of carboxyl group,
methoxycarbonyl group, benzylcarbamoyl group, a carbon cyclic group, and a heterocyclic group), a $C_{1-6}$ alkoxy group, a sulfonyl group (this sulfonyl group may be substituted with a $C_{1-6}$ alkyl group), a carbon cyclic group (this carbon cyclic group may be substituted with methoxyphenyl group), or a heterocyclic group (this heterocyclic group may be substituted with a phenyl group)}.

**6.** The compound or a salt thereof according to claim 4 or 5, wherein A is a phenylene group (this phenylene group may be substituted with one or two or more groups selected from the group consisting of a $C_{1-6}$ alkyl group (this $C_{1-6}$ alkyl group may be substituted with one or two or more groups selected from the group consisting of hydroxy group and a halogen atom), a halogen atom, hydroxy group, nitro group, and amino group, as a substituent other than $R^1$), and $R^1$ is chlorine atom or trifluoromethyl group.

**7.** The compound or a salt thereof according to claim 4 or 5, wherein A is unsubstituted 1,4-phenylene group, and $R^1$ is trifluoromethyl group.

**8.** A compound represented by the following general formula (IB):

[Formula 3]

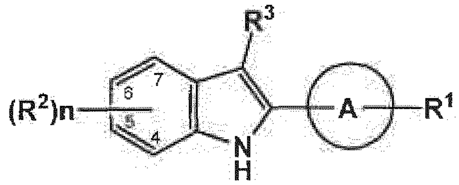

(IB)

($R^1$, A, n, $R^2$, and $R^3$ have the same meanings as those defined in claim 1, provided that when n is 2, and $R^3$ is hydrogen atom, the following compounds are excluded:

(a) those compounds wherein $R^1$ is trifluoromethyl group, and two of $R^2$ are:

- fluorine atoms,
- bromine atom at the 5-position, and fluorine atom at the 7-position,
- chlorine atom at the 4-position, and chlorine atom at the 6- or 7-position,
- chlorine atom at the 5-position, and fluorine atom at the 7-position,
- chlorine atom at the 5-position, and chlorine atom at the 7-position,
- chlorine atom at the 6-position, and fluorine atom at the 7-position, or
- chlorine atom at the 6-position, and chlorine atom at the 7-position, and

(b) those compounds wherein $R^1$ is chlorine atom, and two of $R^2$ are:

- alkyl groups,
- a combination of an alkyl group and a halogen atom,
- chlorine atom at the 4-position, and chlorine atom at the 6-position, or
- fluorine atom at the 4-position, and fluorine atom at the 6-position), or a salt thereof.

**9.** The compound or a salt thereof according to claim 8, wherein:

$R^1$ represents a $C_{1-6}$ alkyl group (this $C_{1-6}$ alkyl group may be substituted with one or two or more groups selected from the group consisting of hydroxy group and a halogen atom), a halogen atom, hydroxy group, nitro group, or an amino group;
A represents a phenylene group or a benzothiazole-diyl group (these phenylene group and benzothiazole-diyl group may be substituted with one or two or more groups selected from the group consisting of a $C_{1-6}$ alkyl group (this $C_{1-6}$ alkyl group may be substituted with one or two or more groups selected from the group consisting of hydroxy group and a halogen atom), a halogen atom, hydroxy group, nitro group, and amino group, as a substituent other than $R^1$);
n represents an integer of 1 to 3;
when n is 1, $R^2$ represents a $C_{1-6}$ alkyl group, a $C_{1-6}$ alkoxy group, an amino group, a halogen atom, or cyano group, provided that these $C_{1-6}$ alkyl group, $C_{1-6}$ alkoxy group, and amino group may be substituted with one or two or more groups selected from the substituents of the substituent group A {a $C_{1-6}$ alkyl group, an acyl group (this acyl group may be substituted with a $C_{1-6}$ alkyl group), a halogen atom, an amino group, and a heterocyclic group},
when n is 2, two of $R^2$ represent two of the same or different groups selected from the group consisting of a $C_{1-6}$ alkyl group (this $C_{1-6}$alkyl group may be substituted with one or two or more halogen atoms), a $C_{1-6}$ alkoxy group (this $C_{1-6}$ alkoxy group may be substituted with one or two or more groups selected from the group consisting of an amino group and a carbon cyclic group), an amino group (this amino group may be substituted with one or two or more groups selected from the substituents of the substituent group B {a $C_{1-6}$ alkyl group (this $C_{1-6}$ alkyl group may be substituted with one or two or more groups selected from the group consisting of hydroxy group, an amino group, a phenyl group, acetylamino group, acetyloxy group, a saturated heterocyclic group which may be substituted with hydroxy group, a partially saturated heterocyclic group, and an aromatic heterocyclic group), a carbon cyclic group (this carbon cyclic group may be substituted with a halogen atom), a heterocyclic group, and a sulfonyl group (this sulfonyl group may be substituted with one or two or more groups selected from the group consisting of dimethylamino group, a phenyl group, and cyclopropyl group)}), an acyl group, carbonyl group, a carbamoyl group (these acyl group and carbamoyl group may be substituted with one or two or more groups selected from the substituents of the substituent group C {a $C_{1-6}$ alkyl group (this $C_{1-6}$

alkyl group may be substituted with one or two or more groups selected from the group consisting of hydroxy group, an amino group, carboxy group, methoxycarbonyl group, and ethoxycarbonyl group), a $C_{1-6}$ alkoxy group, a carbon cyclic group, and a heterocyclic group}), a halogen atom, hydroxy group, nitro group, cyano group, a carbon cyclic group, and a heterocyclic group (these carbon cyclic group and heterocyclic group may be substituted with one or two or more $C_{1-6}$ alkyl groups), two of $R^2$ may bind together to form a 5- to 7-membered carbon ring or heterocyclic ring, and the ring may have one or two or more groups selected from the substituents mentioned above as $R^2$ for the compounds where n is 2,

when n is 3, three of $R^2$ represent a combination of the groups mentioned above as $R^2$ for the compounds where n is 1, or n is 2;

$R^3$ represents hydrogen atom, a $C_{1-6}$ alkyl group, an acyl group, a carbamoyl group, or an amino group, and these $C_{1-6}$ alkyl group, acyl group, carbamoyl group, and amino group may have one or two or more groups selected from the substituents of the substituent group D {a $C_{1-6}$ alkyl group (this $C_{1-6}$ alkyl group may be substituted with one or two or more groups selected from the group consisting of carboxyl group, methoxycarbonyl group, benzylcarbamoyl group, a carbon cyclic group, and a heterocyclic group), a $C_{1-6}$ alkoxy group, a sulfonyl group (this sulfonyl group may be substituted with a $C_{1-6}$ alkyl group), a carbon cyclic group (this carbon cyclic group may be substituted with methoxyphenyl group), or a heterocyclic group (this heterocyclic group may be substituted with a phenyl group)}.

**10.** The compound or a salt thereof according to claim 8, wherein A is a phenylene group (this phenylene group may be substituted with one or two or more groups selected from the group consisting of a $C_{1-6}$ alkyl group (this $C_{1-6}$ alkyl group may be substituted with one or two or more groups selected from the group consisting of hydroxy group and a halogen atom), a halogen atom, hydroxy group, nitro group, and an amino group, as a substituent other than $R^1$), and $R^1$ is chlorine atom or trifluoromethyl group.

**11.** The compound or a salt thereof according to claim 8, wherein A is unsubstituted 1,4-phenylene group, and $R^1$ is chlorine atom or trifluoromethyl group.

**INTERNATIONAL SEARCH REPORT**

International application No.
PCT/JP2015/072442

A. CLASSIFICATION OF SUBJECT MATTER
*C07D209/40*(2006.01)i, *A61K31/4045*(2006.01)i, *A61K31/423*(2006.01)i, *A61K31/424*(2006.01)i, *A61K31/428*(2006.01)i, *A61K31/429*(2006.01)i, *A61P3/06*(2006.01)i, *C07D263/56*(2006.01)i, *C07D417/04*(2006.01)i,

According to International Patent Classification (IPC) or to both national classification and IPC

B. FIELDS SEARCHED

Minimum documentation searched (classification system followed by classification symbols)
C07D209/40, A61K31/4045, A61K31/423, A61K31/424, A61K31/428, A61K31/429, A61P3/06, C07D263/56, C07D417/04, C07D498/04, C07D513/04

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2015 |
| Kokai Jitsuyo Shinan Koho | 1971-2015 | Toroku Jitsuyo Shinan Koho | 1994-2015 |

Electronic data base consulted during the international search (name of data base and, where practicable, search terms used)
CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | JP 2009-516649 A (Merck & Co., Inc.), 23 April 2009 (23.04.2009), claims 1, 14 to 15; paragraph [0063] & US 2009/0264405 A1 claims 1, 14 to 15; paragraph [0143] & JP 5301999 B & WO 2007/070173 A2 & EP 1954287 A2 | 1,3-4 |
| X | SWEIS,R.F. et al, 2-(4-carbonylphenyl) benzoxazole inhibitors of CETP: scaffold design and advancement in HDLc-raising efficacy, Bioorganic & Medicinal Chemistry Letters, 2011, Vol.21, No.6, p.1890-1895, ISSN: 0960-894X, scheme 1, table 1 | 1,3-4 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 26 October 2015 (26.10.15) | 02 November 2015 (02.11.15) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (July 2009)

**INTERNATIONAL SEARCH REPORT**

International application No. PCT/JP2015/072442

C (Continuation). DOCUMENTS CONSIDERED TO BE RELEVANT

| Category* | Citation of document, with indication, where appropriate, of the relevant passages | Relevant to claim No. |
|---|---|---|
| X | KALLASHI,F. et al, 2-Arylbenzoxazoles as CETP inhibitors: Raising HDL-C in cynoCETP transgenic mice, Bioorganic & Medicinal Chemistry Letters, 2011, Vol.21, No.1, p.558-561, ISSN: 0960-894X, fig. 1, schemes 1 to 2, tables 1 to 4 | 1,3-4 |
| X | SMITH,C.J. et al, 2-Arylbenzoxazoles as CETP inhibitors: Substitution of the benzoxazole moiety, Bioorganic & Medicinal Chemistry Letters, 2010, Vol.20, No.1, p.346-349, ISSN: 0960-894X, schemes 1, 3, tables 1 to 4 | 1,3-4 |
| X | JP 6-506467 A (Rhone-Poulenc Rorer International (Holdings) Inc.), 21 July 1994 (21.07.1994), page 13, lower right column; page 14, upper right column; page 14, lower left column & US 5385912 A columns 25 to 26, 29 to 30, 33 to 34 & US 5494918 A & WO 1992/015579 A1 & WO 1995/031458 A1 | 1,3-6 |
| X | JP 2007-528387 A (Pfizer Products Inc.), 11 October 2007 (11.10.2007), claim 1; examples 3, 6, 14, 23, 71, 80, 97, 119, 127, 129, 132, 135, 136, 144, 147, 221, 229, 232, 235, 238, 239, 248, 253, 264, 267, 322, 323 & US 2005/0228015 A1 claim 1; examples 3, 6, 14, 23, 71, 80, 97, 119, 127, 129, 132, 135, 136, 144, 147, 221, 229, 232, 235, 238, 239, 248, 253, 264, 267, 322, 323 & US 2006/0258723 A1 & WO 2005/092845 A1 & EP 1725524 A1 & KR 10-2007-0001173 A & CN 1930121 A | 1,3-4 |
| X | JP 2-167224 A (Tanabe Seiyaku Co., Ltd.), 27 June 1990 (27.06.1990), preparation example 3; referential example 3 & JP 5-194221 A & JP 1-180028 A & WO 1991/019496 A1 | 1-6 |
| X | WO 2014/116593 A1 (ALDEXA THERAPEUTICS, INC.), 31 July 2014 (31.07.2014), schemes 1 to 2; table 1 & AU 2014209585 A & CA 2898869 A | 1-5 |
| X | JP 2014-024839 A (Sumitomo Chemical Co., Ltd.), 06 February 2014 (06.02.2014), preparation examples 66-2, 66-3 (Family: none) | 1-6 |

Form PCT/ISA/210 (continuation of second sheet) (July 2009)

**INTERNATIONAL SEARCH REPORT**

International application No. PCT/JP2015/072442

C (Continuation). DOCUMENTS CONSIDERED TO BE RELEVANT

| Category* | Citation of document, with indication, where appropriate, of the relevant passages | Relevant to claim No. |
|---|---|---|
| X | WO 2014/071044 A1 (ALLERGAN, INC.), 08 May 2014 (08.05.2014), table 7; compounds 142, 149, 151, 153, 154, 170 (Family: none) | 1-5 |
| X | WO 2014/116836 A2 (ALDEXA THERAPEUTICS, INC.), 31 July 2014 (31.07.2014), compound 12 & AU 2014209387 A & CA 2898631 A | 1-5 |
| X | US 2014/0080168 A1 (PANG, YI), 20 May 2014 (20.05.2014), pages 9, 11 & US 2015/0148631 A1 | 1-5 |
| X | KIM,T.H. et al, Synthesis of organogelling, fluoride ion-responsive, cholesteryl-based benzoxazole containing intra- and intermolecular hydrogen-bonding sites, Tetrahedron Letters, 2010, Vol.51, No.42, p.5596-5600, ISSN: 0040-4039, scheme 1 | 1-5 |
| X | JP 2005-520794 A (Wyeth), 14 July 2005 (14.07.2005), examples 16 to 23, 30, 34, 36 to 38, 41 to 50, 52, 53, 58 to 61, 63 & US 2003/0199562 A1 examples 16 to 23, 30, 34, 36 to 38, 41 to 50, 52, 53, 58 to 61, 63 & JP 2011-52010 A & JP 4689960 B & US 2005/0080117 A1 & US 2005/0239851 A1 & US 2007/0167503 A1 & WO 2003/050095 A1 & EP 1982713 A2 & KR 10-2005-0044717 A & CN 1646504 A & CN 101423500 A | 1-6 |
| X | JP 2010-530416 A (Merck Sharp & Dohme Corp.), 09 September 2010 (09.09.2010), example 116 & US 2010/0298288 A1 example 116 & WO 2008/156715 A1 & EP 2170058 A1 | 1-5 |
| X | JP 49-054363 A (Lilly Industries, Ltd.), 27 May 1974 (27.05.1974), example 15k & US 3912748 A example 15k & JP 56-167645 A & JP 56-167673 A & JP 56-167674 A & JP 56-167675 A & JP 59-161346 A & US 3962441 A & US 3962452 A & US 4021440 A | 1-7 |

Form PCT/ISA/210 (continuation of second sheet) (July 2009)

**INTERNATIONAL SEARCH REPORT**

International application No.
PCT/JP2015/072442

C (Continuation). DOCUMENTS CONSIDERED TO BE RELEVANT

| Category* | Citation of document, with indication, where appropriate, of the relevant passages | Relevant to claim No. |
|---|---|---|
| X | JP 5-506456 A (Pfizer Inc.), 22 September 1993 (22.09.1993), pages 8 to 9 & US 5037842 A columns 11 to 14 & JP 7-14924 B & US 5037842 A & WO 1991/019704 A2 & EP 537300 A2 | 1-7 |
| X | JP 3-258770 A (Tanabe Seiyaku Co., Ltd.), 19 November 1991 (19.11.1991), example 10 (Family: none) | 1-7 |
| X | JP 7-013369 A (Ricoh Co., Ltd.), 17 January 1995 (17.01.1995), pages 4 to 5 (Family: none) | 1-7 |
| X | JP 2006-516254 A (Eli Lilly and Co.), 29 June 2006 (29.06.2006), examples & US 2006/0205744 A1 preparations & JP 2006-514069 A & US 2009/0054479 A1 & US 2006/0166983 A1 & US 2006/0217374 A1 & WO 2004/063155 A1 & WO 2004/063190 A1 & WO 2004/092131 A1 & EP 1585726 A1 & EP 1581491 A1 & EP 1581521 A1 | 1-7 |
| X | JP 2005-247848 A (Rottapharm S.p.A.), 15 September 2005 (15.09.2005), example 32 & US 2005/0197331 A1 example 32 & US 2010/0120802 A1 & EP 1571142 A1 | 1-7 |
| X | JP 2009-526034 A (Summit Corporation PLC), 16 July 2009 (16.07.2009), example 15 & US 2009/0075938 A1 example 15 & JP 5376956 B & US 2014/0018320 A1 & WO 2007/091106 A2 & EP 1986633 A2 & KR 10-2009-0010025 A & CN 101420950 A & KR 10-2013-0129476 A | 1-7 |

**INTERNATIONAL SEARCH REPORT**

International application No.
PCT/JP2015/072442

C (Continuation). DOCUMENTS CONSIDERED TO BE RELEVANT

| Category* | Citation of document, with indication, where appropriate, of the relevant passages | Relevant to claim No. |
|---|---|---|
| A | WO 2014/060328 A1 (F. HOFFMANN-LA ROCHE AG), 24 April 2014 (24.04.2014), claim 1; examples 9 to 10 & US 2015/0218133 A1 claim 1; examples 9 to 10 & EP 2909172 A & KR 10-2015-0070290 A & CN 104755461 A | 1-7 |

Form PCT/ISA/210 (continuation of second sheet) (July 2009)

**INTERNATIONAL SEARCH REPORT**

International application No.
PCT/JP2015/072442

Continuation of A. CLASSIFICATION OF SUBJECT MATTER (International Patent Classification (IPC))

*C07D498/04*(2006.01)i, *C07D513/04*(2006.01)i

(According to International Patent Classification (IPC) or to both national classification and IPC)

Form PCT/ISA/210 (extra sheet) (July 2009)

## INTERNATIONAL SEARCH REPORT

International application No.
PCT/JP2015/072442

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

See extra sheet.

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☒ No required additional search fees were timely paid by the applicant. Consequently, this international search report is restricted to the invention first mentioned in the claims; it is covered by claims Nos.:
A part of the invention of claims 1-7 related to a compound represented by general formula (I) wherein X is -O- and Y is -N=.

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

INTERNATIONAL SEARCH REPORT

International application No.
PCT/JP2015/072442

Continuation of Box No.III of continuation of first sheet(2)

The technical feature common to the inventions of claims 1-11 is being related to a compound represented by general formula (I) in claim 1.

However, the above-said technical feature cannot be considered to be a special technical feature, since the technical feature does not make a contribution over the prior art in the light of the contents disclosed in, for example, the following documents 1-23 and so on.

Further, there is no other same or corresponding special technical feature among these inventions.

Accordingly, claims are classified into two inventions each of which has a special technical feature indicated below.

(Invention 1) a part of claims 1-3, and claims 4-7

Invention related to compounds wherein X is "-O-" and Y is "-N=" among the compounds represented by general formula (I) in claim 1.

(Invention 2) a part of claims 1-3, and claims 8-11

Invention related to compounds other than the compounds wherein X is "-O-" and Y is "-N=" among the compounds represented by general formula (I) in claim 1.

Document 1: JP 2009-516649 A (Merck & Co., Inc.), 23 April 2009 (23.04.2009)

Document 2: Bioorganic & Medicinal Chemistry Letters, 2011, Vol.21, No.6, p.1890-1895

Document 3: Bioorganic & Medicinal Chemistry Letters, 2011, Vol.21, No.1, p.558-561

Document 4: Bioorganic & Medicinal Chemistry Letters, 2010, Vol.20, No.1, p.346-349

Document 5: JP 6-506467 A (Rhone-Poulenc Rorer International (Holdings) Inc.), 21 July 1994 (21.07.1994)

Document 6: JP 2007-528387 A (Pfizer Products Inc.), 11 October 2007 (11.10.2007)

Document 7: JP 2-167224 A (Tanabe Seiyaku Co., Ltd.), 27 June 1990 (27.06.1990)

Document 8: WO 2014/116593 A1 (ALDEXA THERAPEUTICS, INC.), 31 July 2014 (31.07.2014)

Document 9: JP 2014-024839 A (Sumitomo Chemical Co., Ltd.), 06 February 2014 (06.02.2014)

Document 10: WO 2014/071044 A1 (ALLERGAN, INC.), 08 May 2014 (08.05.2014)

Document 11: WO 2014/116836 A2 (ALDEXA THERAPEUTICS, INC.), 31 July 2014 (31.07.2014)

Document 12: US 2014/0080168 A1 (PANG, YI), 20 May 2014 (20.05.2014)

Document 13: Tetrahedron Letters, 2010, Vol.51, No.42, p.5596-5600

Document 14: JP 2005-520794 A (Wyeth), 14 July 2005 (14.07.2005)

Document 15: JP 2010-530416 A (Merck Sharp & Dohme Corp.), 09 September 2010 (09.09.2010)

Document 16: JP 49-054363 A (Lilly Industries, Ltd.), 27 May 1974 (27.05.1974)

Document 17: JP 5-506456 A (Pfizer Inc.), 22 September 1993 (22.09.1993)

Document 18: JP 3-258770 A (Tanabe Seiyaku Co., Ltd.), 19 November 1991 (19.11.1991)

Document 19: JP 7-013369 A (Ricoh Co., Ltd.), 17 January 1995 (17.01.1995)

Document 20: JP 2006-516254 A (Eli Lilly and Co.), 29 June 2006 (29.06.2006)

(Continued to next extra sheet)

INTERNATIONAL SEARCH REPORT

International application No.
PCT/JP2015/072442

Document 21: JP 2005-247848 A (Rottapharm S.p.A.), 15 September 2005 (15.09.2005)
Document 22: JP 2009-526034 A (Summit Corporation PLC), 16 July 2009 (16.07.2009)
Document 23: WO 2014/060328 A1 (F. HOFFMANN-LA ROCHE AG), 24 April 2014 (24.04.2014)

Range to be covered by this search:
Although the invention of claim 1 involves an extremely large number of compounds, the matter disclosed within the meaning of PCT Article 5 is merely a slight part thereof, and is not fully supported within the meaning of PCT Article 6, and therefore, a full and meaningful prior art search cannot be carried out on said invention.
Consequently, with respect to the invention of claim 1, this international search report covers only those supported and disclosed by the description, namely some compounds among the compounds represented by general formula (I) in claim 1, wherein X is "-O-", Y is "-N=" and ring A is "an optionally substituted phenylene group" or "a benzothiazole-diyl bonded to an X/Y-containing ring at the 2-position".
The above-said opinion may be also applied to the inventions set forth in claims 2-5 referring to claim 1.

Form PCT/ISA/210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2009143633 A
- JP 4689960 B
- JP 2007528387 A
- JP 2008510827 A
- JP 2008533012 A
- JP 5301999 B
- JP 2009530381 A
- JP 2010530416 A
- JP 2011511803 A
- WO 201437340 A
- US 20030148387 A, Chang
- US 20110071150 A
- US 20060106223 A, Weissmann


### Non-patent literature cited in the description


- *Proc. Natl. Acad. Sci. USA.,* 2009, vol. 106, 9820-9825
- *J. Lipid Res.,* 2007, vol. 48, 763-767
- *Proc. Natl. Acad. Sci. Ursa.,* 2008, vol. 105, 11915-11920
- *Atherosclerosis,* 2008, vol. 201, 266-273
- **PRASENJIT et al.** *J. Org. Chem.,* 2009, vol. 74, 8719
- **JULIEN et al.** *Org. Lett.,* 2008, vol. 10 (13), 2665
- **KAUFMANN et al.** *Bioorg. Med. Chem.,* 2007, vol. 15 (15), 5122
- **EUSTERWIEMANN et al.** *J. Org. Chem.,* 2012, vol. 77, 5461